# EUROPEAN PATENT SPECIFICATION

(11) **EP 0 971 717 B1**
(45) Date of publication and mention of the grant of the patent: **19.12.2001**
(21) Application number: 97939482.2
(22) Date of filing: 21.08.1997
(51) Int. Cl.: A61K 31/70, C07H 17/02, A61P 35/00

(54) **CYTOTOXIC AMINO SUGAR AND RELATED SUGAR DERIVATIVES OF INDOLOPYRROLOCARBAZOLES**
ZYTOTOXISCHER AMINOZUCKER UND VERWANDTE ZUCKERDERIVATE VON INDOLOPYRROLOCARBAZOLEN
AMINOSUCRE CYTOTOXIQUE ET DERIVES DE SUCRE ASSOCIES D'INDOLOPYRROLOCARBAZOLES

(30) Priority: 22.08.1996 US 24657 P
(43) Date of publication of application: 19.01.2000
(73) Proprietor: Bristol-Myers Squibb Company, Wallingford, CT 06492-7660 (US)
(72) Inventor: SAULNIER, Mark, George, Higganum, CT 06441-0209 (US); BALASUBRAMANIAN, Neelakantan, Madison, CT 06443 (US); FRENNESSON, David, Bertil, Naugatuck, CT 06770-2638 (US); ST. LAURENT, Denis, R., Newington, CT 06111 (US); LANGLEY, David, R., Meriden, CT 06450 (US)
(74) Representative: Jones, Alan John
(86) International application number: US9714738
(87) International publication number: WO9807433

(56) References cited:
- EP-A- 0 450 327
- EP-A- 0 545 195
- WO-A-96/04293
- US-A- 5 468 849

## Description

The present invention relates to amino sugar and other sugar derivatives of indolopyrrolocarbazoles, their salts and hydrates, some of which compounds exhibit topoisomerase-I activity, are useful in inhibiting proliferation of tumor cells, are useful by exhibiting antitumor effect, and to a process for the preparation thereof.

Indolo[2,3-a]carbazole alkaloids such as rebeccamycin (U.S. Pat. Nos. 4,487,925 and 4,552,842) and its water-soluble, clinically-active analog, 6-(2-Diethylaminoethyl)rebeccamycin (1) (U.S. Pat.No. 4,785,085) are useful antitumor agents which target DNA. 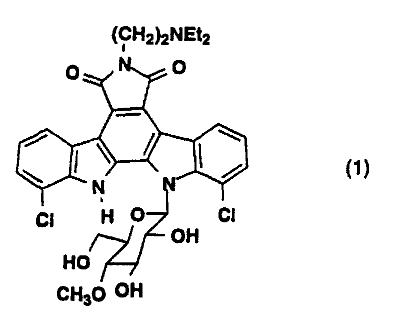 Topoisomerases are vital nuclear enzymes which function to resolve topological dilemmas in DNA, such as overwinding, underwinding and catenation, which normally arise during replication, transcription and perhaps other DNA processes. These enzymes allow DNA to relax by forming enzyme-bridged strand breaks that act as transient gates or pivotal points for the passage of other DNA strands. Topoisomerasetargeting drugs appear to interfere with this breakage-reunion reaction of DNA topoisomerases. In the presence of topoisomerase active agents, an aborted reaction intermediate termed 'cleavable complex' accumulates and results in replication/transcription arrest which ultimately leads to cell death. The development of topoisomerase I active agents, therefore, offers a new approach to the multi-regimental arsenal of therapies currently used in clinics for the treatment of cancer.
The article in Cancer Chemother. Pharmacol (1994), 34 (suppl): S 41-S 45 discusses the Topoisomerase-I active compounds that are in clinical studies and these agents are found to be effective in clinical anti-tumor studies. Structurally these clinical candidates are related to the Camptothecin alkaloid (2) 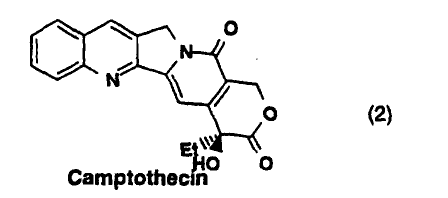 European Patent Application publications 0 545 195 B1 published November 22,1995 and 0,602,597 A2 published June 22,1994 and in Cancer Research **1993**, 53, 490-494 and **1995**, 55, 1310-1315 disclosed indolo[2,3-a]carbazole derivatives (3) related to the Rebeccamycin class and claimed anti-tumor activity; however the major mechanism of action may not be Camptothecin like. Camptothecins act by Topoisomerse I mechanism. The world patent application WO 95/30682 also disclosed indolocarbazoles related to compound (3) and claimed anti-tumor activity. 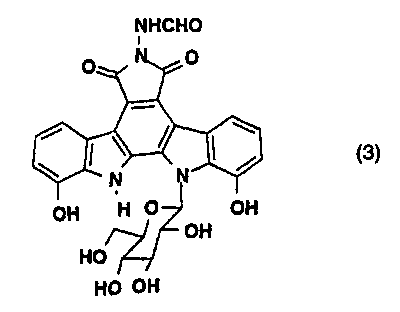 Hudkins et. al. in WO96/11933 published on April 25, 1996 and its corresponding U. S. Pat. No. 5,475,110 disclosed a series of fused pyrrolocarbazoles and showed in vitro biological data such as inhibition of neuronal choline acetyltransferase (ChAT), Protein Kinase C (PKC) inhibition for some compounds. U.S. Pat. No 5,468,849 discloses certain fluororebeccamycin analogs as useful antitumor agents, along with a process for their production by fluorotryptophan analog feeding of a rebeccamycin-producing strain of *Saccharothrix aerocolonigenes,* preferably *Saccharothrix aerocolonigenes* C38,383-RK2 (ATCC 39243).

Glicksman et. al. in U.S. Patent No, 5,468,872 discloses indolocarbazole alkaloids which are different in structure from those of formula I of the present invention.

Kojiri et. al. in WO96/04293 published on February 15, 1996 discloses indolopyrrolocarbazoles having a dissacharide substituent which is different than our amino-substituted sugar compounds.

There is nothing in any of the foregoing references, or in the general prior art, to suggest the novel cytotoxic amino-sugar and other sugar derivatives of indolopyrrolocarbazoles, some of which are topoisomerase I active agents, of the present invention.

### SUMMARY OF THE INVENTION

An object of the present invention is to provide novel sugar derivatives of indolopyrrolocarbazoles which inhibit proliferation of antitumor cells and some of the derivatives exhibit increased aqueous solubility, Topoisomerase-I activity.

This invention relates to novel antitumor compounds represented by formula I, or a pharmaceutically acceptable salt and/or solvate thereof 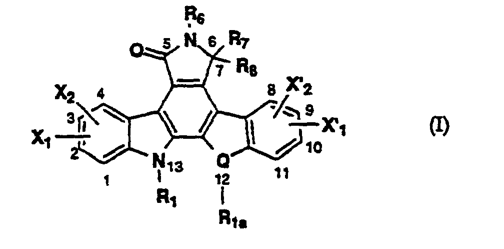 wherein:
R₁ and R₁ₐ are independently hydrogen, a pentose group (A) or a hexose group (B) of the formulas 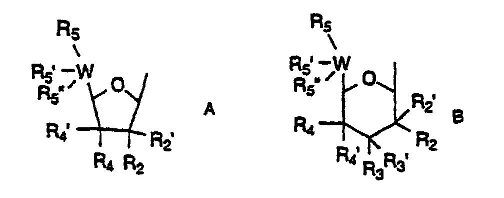 provided that one of R₁ and R₁ₐ is hydrogen and the other is not hydrogen;
R₂, R₃, R₄, R₅ and R_{2'}, R_{3'}, R_{4'}, R_{5"} and R_{5'} are independently hydrogen, C₁₋₇ alkyl, C₁₋₇ cycloalkyl, O, azido, halogen, NR₉R₁₀, NHC(O)NR₉R₁₀, NHC(O)OR, OR, -C(O)Rₐ, SR, -OSO₂R_{c}, or together form =N-OH,, =O, =NR, provided R₂, R₃, R₄, R₅ and R_{2'}, R_{3'}, R_{4'}, R_{5"} and R_{5'} are not simultaneously all hydrogen, OH, alkoxy or alkyl, and further provided that R₃ or R3' is not -NH₂, except when R₆ is
   -(CH₂)ₙNHC(=NH)NH₂, said C₁₋₇ alkyl optionally substituted with one to six of the same or different halogen, CN, NO₂, aryl or heteroaryl, said aryl or heteroaryl substituted with one or two groups independently selected from NR₉R₁₀, OH, COOR₉, SO₃R₉ or OCOR₉;
Rₐ is H, OH, C₁₋₇ alkoxy or NR₉R₁₀;
R_{c} is C₁₋₇ alkyl or aryl;
R and R₁₁ are independently hydrogen, C₁₋₇ alkyl, C₁₋₇ cycloalkyl, heteroaryl, non-aromatic cyclic 5-8 membered ring containing either one or two heteroatoms selected from O or N, (CH₂)ₙNR₉R₁₀, (CH₂)ₙOR₉ or (CH₂)ₙCOOR₉, said C₁₋₇ alkyl optionally substituted with one to six of the same or different halogen, OH, CN, NO₂, aryl or heteroaryl, said aryl or heteroaryl substituted with one or two groups independently selected from NR₉R₁₀, OH, COOR₉, SO₃R₉ or OCOR₉;
R₉ and R₁₀ are independently hydrogen, C₁₋₇ alkyl, C₁₋₇ cycloalkyl, benzyl, aryl, heteroaryl, any of which groups except hydrogen can be substituted with one to six of the same or different halogen, OH, NH₂, CN, NO₂, -C(=NH)NH2, -CH(=NH), CH(R_{b}) (CH₂)ₙ COOH or CH(R_{b}) (CH₂)ₙ NH₂ or COOR₁₁, or R₉ and R₁₀ together with the nitrogen atom to which they are attached form a cyclic 5-8 membered non-aromatic ring containing either one or two heteroatoms selected from O, N, or S or R₉ and R₁₀ together form =CHRR₁₁.
R_{b} is H or COOH;
R₆ is hydrogen, C₁₋₇ alkyl, aryl, arylalkyl, OR₁₀, NR₉R₁₀, or OCO(CH₂)ₙNR₉R₁₀, said C₁₋₇ alkyl optionally substituted with one to six of the same or different halogen, NR₉R₁₀, CN, NO₂, aryl, said aryl
substituted with one or two groups independently selected from NR₉R₁₀, OH, COOR₉, SO₃R₉ or OCOR₉;
R₇ and R₈ are independently OH or H, or taken together is O;
X₁, X'₁, X₂ and X'₂ are independently H, halogen, OH, -CN, -NC, CF₃, -CORₐ, NO₂, OR, O(CH₂)ₙNR₉R₁₀, O(CH₂)ₙOR₉ or O(CH₂)ₙCOOR₉; provided X₂, X'₂, X₁ and X'₁ are not 1, 11-dichloro; provided further when X₂ and X'₂ are each H, X₁ and X'₁ are each independently H or halogen, R, is hexose, R₇ and R₈ together is O, and each of R₂, R₅, and R₄ are OH, R_{2'}, R_{3'}, R_{4'}, and R₅' and R_{5"} are each H, Q is NH, and then each of R₃ and R₆ are not NH₂ and R₃ is not methoxy when R₆ is H;
W is C or N;
Q is O, NR₉, S or CH₂; and
n is an integer from 0-4.

Another aspect of the present invention provides a tumor-growth inhibiting amount of a compound of formula I for inhibiting tumor growth in a mammalian host.

Yet another aspect of the present invention provides a pharmaceutical formulation which comprises an antitumor effective amount of a compound of formula I in combination with one or more pharmaceutically acceptable carriers, excipients, diluents or adjuvants.

### DETAILED DESCRIPTION OF THE INVENTION

The present invention provides novel amino sugar and related derivatives of indolopyrrolocarbazoles and their salts thereof some of which are topoisomerase- I active agents. These compounds are useful in inhibiting proliferation of antitumor cells and exhibit antitumor effect.

In the application, unless otherwise specified explicitly or in context, the following definitions apply. The numbers in the subscript after the symbol "C" define the number of carbon atoms a particular group can contain. For example "C₁₋₆ alkyl" means a straight or branched saturated carbon chain having from one to six carbon atoms; examples include methyl, ethyl, n-propyl, isopropyl, n-butyl, sec-butyl, isobutyl, t-butyl, n-pentyl, sec-pentyl, isopentyl, and n-hexyl. Depending on the context, "C₁₋₆ alkyl" can also refer to C₁₋₆ alkylene which bridges two groups; examples include propane-1,3-diyl, butane-1,4-diyl, 2-methyl-butane-1,4-diyl, etc. "C₂₋₆ alkenyl" means a straight or branched carbon chain having at least one carbon-carbon double bond, and having from two to six carbon atoms; examples include ethenyl, propenyl, isopropenyl, butenyl, isobutenyl, pentenyl, and hexenyl. Depending on the context, "C₂₋₆ alkenyl" can also refer to C₂₋₆ alkenediyl which bridges two groups; examples include ethylene-1,2-diyl (vinylene), 2-methyl-2-butene-1,4-diyl, 2-hexene-1,6-diyl, etc. "C₂₋₆ alkynyl" means a straight or branched carbon chain having at least one carbon-carbon triple bond, and from two to six carbon atoms; examples include ethynyl, propynyl, butynyl, and hexynyl.

"Aryl" means aromatic hydrocarbon having from six to ten carbon atoms; examples include phenyl and naphthyl. "Substituted aryl" means aryl independently substituted with one to five (but preferably one to three) groups selected from C₁₋₆ alkanoyloxy, hydroxy, halogen, C₁₋₆ alkyl, trifluoromethyl, C₁₋₆ alkoxy, aryl, C₂₋₆ alkenyl, C₁₋₆ alkanoyl, nitro, amino, cyano, azido, C₁₋₆ alkylamino, di-C₁₋₆ alkylamino, and amido. "Halogen" means fluorine, chlorine, bromine, and iodine; fluorine is preferred.

"Heteroaryl" means a five- or six-membered aromatic ring containing at least one and up to four non-carbon atoms selected from oxygen, sulfur and nitrogen. Examples of heteroaryl include thienyl, furyl, pyrrolyl, imidazolyl, pyrazolyl, thiazolyl, isothiazolyl, oxazolyl, isoxazolyl, triazolyl, thiadiazolyl, oxadiazolyl, tetrazolyl, thiatriazolyl, oxatriazolyl, pyridyl, pyrimidyl, pyrazinyl, pyridazinyl, triazinyl, tetrazinyl, and like rings.

The compounds of the present invention have the general formula (I) 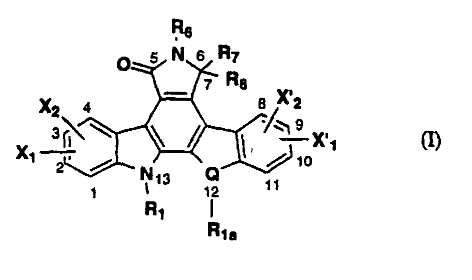 Wherein R₁ represents a pentose group (A) or a hexose group (B) of the formulas

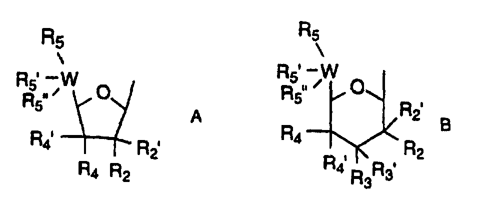

Preferred compounds of formula I are those wherein in R₁ or R₁ₐ all substituents are H except R₂, R₃, and R₄ are each OH; and R₅ is NR₉R₁₀.

Other preferred compounds of formula I are those wherein in R₁ or R₁ₐ all substituents are H except R₂, R₃ and R₅ are each OH; and R₄ is NR₉R₁₀, halogen or N₃ (i.e. azido).

Other preferred compounds of formula I are those wherein in R₁ or R₁ₐ all substituents are H except R₂, R₃ and R₄ are each OH; and R₅ is halogen.

Other preferred compounds of formula I are those wherein in R₁ or R₁ₐ all substituents are H except R₅, R₃ and R₄ are each OH; and R₂ is halogen.

Other preferred compounds of formula I are those wherein in R₁ or R₁ₐ all substituents are H except R₂, R₃ are each OH; and R₅ is halogen and R₄ is azido or NR₉R₁₀ or OR.

Other preferred compounds of formula I are those wherein in R₁ or R₁ₐ all substituents are H except R₂, R₃ are each OH; and R₅ is halogen and R₄ is halogen or H or alkyl.

Other preferred compounds of formula 1 are those wherein in R₁ or R₁ₐ all substituents are H except R₂ and R₃ are each hydrogen or hydroxy; R₄ is hydrogen, halogen, C₁₋₇ alkyl or azido; and R₅ is hydroxy, azido, C₁₋₇ alkyl, halogen or NR₉R₁₀.

Other preferred compounds of formula 1 are those wherein in R₁ or R₁ₐ all substituents are H except R₃ and R₄ are each hydrogen or hydroxy; R₂ is hydrogen, halogen, C₁₋₇ alkyl or azido; and R₅ is hydroxy, azido, C₁₋₇ alkyl, halogen or NR₉R₁₀.

Other preferred compounds of formula 1 are those wherein in R₁ or R₁ₐ all substituents are H except R₃ and R₅ are each hydrogen or hydroxy; R₂ is hydrogen, halogen, C₁₋₇ alkyl or azido; and R₄ is hydroxy, azido, C₁₋₇ alkyl, halogen or NR₉R₁₀.

Other preferred compounds of formula 1 are those wherein in R₁ or R₁ₐ all substituents are H except R₂ and R₄ are each hydrogen or hydroxy; R₃ is hydrogen, halogen, C₁₋₇ alkyl or azido; and R₅ is hydroxy, azido, C₁₋₇ alkyl, halogen or NR₉R₁₀.

Other preferred compounds of formula I are those wherein R₇ and R₈ taken together is O.

Other preferred compounds of formula I are those wherein X₁, X'₁, X2 and X'₂ are independently halogen (preferably fluoro).

Pharmaceutically acceptable salts and/or solvates of formula I compounds also comprise the present invention which further includes stereoisomers such as enantiomers which can arise as a consequence of structural asymmetry in selected compounds of formula I and anomers which arise from the R₁ substitution stereochemistry.

The compounds of this invention can exist in the form of pharmaceutically acceptable salts. Such salts include addition salts with inorganic acids such as, for example, hydrochloric acid and sulfuric acid, and with organic acids such as, for example, acetic acid, citric acid, methanesulfonic acid, toluene sulfonic acid, tartaric acid and maleic acid. Further, in case the compounds of this invention contain an acidic group, the acidic group can exist in the form of alkali metal salts such as, for example, a potassium salt and a sodium salt; alkaline earth metal salts such as, for example, a magnesium salt and a calcium salt; and salts with organic bases such as an ethylammonium salt and an arginine salt.

The compounds of the present invention are useful pharmacologic agents with anti-tumor properties. With Topoisomerase-I active properties, the compounds can be useful as anti-tumor agents. In recent years, numerous reports have surfaced the literature suggesting the role of Topoisomerase-I targeting drugs is to stabilize a covalent DNA-topoisomerase I complex to yield enzyme -linked DNA single-strand breaks. From a pharmacologic standpoint there are advantages to target Topoisomerase I; first relatively high levels of its presence in both proliferating and quiescent cells suggests that its function may be independent of cellular growth rate and second, topoisomerase I active agents may be effective in slow growing as well as rapidly proliferating tumors. Cells from colon tumors have been shown to contain higher intracellular levels of topoisomerase I than normal mucosal cells suggesting the possibility for selective cytotoxic advantage. Thus, inhibition of proliferation of tumor cells by compounds of formula I was initially demonstrated by effective inhibition of topoisomerase I. Selected compounds of formula I, usually having EC₅₀ values less than 10 µM in the Topoisomerase I assay, were also tested in an inhibition of human/mouse tumor cell proliferation assay. Compounds of this invention were also examined for their therapeutic effect in vivo against mouse tumor (P388) and the results are shown in the following pharmacological test examples (Table 1).

### In-Vivo Anti-Tumor Efficacy

In-vivo antitumor experiments were initiated by implanting mice (BDF1 or CDF1) ip with 10(6) P388 leukemia cells. Treatments were initiated one day after implant and involved one ip injection per dose level; several dose levels were evaluated per compound. Six mice were typically used per dose level in treatment groups, and eight to ten mice were used as parallel untreated leukemic controls. Evaluation of activity was made on the basis of comparing the median survival time (MST) of treated (T) mice with the MST of control (C) mice. Activity was defined as a %T/C of > or = 125%, calculated as: MST(T)/MST(C) x 100 = %T/C

**Table I**

| Compound Made By Example No. | Tumor | Dose(mg/kg/dose) | MST | T/C (%) |
|---|---|---|---|---|
| 19 | P388 | 200 | 9.0 | 82 |
| | | 100 | 20.0 | 182 |
| | | 50 | 18.5 | 168 |
| | | 25 | 17.0 | 155 |
| 29 | P388 | 200 | 10 | 91 |
| | | 100 | 16.5 | 150 |
| | | 50 | 12.0 | 109 |
| | | 25 | 14.0 | 127 |

### Topoisomerase I Activity (In-Vitro)

Topoisomerase I activity was measured as described below: The procedure for assaying compound induced, topoisomerase I mediated single strand break formation in DNA was essentially that described by Hsiang, et al., appearing in J. Biol. Chem. 260: 14873-14878 (1985). Samples dissolved in 100% DMSO as either 10 µM or 10 mg/ml solutions, unless otherwise stated, were diluted in Tris-EDTA buffer. Marine bacteriophage PM2 DNA (Boehringer Mannheim) was also diluted in Tris-EDTA buffer to a concentration of 0.02 µg/µl. Different dilutions of compound being evaluated were mixed with diluted DNA and this mixture was added to 1000 unit (one unit of enzyme activity is defined as the amount capable of relaxing 100 ng of supercoiled DNA in approximately 30 minutes at 37°C) aliquots of purified human topoisomerase I (Topogen) in 2X reaction buffer to start the reaction. The compound - DNA - enzyme mixture was incubated for 30 minutes at 37° C before stopping the reaction with warm stop buffer containing sodium dodecyl sulfate and proteinase K (Sigma). These mixtures were allowed to incubate at 37° C for another 10 minutes, at which time the mixtures were removed from the waterbath and extracted with a 24:1 mixture of chloroform/isoamyl alcohol. Following centrifugation, aliquots of the aqueous phases were placed in wells of a 0.9% agarose (SeaKem) gel in Tris-borate buffer containing 0.5 µg/ml of ethidium bromide and subjected to electrophoresis for 15 hours to separate the different topological isomers and nicked and broken DNAs. After destaining the gel in water, the ethidium bromide stained DNA reaction products were visualized by exposing the gel to UV irradiation. Negatives of photographs of the irradiated gels were scanned with a densitometer and areas under the peaks were calculated in order to obtain percent single strand DNA break formation for each sample. A median effective concentration(EC₅₀) was obtained for each compound by interpolation between points of the resulting dose - effect curve which defines the potency of the compound for its effect for inducing topoisomerase I mediated single strand breaks in DNA.

The topoisomerase I activity for selected compounds of the present invention is shown below in Table II.

**Table II**

| Example No | EC₅₀ (µM) |
|---|---|
| 15 | 0.03 |
| 18 | >100 |
| 19 | 0.04 |
| 29 | 0.01 |
| 30 | <0.01 |
| 31 | <0.01 |
| 33 | 0.23 |
| 34 | 023 |
| 35 | 0.75 |
| 45 | 0.28 |
| 47 | 0.10 |
| 48 | 0.40 |
| 68 | 0.03 |
| 69 | 0.03 |

The novel compounds of the present invention, as exemplified by amino and other sugar derivatives in Table II, show remarkable topoisomerase-I activity even in the sub-micro molar range of concentration. A priori, this activity, however, is unexpected and not predictable by one in the skilled art since a small change in substitution patterns seems to result in a highly unexpected change in activity. This is exemplified by the topoisomerase I activity difference for the compounds prepared by Examples 18 and 19. Example 19 is an effective anti-tumor agent with sub micro molar Topo-I activity whereas Example 18 did not show Topo-I activity even at greater than 100 micro molar concentration. The only difference between the compounds of Examples 18 and 19 is that in Example 18 X₁ and X'₁ is 2, 10-difluoro and R₆ is amino, whereas in Example 19 X₁ and X'₁ is 3,9-difluoro and R₆ is hydrogen. Moreover, rebeccamycin, where X₁ and X'₁ is 1, 11-dichloro, R₄ is methoxy and R₅ is hydroxy, is also not Topo-I active.

### In-Vitro Cell-Based Cytotoxicity Activity

The proliferation inhibition activity against human colon cell line was measured as follows.

Cytotoxicity was assessed in HCT116 human colon carcinoma cells by XTT (2,3-bis(2-methoxy-4-nitro- 5-sulfophenyl)-5-[(phenylamino)carbonyl]-2H-tetrazolium hydroxide assay as described in the literature by Scudiero, DA, Shoemaker, RH, Paull, KD, Monks, A, Tierney, S, Nofziger, TH, Currens, MJ, Seniff, D, and Boyd, MR. Evaluation of a soluble tetrazolium/formazan assay for cell growth and drug sensitivity in culture using human and other tumor cell lines, was done according to the procedure described in Cancer Res. 48: 4827-4833, 1988. Cells were plated at 4000 cells/well in 96 well microtiter plates and 24 hrs later drugs were added and serial diluted. The cells were incubated at 37° C for 72 hrs at which time the tetrazolium dye, XTT, containing phenazine methosulfate was added. A dehydrogenase enzyme in live cells reduces the XTT to a form that absorbs light at 450 run which can be quantitated spectrophotometrically. The greater the absorbance the greater the number of live cells. The results are expressed as an IC50 which is the drug concentration required to inhibit cell proliferation (i.e. absorbance at 450 nm) to 50% of that of untreated control cells.

The results for selected compounds of the present invention are shown in Table III.

**Table III**

| Example No | IC₅₀ (µM) |
|---|---|
| 15 | 0.26 |
| 18 | >1.45 |
| 19 | 0.11 |
| 29 | 0.09 |
| 33 | 0.17 |
| 35 | 0.50 |
| 45 | 0.37 |
| 58 | 0.07 |
| 60 | 0.17 |
| 62 | 0.56 |
| 63 | 0.77 |
| 67 | 0.74 |

Administration of a compound of Formula I or a pharmaceutically acceptable salt and/or solvate thereof, to a mammal implanted with tumor or susceptible to cancer formation, is now described. In general the compound would be given in a dose range of from about 0.01mg/kg to about the MTD (maximum tolerated dose). Although the dosage and dosage regimen and scheduling of a Formula I compound must in each case be carefully adjusted, utilizing sound professional judgment and considering the age, weight and condition of the recipient, the route of administration and the nature or extent of the cancer disease condition. The term "systemic administration" as used herein refers to oral sublingual, buccal, transnasal, transdermal, rectal, intramascular, intravenous, intraventricular, intrathecal, and subcutaneous routes. In accordance with good clinical practice, it is preferred to administer the instant compounds at a concentration level which will produce effective beneficial effects without causing any harmful or untoward side effects.

### DESCRIPTION OF THE SPECIFIC EMBODIMENTS

The procedure for the preparation of formula I compounds is illustrated in Scheme I, and the preparation of the key intermediates/starting materials is illustrated in Scheme II. 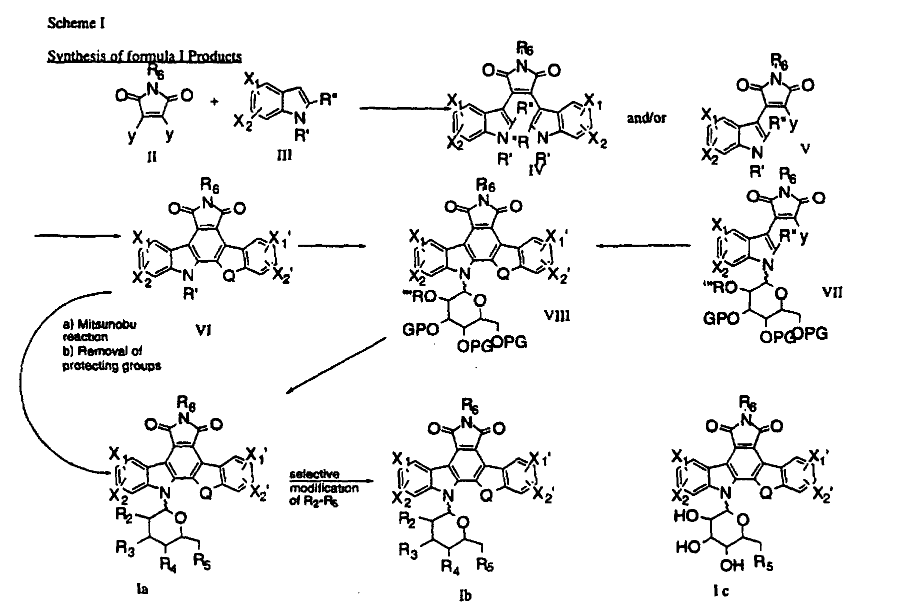 wherein:
y = Br or Cl;
R' = H;
R"' = H or a monosaccharide derivative;
R" = H or aryl or heteroaryl

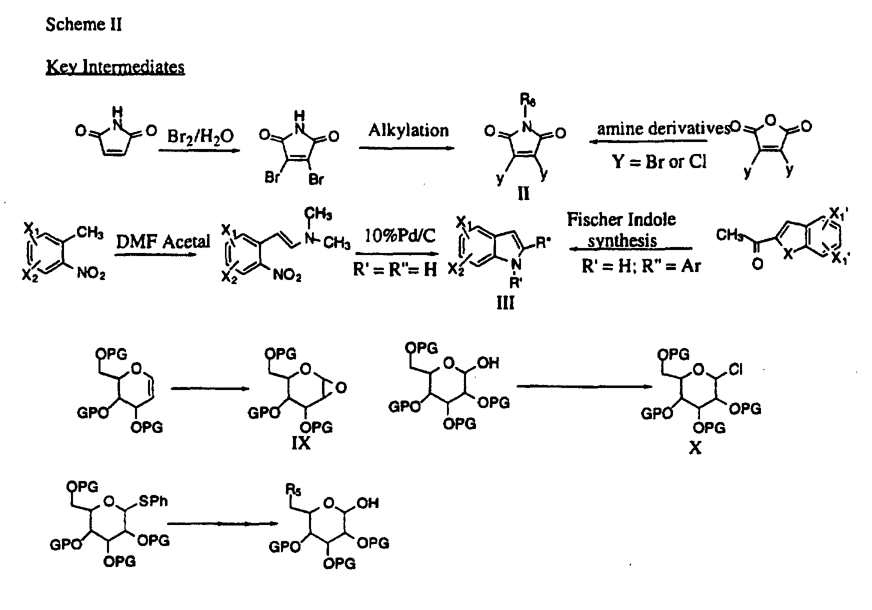

In schemes I and II, R₂ to R₆ X₁, X₂, X_{1'}, X_{2'} and Q are as defined supra. PG is a synthetic organic "protecting group" of the type generally used to "protect" an hydroxyl functionality e.g. an acyl type group such as a acetyl, trifluoroacetyl or an arylalkyl group like benzyl group or the like. Suitable "protecting" or "blocking" groups used in organic synthesis are well known to the practitioner and are adequately described in the appropriate literature. See, e.g. Theodora Greene, Protective Groups in Organic Synthesis, John Wiley and Sons, New York.
The starting materials in Scheme I are di-halo maleimide derivatives (II), such as 3, 4-dibromomaleimide; and the substituted indole derivatives of formula III. Addition of indole derivatives III (R" = H or Ar) to maleimide II in the presence of a base like ethylmagnesium bromide or the like in organic solvents like THF (i.e. tetrahydrofuran), benzene or toluene or combinations thereof at -20°C to reflux temperature would result in mono and bis derivatives V and IV respectively. The amount of reactants can be controlled to vary the product ratio of V vs IV to one's favor. Intermediate IV can be further transformed into indolopyrrolocarbazole core VI by using oxidative cyclization conditions such as Dicyanodichloroquinone (DDQ)/acid/heat or palladium acetate/acid or iodine, light and the like. Glycosylation of VI with reactive sugar derivatives like the 1,2-epoxide described in publications J. Org. Chem. 1993, 58, 343-349 and J. American Chemical Society 1989, 111, 6661-6666 or 1-halo and others in the presence of an appropriate base such as diisopropylethyl amine, hexamethyldisilazide, to form the mono- or di-or tri- anion of the indolopyrrolocarbazole core VI in organic solvent like THF, DMF (i.e. dimethylformamide), dioxane, benzene, DME (i.e. dimethoxyethane) produces the fully protected derivative VIII. Alternatively, more preferably, glycosylation may also be achieved by reacting the 1-hydroxy form of an appropriately protected sugar derivative with the core VI under the well known Mitsunobu procedure [PPh₃/ dialkyazidodicarboxylate] in ethereal solvent like THF or chlorinated solvent like CH₂Cl₂. Appropriate sugar derivative for the glycosylation may be obtained by selective modification of the different hydroxyl groups employing literature methods. For example, the article appearing in J. Carbohydrate Chemistry (1995), 14 (9), p1279-94 discloses the 6-halo-6-deoxy glucose derivative. Another method to obtain compound VIII involves glycosylation of the mono adduct first under the conditions described above to give the intermediate VII followed by dehydrohalogenation and cyclization using a number of well known method published in the art for such a transformation including heat or shining UV irradiation to a solution VII in solvents like Dioxane, Ethanol or an appropriate mixture of solvents. The compound VIII is a protected form of Formula I. Judicious choice of the protecting group in the sugar would allow selective manipulation of the primary hydroxyl group. For example, when the primary 6'-hydroxyl group was protected with the p-methoxybenzyl protecting group (PMB) and the rest of the hydroxyls were protected with simple benzyl groups (Bn), it is possible for one skilled in the art to remove the PMB group without deprotecting the benzyl group. In this manner, the primary 6-hydroxyl group is oxidized to give the corresponding acid, and their ester and amide derivatives. Furthermore, under controlled oxidation conditions using Dess-Martin reagent or the like, the 6-hydroxyl group is oxidized to the corresponding aldehyde. Treatment of the aldehyde with the well known fluorinating reagent like DAST resulted in the 6-difluoromethyl derivative. Similary, other hydroxyl groups were also modified. For example, when the 4-hydroxyl group of the galactose sugar derivative (hexose sugar in pyranose form in which the 4-hydroxyl is orienting the axial position) can be used to modify the 4-position. Oxidation of the 4-hydroxyl group of the appropriately protected sugar derivative furnished the ketone which could be further functionalized to different derivatives including the 4-difluro derivative employing the known procedures. If the 4-hydroxyl group is activated, for example in the case of 4-mesylate, it is succeptable for nucleophilic displacement by agents such as an azide (e.g. sodium azide) to provide 4-azido derivative. On the other hand, when the sugar derivative with the unprotected 4-hydroxyl group is treated with the fluorinating agent, DAST, one obtains the 4-fluoro derivative. Similarly, other hydroxyl groups are also selectively modified. For example modification of the published literature procedure allows for introduction of a fluorine atom at the 2-position of the sugar (Bioorg. Med. Chem., Vol. 5, No. 3, pp. 497-500 (1997). This is illustrated in example 90. Simple protecting group manipulation such as hydrogenation or transfer hydrogenation with Pearlman's catalyst to remove the benzyl protecting groups of the sugar moiety or if necessary, hydrolysis with KOH or NaOH of the maleimide nitrogen protecting group to give the anhydride after an acid workup followed by heating with an appropriate amine would produce the desired Formula Ia with the correct substitution pattern. Selective derivatization of each hydroxyl group in the sugar moiety of compound Ia may be achieved by using the protecting group manipulation and removal of the primary hydroxyl protecting group, in the presence of the secondary hydroxyl protecting groups. For example, when the compound of formula Ia, in a glucopyranosyl form (i.e. hexose formula (B)), wherein all the sugar hydroxyl groups are free, is treated with a silyl reagent like trimethysilyl or t-butyl-di-phenyl silyl or preferably t-butyl-di-methylsilyl triflate in the presence of a base in a solvent like CH₂Cl₂ or THF at cold or room temperature one obtains the derivative wherein the 3 and 6 positions are protected as silylethers. Selective deprotection of the 6 position silylether may be achieved under controlled aqueous mineral or organic acid conditions such as trifluoroacetic acid and water mixture as a solvent at cold temperature such as -25°C to 0°C over a period of 30 minutes to 3hrs or until the reaction is over as followed by thin layer chromatography. This intermediate is now set for further derivatization at the 6-position to activate the 6-hydroxyl to a good leaving group like mesylate or halide. Thus, methanesulfonyl or toluenesulfonyl or trifluoromethanesulfonyl chloride in the presence of a base like triethylamine or pyridine would produce the corresponding mesylate or tosylate. Alternatively, selective mesylation of the primary 6-hydroxyl group may also be achieved directly from compound of Formula Ia wherein all sugar hydroxyl groups are unprotected under pyridine and mesyl chloride in pyridine at 0°C. Nucleophilic displacement with an appropriate amine or other nucleophiles like azide followed by reduction of the azide to amine would then produce desired compounds of formula I such as 6' amino sugar derivatives I b. In this manner, the 6-methyl sulfide derivative was prepared employing either the sodium salt of the thiol derivative or the thiol and a base like K2CO3 in DMF or an organic amine base like triethylamine or Hunig's base at varying temperatures from room temperature to 150°C. The 6-alkyl sulfide, for example 6-methyl sulfide, is further oxidized by known oxidizing agents like Oxone or m-chloroperbenzoic acid or, preferably, monoperoxyphthalic acid magnesium salt (MMPP) to their sulfoxides and sulfone under controlled conditions. Similarly, other hydroxyl groups may also be derivatized as desired. Yet another modification of the formula I would result in N-maleimide substituted (i.e. R₆) indolopyrrolocarbazole derivatives Ic. For example, the anhydride obtained from the base hydrolysis of the appropriately protected form of Formula I may be reacted with a number of amine derivatives to produce the desired N-substituted maleimides.

As depicted in Scheme II, the N-substituted maleimide derivative may be obtained either directly from the dihalomaleic anhydride upon treatment with an appropriate amine derivative or halogenation of maleimide followed by alkylation. The starting indole derivatives which have no substitution at the 2-position may be obtained by following published procedures and 2-aryl indoles may be prepared from the aryl methyl ketones following the well known Fischer Indole synthetic procedures.

The compounds which constitute this invention and their methods of preparation will appear more fully from a consideration of the following examples.

Several intermediate compounds as well as other conventional starting materials, e.g. II, III and IX; used in the preparation of final products I were generally commercially available. Representative syntheses of some of the final compounds of formula I (wherein in R₁ all substituents are hydrogen unless otherwise mentioned) are provided herein below. The syntheses of some of the intermediates are also provided such as in examples 1-11, 14, 91-96, 98-102, 104 and 105.

All anhydrous reactions were performed under an atmosphere of nitrogen or argon using dry solvents from Aldrich Sure Seal bottles or freshly distilled solvents. Column chromatography was performed with silica gel 60 (E M Science, 230-400 mesh) using the mentioned solvent system as eluant. Thin-layer chromatography was conducted on Anatech GFLH or Whatman MK6F silica gel plates . Melting points were determined in an open capillary tube with a Thomas-Hoover melting point apparatus unless otherwise stated and are not corrected. Infared spectra were recorded on a Perkin-Elmer 1800 Fourier transform spectrophotometer as thin films or KBr pellets. ¹H NMR spectra and ¹³C NMR spectra were recorded on either Bruker AM-300 or JEOL 300 or Bruker AC-300 or 500 MH_{z} NMR instruments and are expressed as parts per million (ppm or δ) from the mentioned solvent as the internal standard. Coupling constants are in hertz and signals are quoted as apparent singlet (s), triplet (t), quartet (q), muliplet (m), and broad (br). Low resolution mass spectra were determined either on a Finnigan Model 4500 Quadrapole mass spectrometer by direct chemical ionization (DCI) with isobutane as the positive CI gas, a Finnigan Model SSQ-7000 instrument (neg. or pos. ESI) or on a Kratos MS-25 or Finnigan TSQ-70 instrument (FAB). High resolution mass spectra (HRMS) were determined either on a Kratos MS-50 mass spectrometer using fast atom bombardment with CsI in glycerol as the reference agent or on a Finnigan MAT-900 instrument using electrospray ionization with polypropylene glycol as the reference agent.

### Example 1

### 3,4-Dibromomaleimide (II ; R₆ = H, y=Br).

To a magnetically stirred solution of maleimide (25.0 g, 0.258 mol) in deionized water (250 mL) was rapidly added bromine (100 g, 0.626 mol) followed by benzoyl peroxide (300 mg). The reaction mixture was heated to 50° C for 6.5 h, and then stirred at room temperature for 11.5 h. The reaction mixture was cooled in an ice/water bath for 45 min, and the solid precipitate was filtered off, washed with water, and air dried to give 36.35 g (55.4%) of the title compound as a white solid, 75 MHz ¹³C NMR (d6-acetone): δ 165.08, 130.73; FAB mass spectrum, m/e 253 (M⁺); Anal. Calcd for C₄HBr₂NO₂: C, 38.30; H, 2.05; N, 4.06; Br 46.32. Found: C, 38.28; H, 2.06; N, 4.07; Br, 46.24.

### Example 2

### 1-(tert-Butylbenzyl)-3,4-dibromomaleimide (II; R₆=tert-butylbenzyl, y=Br).

To a magnetically stirred solution of 3,4-dibromomaleimide (20.0 g, 78.5 mmol) in acetone (1200 mL) was added potassium carbonate (132 g, 0.954 mol). 4-(t-Butyl)benzyl bromide (24.98 g, 20.2 mL, 110 mmol) was slowly added over 15 min and the reaction mixture was then stirred for 6 h in the dark. The reaction mixture was filtered over a bed of Celite and washed with acetone (250 mL). Evaporation *in vacuo,* followed by flash chromatography on silica gel with 50% hexane in dichloromethane, gave 22.5 g (71.5%) of the title compound as a white solid. 300 MHz ¹H NMR (CDCl₃) δ 7.45-7.25 (m, 4 H), 4.75 (s, 2 H), 1.32 (s, 9 H); DCI mass spectrum, m/e 399 (M⁺); Anal. Calcd for C₁₅H₁₅Br₂NO₂: C, 44.92; H, 3.77; N, 3.49. Found: C, 45.04; H, 3.81; N, 3.38.

### Example 3

### 2-(2-Benzo[b]thienyl)-5-fluoro-1H-indole (III : X₁ = 5F; X₂ = H; R"= 2-benzo[b]thienyl, R'=H)

n-Butyllithium in hexanes (1.6M, 255mL, 0.41 mol) was added in 5 mL portions to a cold (4°C) solution of thianaphthene (47.8g, 0.36 mol) in dry tetrahydrofuran/diethyl ether (1:1, 400 mL) under an atmosphere of nitrogen. The alkyllithium was added at a such a rate that the internal reaction temperature never exceeded 8°C. After the addition was complete, the reaction mixture was allowed to warm up to ambient temperature where it stirred for 1h before it was cooled back to 4°C and treated cautiously with a solution of acetaldehyde (50 mL) in dry tetrahydrofuran (50 mL). The mixture was allowed to warm up to room temperature for 0.5h before it was quenched with water and diluted with ethyl acetate. The organic phase was separated, washed with brine, dried and concentrated. The aqueous phase was extracted twice more with ethyl acetate and combined with the original organic extract prior to further workup. Purification of the resultant residue by flash chromatography on silica gel (gradient elution with 7% ethyl acetate in hexanes followed by 15% ethyl acetate in hexanes) afforded 29.25g (46%) of the intermediate alcohol as a pale yellow solid which was carried on directly. The alcohol (29.15g, 0.16 mol) was dissolved in anhydrous dichloromethane (800 mL) and the reaction mixture was
treated with Celite (36g) and pyridinium chlorochromate (35g). After one hour at room temperature, additional dichloromethane (400 mL), Celite (36g) and PCC (35g) were added. The mixture was stirred further for an additional 1h at room temperature before it was diluted with ether (1L), suction-filtered through a bed of silica gel and concentrated. There was isolated the methyl ketone (27.6g, 96%) as a pure, white solid. Anhydrous sodium acetate (16.6g, 0.20 mol) was added in one portion to a stirred suspension of 4-fluorophenylhydrazine hydrochloride (32.98g, 0.20 mol) and the methyl ketone (27.5g, 0.156 mol) in absolute ethanol (150 mL). The mixture was refluxed for 2h before it was cooled, diluted with dichloromethane, and washed with saturated sodium bicarbonate solution, IN hydrochloric acid and brine. Following drying and solvent evaporation, the residue was recrystallized from hot ethanol to furnish the pure hydrazone (40.08g, 90%) as a yellow solid. The hydrazone (18.3g, 64.4 mmol) was placed into a 500 mL single neck round bottom flask (equipped with a reflux condenser) which contained freshly-fused zinc chloride held under a nitrogen atmosphere. The flask was then placed in a preheated oil bath (180°C) for 1h. After 1h, the oil bath temperature was allowed to cool to 140°C before the flask was charged cautiously with absolute ethanol. The mixture was refluxed for 6h before it was cooled, diluted with ethyl acetate and washed with IN hydrochloric acid and brine prior to drying and solvent concentration to 1/3 volume. Suction-filtration afforded the title compound (22.4g, 65%) as a white solid, m.p. 263-264°C ; ¹H NMR (300 MHz, DMSO-d₆) δ 11.91 (s, 1H), 7.99-7.96 (m, 1H), 7.89-7.86 (m, 2H), 7.83 (s, 1H), 7.43-7.29 (series of m, 4H), 7.03-6.96 (m, 1H), 6.81 (d, *J*=1.5 Hz, 1H); ¹³C NMR (75 MHz, DMSO-d₆) ppm 158.79, 155.72, 140.01, 138.35, 134.92, 133.92, 133.79, 128.64, 128.50, 124.93, 124.79, 123.70, 122.44, 119.73, 112.35, 112.22, 110.76, 110.41, 104.90, 104.59, 100.90, 100.84; IR (KBr, cm⁻¹) 3421, 1625, 1586, 1567, 1501, 1448, 1412, 1286, 1201, 1188, 1128, 862, 825, 783, 744, 725, 559, 515; MS (neg. ESI, M-H-) *m/z* 266. *Anal.* Calc'd for C₁₆H₁₀FNS: C, 71.89; H, 3.77; N, 5.24. Found: C, 71.82; H, 3.76; N, 5.13.

### Example 4

### (E)-4-Fluoro-2-nitro-β-dimethylaminostyrene.

A mixture of 4-fluoro-2-nitrotoluene (185.0g, 1.19 mol) and N,N-dimethylformamide dimethyl acetal (500mL, 3.77 mol) in dry dimethylformamide was refluxed for 2h under nitrogen prior to azeotropic distillation of methanol from the reaction mixture. Another reaction (169.2g of 4-fluoro-2-nitrotoluene) was performed in tandem. There was isolated a combined weight of 450g (95%) of the title compound as a red solid (solidifies on cooling) which was sufficiently pure to be carried forward. Kugelrohr distillation afforded the title compound (analytically pure for characterization purposes) as a reddish-black crystalline solid, m.p. 54-55°C; ¹H NMR (300 MHz, DMSO-d₆) δ 7.72-7.62 (m, 2H), 7.37-7.32 (m, 1H), 7.33 (d, *J*=13.4 Hz, 1H), 5.58 (d, *J*=13.4 Hz, 1H), 2.58 (s, 6H); ¹³C NMR (75 MHz, DMSO-d₆) ppm 158.19, 154.99, 146.16, 143.13, 143.02, 132.57, 132.54, 126.14, 126.04, 120.98, 120.69, 111.41, 111.07, 88.23; IR (KBr, cm⁻¹) 3446, 1622, 1570, 1508, 1386, 1270, 1092, 940, 822, 798; MS (MH⁺) *m*/*z* 211. *Anal.* Calc'd for C₁₀H₁₁FN₂O₂: C, 57.14; H, 5.27; N, 13.33. Found: C, 57.09; H, 5.16; N, 13.46.

### Example 5

### 6-Fluoroindole (III: X₁ = F, X₂ = R"= R' = H).

(E)-4-Fluoro-2-nitro-β-dimethylaminostyrene (120g, 0.5 mol) was dissolved in tetrahydrofuran (1L) and subjected to Parr hydrogenation (50 psi H₂, room temperature, 24h) using 10% palladium on carbon (30g). The mixture was filtered through Celite (washed with THF, methanol and methylene chloride) and concentrated down to dryness. This reaction was done three additional times in order to consume the starting material mentioned above. Steam distillation of the residue afforded the title compound (192.6g, 62%) as white needles, m.p. 73-75°C; ¹H NMR (300 MHz, DMSO-d₆) δ 11.14 (br s, 1H), 7.51 (dd, *J*=8.6, 5.5 Hz, 1H), 7.32 (t, *J*= 2.9 Hz, 1H), 7.16 (dd, *J*=10.1, 2.3 Hz, 1H), 6.87-6.80 (m, 1H), 6.43-6.41 (m, 1H); ¹³C NMR (75 MHz, DMSO-d₆) ppm 160.26, 157.16, 135.79, 135.63, 125.94, 125.89, 124.42, 120.97, 120.83, 107.43, 107.10, 101.17, 97.47, 97.13; IR (KBr, cm⁻¹) 3392, 3072, 1626, 1508, 1448, 1342, 1144, 954, 846, 802, 728, 508; MS (MH⁺) *m/z* 136. *Anal.* Calc'd for C₈H₆FN: C, 71.10; H, 4.47; N, 10.36. Found: C, 71.28; H, 4.69; N, 10.24.

### Example 6

### 3,4-bis(5-Fluoro-1H-indol-3-yl)-N-[4-(t-butyl)benzyl]-pyrrole-2,5-dione (IV: X₁=F; X₂=R'=R"= H; R₆ =4-(t-butyl)benzyl).

To a magnetically stirred solution of 5-fluoroindole (7.0 g, 51.8 mmol) in anhydrous benzene (125 mL) was added under argon with stirring, methyl magnesium iodide (3.0 M in ether; 18.0 mL, 54.0 mmol). The mixture was stirred at room temperature for 30 min and then this solution was added via cannula over 10 min to a rapidly stirred solution of N-[4-(t-butyl)benzyl]-3,4-dibromomaleimide (6.50 g, 16.2 mmol) in anhydrous benzene (60 mL). The resulting dark purple solution was stirred at room temperature under argon for 16 h, and poured into a mixture of 20% aqueous citric acid (350 mL) and ethyl acetate (500 mL). The organic layer was washed with water (200 mL) and brine (200 mL), and dried (Na₂SO₄). Evaporation *in vacuo,* followed by flash chromatography on silica gel with 100% dichloromethane, followed by 10% ethyl acetate in dichloromethane, gave 3.68 g (44%) of the title compound as a red solid. 300 MHz ¹H NMR (CDCl₃) δ 8.63 (brs, 2H), 7.87 (d, 2H, J = 2.9 Hz), 7.46-7.34 (m, 4H), 7.23 (dd, 2H, J = 8.9, 4.4 Hz), 6.79 (ddd, 2H, J = 9.1, 8.9, 2.5 Hz), 6.48 (dd, 2H, J = 10.2, 2.5 Hz), 4.83 (s, 2H), 1.30 (s, 9H); 75 MHz ¹³C NMR (CDCl₃) δ 172.15 (s), 157.73 (d, J = 235 Hz), 150.82 (s), 133.79 (s), 132.27 (s), 129.92 (s), 128.32 (s), 127.14 (s), 126.16 (d, J = 10.4 Hz), 125.72 (s), 112.19 (d, J = 9.8 Hz), 111.10 (d, J = 26.6 Hz), 106.95 (d, J = 4.3 Hz), 106.43 (d, J = 25.1 Hz), 41.68 (s), 34.55 (s), 31.33 (s); FAB mass spectrum, m/e 509 (M⁺).

### Example 7

### 3-Bromo-4-(6-fluoro-1H-indol-3-yl)-1H-pyrrole-2,5-dione (V:X₁=6F; R'= R"=R₆=H; y=Br) and 3,4-bis(6-fluoro-1H-indol-3-yl)-1H-pyrrole-2,5-dione (IV: X₁=F; X₂=R'= R"=R₆=H).

To a solution of 6-fluoroindole (50.0g, 0.37 mol) in anhydrous benzene (1L) under nitrogen was added via syringe ethylmagnesium bromide (3M, 130 mL, 0.43 mol) at a rate which kept the internal temperature between 45-50°C. The mixture was then heated to 50-55°C for 0.5h before a suspension of 2,3-dibromomaleimide (24.9g, 0.093 mol) in anhydrous benzene was poured into the reaction mixture. The mixture was refluxed for 22h, cooled, diluted with ethyl acetate, and acidified to pH=1 with 1N HCI. The organic phase was then separated, washed with brine, dried and concentrated. The aqueus phase was diluted once more with ethyl acetate and separated. The resulting organic phase was treated in identical fashion as mentioned above and combined with the original. Another 50g reaction was performed and combined prior to chromatography. Purification of the combined residues by flash chromatagraphy on silica gel (gradient elution with 10% ethyl acetate in hexanes followed by 20% and finally 40% ethyl acetate in hexane) afforded two major products. There was isolated title compound of formulaV (12.31g, 21.5%) as a brick-red solid and title compound of formula IV (31.5g, 41%) as a reddish-orange foam.

For the title compound of formula V: M.p. 73-75°C; ¹H NMR (300 MHz, DMSO-d₆) δ 11.14 (br s, 1H), 7.51 (dd, *J*=8.6, 5.5 Hz, 1H), 7.32 (t, *J*=2.9 Hz, 1H), 7.16 (dd, *J*=10.1, 2.3 Hz, 1H), 6.87-6.80 (m, 1H), 6.43-6.41 (m, 1H); ¹³C NMR (75 MHz, DMSO-d₆) ppm 160.26, 157.16, 135.79, 135.63, 125.94, 125.89, 124.42, 120.97, 120.83, 107.43, 107.10, 101.17, 97.47, 97.13; IR (KBr, cm⁻¹) 3392, 3072, 1626, 1508, 1448, 1342, 1144, 954, 846, 802, 728, 508; MS (MH⁺) *m/z* 136. *Anal.* Calc'd for C₈H₆FN: C, 71.10; H, 4.47; N, 10.36. Found: C, 71.28; H, 4.69; N, 10.24.

For the title compound of formula IV: M.p. 207-208°C (dec.); ¹H NMR (300 MHz, DMSO-d₆) δ 12.10 (s, 1H), 11.37 (s, 1H), 8.03 (d, *J*=2.9 Hz, 1H), 7.86 (dd, *J*=8.9, 5.4, 2.3 Hz, 1H), 7.28 (dd, *J*=9.6, 2.3 Hz, 1H), 7.00 (dt, *J*=9.3, 2.3 Hz, 1H); ¹³C NMR (75 MHz, DMSO-d₆) ppm 170.12, 167.35, 160.70, 157.56, 137.65, 136.62, 136.45, 131.58, 131.56, 123.51, 123.38, 121.27, 115.40, 109.03, 108.70, 103.89, 98.47, 98.14; IR (KBr, cm⁻¹) 3328, 3222, 1726, 1604, 1450, 1338, 1240, 1192, 1148, 840, 794; MS (MH⁺) *m*/*z* 309, 311. *Anal.* Calc'd for C₁₂H₆BrFN₂O₂: C, 46.63; H, 1.96; N, 9.06. Found: C, 46.70; H, 2.00; N, 8.94.

### Example 8

### 3,9-Difluoro-12,13-dihydro-5H-indolo[2,3-a]pyrrolo[3,4-c]carbazole-5,7(6-N-[4-(t-butyl)benzyl]-)-dione (VI: X₁=X_{1'}=F; X₂=X_{2'}=R'= H; R₆ =4-(t-butyl)benzyl; Q=NH).

To a magnetically stirred solution of the product from Example 6 (11.6 g, 22.8 mmol) in anhydrous benzene (1700 mL) was added under argon with stirring, p-toluenesulfonic acid monohydrate (170 mg) and DDQ (10.9 g, 48.0 mmol). The mixture was refluxed under argon with stirring for 1 h, and stirred at room temperature for 16 h. The resulting dark precipitate was collected by filtration, washed with cold ethyl acetate (50-70 mL), and dried for 1-2 h *in vacuo* to give 12.80 g (94%) of the title compound as a yellow-brown solid (mono ethyl acetate complex by proton NMR; further drying *in vacuo* can reduce ethyl acetate content by one-half to two thirds ). Recrystallization from THF/ethyl acetate gives the analytically pure product as a bright yellow solid (mono ethyl acetate complex by proton NMR). 300 MHz ¹H NMR (d6-DMSO) δ 11.83 (brs, 2H), 8.46 (dd, 2H, J = 9.7, 2.6 Hz), 7.69 (dd, 2H, J = 8.9, 4.6 Hz), 7.36 (ddd, 2H, J = 9.1, 8.9, 2.6 Hz), 7.34-7.22 (m, 4H), 4.63 (s, 2H), 1.22 (s, 9H); FAB mass spectrum, m/e 507 (M⁺); Anal. Calcd for C₃₁H₂₃F₂N₃O₂·C4H₈0₂: C, 70.58; H, 5.25; F, 6.38; N, 7.06. Found: C, 70.52; H, 5.31; F, 6.14; N, 7.00.

### Example 9

### 2,10-Difluoro-12,13-dihydro-5H-indolo[2,3-a]pyrrolo[3,4-c]carbazole-5,7(6H)-dione (VI: X₁= X_{1'}=F; X₂=X_{2'}=R'=R₆=H; Q=NH).

2,3-Dichloro-5,6-dicyano-1,4-benzoquinone (15.81g, 0.70 mol) was added in one portion to a stirred suspension of 3,4-bis(6-fluoro-1H-indol-3-yl)-1H-pyrrole-2,5-dione (11.5g, 0.032 mol) and toluenesulfonic acid monohydrate (0.44g) in benzene (1L) under nitrogen and the mixture was refluxed for 2h before it was cooled, stored at room temperature for 16h, and suction-filtered through a sintered glass funnel. The precipitate was washed successively with benzene, ethyl acetate and ether until a colorless filtrate was observed. Another 20.0g reaction was performed in tandem using proportionally the same amount of reagents used in the first reaction. There was isolated, after consolidation, the title compound (15.51g, 40%) as a yellowish-green solid, m.p. >305°C; ¹H NMR (300 MHz, DMSO-d₆) δ 11.67 (br s, 2H), 10.93 (br s, 1H), 8.84 (dd, *J*=8.8, 5.8 Hz, 2H), 7.55 (dd, *J*=10.0, 2.3 Hz, 2H), 7.11 (dt, *J*=9.2, 2.3 Hz, 2H), 4.00 (q, *J*=7.1 Hz, 2H), 1.96 (s, 3H), 1.15 (t, *J*=7.1 Hz, 3H); ¹³C NMR (75 MHz, DMSO-d₆) ppm 171.09, 170.32, 163.27, 160.08, 141.00, 140.83, 129.25, 125.69, 125.55, 119.54, 118.23, 115.09, 108.51, 108.19, 98.64, 98.28, 59.74, 20.74, 14.07; IR (KBr, cm⁻¹) 3320, 1748, 1690, 1572, 1404, 1376, 1312, 1232, 1144, 1116, 840; MS (M⁺) *m*/*z* 361. *Anal.* Calc'd for C₂₀H₉F₂N₃O2•1.0EtOAc •0.1H₂O: C, 63.89; H, 3.84; N, 9.31. Found: C, 64.12; H, 3.76; N, 9.55.

### Example 10

### 3,9-Difluoro-12,13-dihydro-13-[3,4,6-tris-O-(phenylmethyl)-β-D-glucopyranosyl]-5H-indolo[2,3-a]pyrrolo[3,4-c]carbazole-5,7(6N-[4-(t-butyl)benzyl]-)-dione (VIII: X₁=X_{1'}=F; X₂=X_{2'}=R'=R"'= H; R₆ =4-(t-butyl)benzyl; Q=NH; PG = benzyl).

To a magnetically stirred suspension of core from Example 8 (9.50 g, 17.7 mmol) in anhydrous THF (450 mL) was added over 5-10 min under argon with stirring, sodium bis(trimethylsilyl)amide (1.0 M in THF; 50.0 mL, 2.82 equiv). The resulting deep red solution was stirred at room temperature for 45 min, treated neat via syringe with chlorotrimethylsilane (5.06 mL, 40.0 mmol), and stirred at room temperature for a further 1 h. The reaction mixture was then heated to reflux, and maintained at reflux while a solution of the 1,2-anhydrosugar IX (13.5 g, 31.2 mmol; see J. Org. Chem. **1993,** 58, 343-349) in anhydrous THF (200 mL) was added over 3.5 h via a constant addition funnel. After the addition was completed, the reaction was refluxed for 10 h under argon, treated with a solution of additional 1,2-anhydrosugar IX (800 mg) in anhydrous THF (40 mL) and refluxed further for 2.5 h. The mixture was cooled to room temperature over 45 min, treated with HCl (1.0 N, 280 mL), and stirred for 75 min. The mixture was partitioned between ethyl acetate (2000 mL) and 0.5 *N* HCl (600 mL). The organic layer was washed with saturated aqueous sodium bicarbonate (300 mL), water (350 mL), and brine (400 mL). The original acidic aqueous layer was extracted with fresh ethyl acetate (600 mL) which was then washed with water (100 mL), and brine (150mL). The combined organic extracts were dried (Na₂SO₄) and rotary evaporated to a volume of about 300 mL, whereupon the resulting yellow precipitate was collected by filtration, washed with ethyl acetate (300 mL), and dried *in vacuo* to give 3.00 g (31.6%) of recovered pure starting core. The filtrate was concentrated *in vacuo,* dissolved in methylene chloride/hexane, and purified by flash chromatography on silica gel using 5-10% ethyl acetate in hexanes. The yellow fractions containing the least polar spot were evaporated to a volume of about 300 mL and let stand overnight to yield 625mg (3.8%) of beautiful yellow prisms of N₁₂, N₁₃-doubly glycosylated product (structure determined by X-RAY crystalography). An additional 380 mg (2.3%) of the same was obtained from the mother liquor. Further elution with 10-15% ethyl acetate in hexanes gave 9.23 g (55.5%, 81% based on recovered starting core) of analytically pure title compound of formula VIII as a yellow solid. 500 MHz ¹H NMR (d6-DMSO) δ 10.85 (brs, 1H), 8.84 (dd, 1H, J = 9.6, 2.7 Hz), 8.75 (dd, 1H, J = 9.6,2.5 Hz), 7.54 (ddd, 1H, J = 9.1, 9.1, 2.7 Hz), 7.42-7.18 (m, 21H), 6.43 (d, 1H, J = 8.8 Hz, 1'H), 5.38 (d, 1H, J = 6.3 Hz, 2'OH), 4.94-4.56 (m, 8H), 4.30-4.15 (m, 2H), 3.98-3.72 (m, 4H), 1.24 (s, 9H); FAB mass spectrum, m/e 939 (M⁺); Anal. Calcd for C₅₈H₅₁F₂N₃O₇: C, 74.11; H, 5.47; F, 4.04; N, 4.47. Found: C, 73.89; H, 5.51; F, 3.77; N, 4.26.

### Example 11

### 2,10-Difluoro-12,13-dihydro-13-[3,4,6-tris-O-(phenylmethyl)-β-D-glucopyranosyl]-5H-indolo[2,3-a]pyrrolo[3,4-c]carbazole-5,7(6H)-dione (A=VIII: X₁= X_{1'}=F; X₂=X_{2'}=R₆=R"'=H; Q=NH; PG=benzyl) and 2,10-difluoro-12,13-dihydro-13-[2-O-[3,4,6-tris-O-(phenylmethyl)-β-D-glucopyranosyl]-3,4,6-tris-O-(phenylmethyl)-β-D-glucopyranosyl]-5H-indolo[2,3-a]pyrrolo[3,4-c]carbazole-5,7(6H)-dione (B =VIII: X1= X1'=F; X2=X2'=R6= H; Q=NH; PG=benzyl; R"'= 3,4,6-tris-O-(phenylmethyl)-β-D-glucopyranosyl).

A solution of sodium bis(trimethylsilyl)amide (1*M*, 9.1 mL, 3.3 eq.) in tetrahydrofuran was added via syringe to a solution of 2,10-difluoro-12,13-dihydro-5H-indolo[2,3-a]pyrrolo[3,4-c]carbazole-5,7(6H)-dione (11.17g, 32.38 mmol) in anhydrous tetrahydrofuran (600 mL) at room temperature. After 25 min, a solution of epoxide (24.27g, 56.11 mmol, 1.7 eq.) in anhydrous THF (50 mL) was canulated dropwise into the reaction mixture. The mixture was refluxed for 6h before it was quenched with saturated ammonium chloride solution and diluted with ethyl acetate. The organic phase was separated, washed with brine, dried and evaporated. Purification of the residue by flash chromatography on silica gel (gradient elution with 15% tetrahydrofuran in hexanes followed by 20% and 40% tetrahydrofuran in hexanes) furnished the title compound **A** of formula VIII (6.1g, 30%) as a yellow foam, unreacted aglcone (4.00g) and the bis-sugar compound **B** (10.9g, 34%; MS(M⁺) calc'd for C₁₄H₆₆F₂N₃O₁₂ 1226.4615, obsd 1226.4566) as a yellow foam which was converted directly to the title compound **A** of formula VIII as described below. For title compound of formula **A**=VIII: M.p. 140-147°C; ¹H NMR (300 MHz, CDCl₃) δ 10.50 (s, 1H), 8.79-8.74 (dd, *J*=8.8, 5.8 Hz, 1H), 8.21 (dd, *J*=8.8, 5.6 Hz, 1H), 7.59-7.33 (m, 11H), 7.28-7.12 (2m,11H), 7.06-7.00 (m, 1H), 6.81 (dd, *J*=9.1, 2.2 Hz, 1H), 6.61-6.58 (m, 1H), 5.83 (d, *J*=9.0 Hz, 1H), 5.12 (d, *J*=5.3 Hz, 1H), 5.08 (d, *J*=5.2 Hz, 1H), 5.01-4.98 (m, 1H), 4.85 (d, *J*=10.8 Hz, 1H), 4.73-4.72 (m, 1H), 4.55-4.35 (m, 4H), 4.07-3.93 (m, 3H), 3.76-3.72 (m, 1H); ¹³C NMR (75 MHz, CDCl₃) ppm 169.55, 169.10, 164.43, 164.25, 161.18, 161.00, 143.00, 142.83, 141.68, 141.50, 137.69, 137.59, 136.32, 130.45, 128.91, 128.74, 128.63, 128.56, 128.43, 128.06, 127.02, 126.89, 126.15, 126.01, 120.00, 119.01, 118.44, 118.17, 118.09, 110.33, 110.02, 109.48, 109.16, 98.04, 97.70, 97.34, 85.89, 85.62, 76.35, 75.36, 75.05, 74.09, 73.74, 66.75; IR (KBr, cm⁻¹) 3430, 3334, 2914, 2870, 1752, 1702, 1580, 1452, 1328, 1232, 1140, 1062, 698; MS (M⁺) calc'd for C₄₇H₃₈F₂N₃O₇ 794.2678, obsd 794.2687. *Anal.* Calc'd for C₄₇H₃₇F₂N₃O₇•1.0H₂O: C, 69.54; H, 4.84; N, 5.17; H₂O, 2.2. Found: C, 69.57; H, 4.70; N, 5.10; H₂O, 0.4.

Compound **B** is converted to compound **A** by stirring a solution of 2,10-difluoro-12,13-dihydro-13-[2-O-[3,4,6-tris-O-(phenylmethyl)-β-D-glucopyranosyl]-3,4,6-tris-O-(phenylmethyl)-β-D-gluco-pyranosyl]-5H-indolo[2,3-a]pyrrolo[3,4-c]carbazole-5,7(6H)-dione (10.9g, 8.89 mmol) in dry ethyl acetate (25 mL) and treating with a solution of anhydrous methanolic hydrogen chloride (8*M*, 500 mL) in a sealed flask for 96h at room temperature. The solvent(s) were then removed *in vacuo* and the resulting residue was diluted with ethyl acetate and tetrahydrofuran and neutralized to pH=8 with saturated sodium bicarbonate solution. The organic phase was separated and washed with brine prior to drying and solvent concentration. Purification of the residue by flash chromatography on silica gel (as described above) yielded title compound **A** of formula VIII (5.7g, 81%) as a yellow foam, as well as the aglycone starting material (0.7g).

### Example 12

### 3,9-Difluoro-12,13-dihydro-13-[β-D-glucopyranosyl]-5H-indolo[2,3-a]pyrrolo [3,4-c] carbazole-5,7(6N-[4-(t-butyl)benzyl]-)-dione (Ia: X₁ =X_{1'}=F; X₂=X_{2'}=H; R₆ =4-(t-butyl)benzyl; Q=NH; R₂-R₅=OH).

To a magnetically stirred solution of compound from example 10 (2.10 g, 2.23 mmol) in 95% ethanol (450 mL) and cyclohexene (175 mL) was added 20% palladium hydroxide on carbon (0.49 g) and the mixture was refluxed under nitrogen with vigorous stirring for 20 h. The mixture was filtered hot through Celite and washed with methanol. The filtrate was concentrated *in vacuo* and purified by flash chromatography on silica gel using 3-5% methanol in methylene chloride to give 1.35 g (90%) of the pure title compound as a yellow-orange solid. The analytical sample was evaporated from ethyl acetate, dried *in vacuo,* and obtained as 1/2 EtOAc complex per molecule. 300 MHz ¹H NMR (d6-DMSO) δ 11.81 (brs, 1H), 8.84 (dd, 1H, J = 9.7, 2.7 Hz), 8.76 (dd, 1H, J = 9.7, 2.6 Hz), 8.04 (dd, 1H, J = 9.3, 4.4 Hz), 7.70 (dd, 1H, J = 8.9, 4.6 Hz), 7.55-7.46 (m, 2H), 7.40-7.33 (m, 4H), 6.31 (d, 1H, J = 8.9 Hz), 6.12 (brs, 1H), 5.43 (d, 1H, J = 4.4 Hz), 5.17 (d, 1H, J = 5.5 Hz), 4.93 (d, 1H, J = 5.4 Hz), 4.90 (s, 2H), 4.13-4.07 (m, 1H), 4.00-3.96 (m, 2H), 3.87-3.80 (m, 1H), 3.63-3.46 (m, 2H), 1.24 (s, 9H); FAB mass spectrum, m/e 669 (M⁺); Anal. Calcd for C₃₇H₃₃F₂N₃O₇ · 1/2 C₄H₈O₂: C, 65.63; H, 5.22; F, 5.32; N, 5.89. Found: C, 65.04; H, 5.20; F, 5.32; N, 5.91.

### Example 13

### 2,10-Difluoro-12,13-dihydro-13-[β-D-glucopyranosyl]-5H-indolo[2,3-a]pyrrolo [3,4-c]carbazole-5,7(6H)-dione (Ia: X₁= X_{1'}=F; X₂=X_{2'}=R₆=H; Q=NH; R₂-R₅=OH).

A solution of 2,10-difluoro-12,13-dihydro-13-[3,4,6-tris-O-(phenyl-methyl)-β-D-glucopyranosyl]-5H-indolo[2,3-a]pyrrolo[3,4-c]carbazole-5,7(6H)-dione (4.36g, 5.49 mmol)) in a mixture of ethanol and ethyl acetate (1:1, 100mL) was subjected to Parr hydrogenation (H₂, 60 psi) for 6h at ambient temperature with 20% Pd(OH)₂ on carbon (4.4g) as catalyst. After suction filtration through Celite, the filtrate was concentrated down to dryness to yield an orange-yellow residue which was purified by column chromatography on silica gel (elution with 50% tetrahydrofuran in hexanes) to furnish the title compound (2.33g, 77%) as a yellow solid, m.p. 255-260°C; ¹H NMR (300 MHz, DMSO-d₆) δ 11.77 (s, 1H), 11.19, (s, 1H), 9.14-9.03 (m, 2H), 7.89 (dd, *J*=11.0, 2.0 Hz, 1H), 7.41 (dd, *J*=9.8, 2.3 Hz, 1H), 7.27-7.19 (m, 2H), 6.26 (d, *J*=8.9 Hz, 1H), 6.14-6.12 (m, 1H), 5.43 (d, *J*=4.0 Hz, 1H), 5.17 (d, *J*=5.5 Hz, 1H), 4.98 (d, *J*=5.5 Hz, 1H), 4.24-3.94 (m, 3H), 3.83-3.81 (m, 1H), 3.59-3.57 (m, 1H), 3.50-3.42 (m, 1H); ¹³C NMR (75 MHz, *DMSO*-d₆) ppm 170.99, 170.91, 163.43, 160.24, 143.12, 141.63, 130.09, 128.74, 126.02, 120.90, 119.35, 118.22, 118.14, 117.74, 116.54, 108.78, 108.57, 98.77, 98.55, 98.20, 84.64, 78.56, 76.44, 73.06, 67.51, 58.29; IR (KBr, cm⁻¹) 3326, 1744, 1700, 1578, 1452, 1328, 1232, 1114, 1074, 828; MS (M⁺) *m*/*z* 523. *Anal.* Calc'd for C₂₆H₁₉F₂N₃O₇•0.25EtOAc•0.40H₂O: C, 58.68; H, 3.98; N, 7.60; H₂O, 1.30. Found: C, 58.55; H, 4.18; N, 7.38; H₂O, 1.20.

### Example 14

### 2,10-Difluoro-12,13-dihydro-13-[2-O-(triethylsilyl)-3,4,6-tris-O-(phenylmethyl)-β-D-glucopyranosyl]-6-triethylsilyl-5H-indolo[2,3-a]pyrrolo[3,4-c]carbazole-5,7-dione (A= VIII: X₁= X_{1'}=F; X₂=X_{2'}=H; R₆=R'''=triethylsilyl; Q=NH; PG=benzyl ) and 2,10-difluoro-12,13-dihydro-13-[2-O-(triethylsilyl)-3,4,6-tris-O-(phenylmethyl)-β-D-glucopyranosyl]-5H-indolo[2,3-a]pyrrolo[3,4-c]carbazole-5,7(6H)-dione (B= VIII: X₁= X_{1'}=F; X₂=X_{2'}=R₆=H; R"'=triethylsilyl; Q=NH; PG=benzyl).

Triethylsilyl triflate (9.0 mL, 40.0 mmol, 20 eq.) was added in one portion to a solution of 2,10-difluoro-12,13-dihydro-13-[3,4,6-tris-O-(phenylmethyl)-β-D-glucopyranosyl]-5H-indolo[2,3-a]pyrrolo[3,4-c]carbazole-5,7(6H)-dione (1.60g, 2.0 mmol) in dry pyridine (100 mL). The mixture was stirred in a sealed flask at room temperature for 48h before absolute ethanol (5 mL) was added. After 10 min., the solvent was removed *in vacuo* at room temperature. The residue was then diluted with ethyl acetate and tetrahydrofuran and acidified with 0.1*N* hydrochloric acid until acidic. Following the separation of the organic phase, the organic phase was washed with brine, dried over anhydrous sodium sulfate, and concentrated. Attempted purification of the residue by flash chromatography (2x) on silica gel (elution with 10% tetrahydrofuran in hexanes for the first time and 40% ether in hexanes the second time) yielded title compound **A** (1.4g) as a slightly impure yellow foam . The impurity proved to be **B**. Complete conversion of **A** to **B** could be accomplished by dissolving **A** (1.4g, 1.37mmol) in absolute ethanol (20mL) and treating the solution with ammonium acetate (0.11g, 1.40 mmol) at ambient temperature for 2h. The mixture was then diluted with ethyl acetate and washed successively with saturated sodium bicarbonate solution and brine prior to drying and concentration to dryness. There was isolated title compound **B** (1.19g, 96%) as a yellow foam.

For **A**: M.p. 77-79°C; ¹H NMR (300 MHz, CDCl₃) δ 10.64 (s, 1H), 9.29 (dd, *J*=8.8, 5.8 Hz, 1H), 9.23-9.20 (m, 1H), 7.33-7.20 (m, 15H), 7.13-7.04 (m, 4H), 5.77 (d, *J*=8.8 Hz, 1H), 5.05 (d, *J*=11.6 Hz, 1H), 4.89-4.79 (m, 2H), 4.67-4.63 (m, 2H), 4.54 (d, *J*=12.4 Hz, 1H), 4.28 (m, 1H), 4.16 (m, 1H), 3.97 (m, 2H), 3.79-3.76 (m, 2H), 1.23-1.16 (m, 6H), 1.11-1.06 (m, 9H), 0.24 (t, *J*=8.0 Hz, 9H), -0.29-(-0.50) (m, 6H); ¹³C NMR (75 MHz, CDCl₃) ppm 175.34, 142.70, 141.88, 138.08, 137.40, 136.28, 128.72, 128.56, 128.27, 128.13, 128.05, 127.52, 127.40, 127.17, 126.73, 121.40, 118.12, 109.84, 109.53, 109.21, 98.26, 97.52, 86.12, 77.71, 77.44, 77.01, 76.72, 76.59, 75.53, 75.25, 74.26, 66.82, 6.91, 6.12, 4.03, 3.95; IR (KBr, cm⁻¹) 3458, 3334, 2954, 2876, 1692, 1454, 1328, 1300, 1116, 1072, 732, 696; MS (M⁺) calc'd for C₅₉H₆₆F₂N₃O₇Si₂ 1022.4407, obsd 1022.4377.

For **B**: M.p. not determined; ¹H NMR (300 MHz, CDCl₃) δ 10.65 (s, 1H), 9.25-9.22 (m, 1H), 9.14 (dd, *J*=8.8, 5.6 Hz, 1H), 7.72 (s, 1H), 7.33-7.21 (m, 14H), 7.16-7.03 (m, 4H), 5.77 (d, *J*=8.8 Hz, 1H), 5.05 (d, *J*=11.5 Hz, 1H), 4.87-4.78 (m, 2H), 4.69-4.63 (m, 2H), 4.59-4.51 (m, 1H), 4.28 (t, *J*=8.8 Hz, 1H), 4.15 (t, *J*=8.8 Hz, 1H), 4.02-3.91 (m, 2H), 3.79-3.73 (m, 2H), 0.23 (t, *J*=8.0 Hz, 9H), -0.28-(-0.52) (m, 6H); ¹³C NMR (75 MHz, CDCl₃) ppm 169.70, 169.66, 164.39, 161.18, 142.80, 142.64, 142.16, 141.99, 138.05, 137.37, 136.26, 130.65, 130.65-126.71 (11 lines, olefinic), 121.11, 119.48, 119.11, 118.93, 118.68, 118.44, 110.04, 109.74, 109.44, 98.39, 98.04, 97.63, 97.26, 86.11, 86.06, 77.76, 77.21, 76.74, 75.52, 75.25, 74.30, 66.86, 6.10, 4.03; IR (KBr, cm⁻¹) 3434, 2876, 1754, 1718, 1624, 1582, 1454, 1326, 1116, 1086, 738; MS (FAB, MH⁺) m/z calc'd for C₅₃H₅₂F₂N₃O₇Si 908.3543, obsd 908.3547.

### Example 15

### 3,9-Difluoro-12,13-dihydro-13-[6-O-(methylsulfonyl)-β-D-glucopyranosyl]-5H-indolo[2,3-a]pyrrolo[3,4-c]carbazole-5,7(6H)-dione (Ib: X₁=X_{1'}=F; X₂=X_{2'}=H=R₆; R₂-R₄=OH; R₅=Mesylate)

To a magnetically stirred solution of 3,9-Difluoro-12,13-dihydro-13-[β-D-glucopyranosyl]-5H-indolo[2,3-a] pyrrolo[3,4-c]carbazole-5,7(6H)-dione (330 mg, 0.630 mmol) in anhydrous pyridine (8 mL) at 0 °C under N₂ was added neat via syringe, methanesulfonyl chloride (80 µL, 1.03 mmol). The reaction mixture was stirred at 0 °C for 3.25 h and monitored by TLC on silica gel (EtOAc elution) to show the new higher Rf product spot in addition to starting material. Additional methanesulfonyl chloride (20 µL, 0.26 mmol) was added and the reaction was stirred at 0 °C for 1.25 h, and then worked up using EtOAc (350 mL) and saturated aqueous copper(II) sulfate (100 mL). The ethyl acetate layer was washed with saturated aqueous copper(II) sulfate (3 x 150 mL), 0.1 N HCl (200 mL), water (200 mL), and brine (200 mL), and dried (Na₂SO₄). Evaporation in vacuo, followed by flash chromatography on silica gel with 4-6% methanol in dichloromethane, gave 146 mg (39%) of the title compound as a yellow solid: 500 MHz COSY ¹H NMR (CD₃COCD₃) δ 10.40 (s, 1H), 10.00 (brs, 1H), 8.97-8.86 (m, 2H), 8.02-7.96 (m,2 H), 7.42-7.33 (m, 2H), 6.43 (d, 1H, J = 9.2 Hz, 1'H), 4.98 (d, 1H, J = 11.0 Hz, 6'H), 4.76 (d, 1H, J = 11.0 Hz, 6"H), 4.48 (d, 1H, J = 10.0 Hz, 5'H), 4.22 (t, 1H, J = 9.7 Hz, 4'H), 4.00 (t, 1H, J = 9.3 Hz, 2'H), 3.89 (t, 1H, J = 9.0 Hz, 3'H), 3.23 (s, 3H); FAB mass spectrum, m/e 601 (M⁺).

### Example 16

### 2,10-Difluoro-12,13-dihydro-13-[6-deoxy-6-(1,3-dihydro-1,3-dioxo-2-isoquino-linyl)-β-D-glucopyranosyl]-5H-indolo[2,3-a]pyrrolo[3,4-c]carbazole-5,7(6H)-dione (A=Ib: X₁=X_{1'}=F; X₂=X_{2'}=R₂=R₆=H; R₂-R₄=OH; R₅=phthalimide).

Potassium phthalimide (0.45g, 2.43 mmol) was added in one portion to a stirred solution of 2,10-difluoro-12,13-dihydro-13-[6-O-(methylsulfonyl)-β-D-glucopyranosyl]-5H-indolo[2,3-a]pyrrolo[3,4-c]carbazole-5,7(6H)-dione (200mg, 0.33 mmol) in anhydrous dimethylformamide (10 mL) before the mixture was heated to 130°C for 3h, cooled to ambient temperature, and concentrated down *in vacuo* overnight. The residue was then taken up in ethyl acetate (some tetrahydrofuran was added) and washed with 0.1*N* hydrochloric acid and brine. Following drying and solvent concentration, the residue was purified by flash chromatography on silica gel (elution with 7% methanol in chloroform) to yield title compound (46 mg, 21%) as a yellow solid.

m.p. >300°C; ¹H NMR (500 MHz, DMSO-d₆) δ 11.21 (m), 10.80 (s), 9.15-9.13 (m), 9.07-9.02 (m), 7.97 (d, *J*=8.8 Hz, 1H), 7.86 (d, *J*=11.1 Hz, 1H), 7.74-7.69 (m), 7.40 (d, *J*=10.3 Hz, 1H), 7.31-7.23 (m), 7.20-7.17 (m), 6.09 (d, *J*=9.1 Hz, 1H), 5.73 (m), 5.64 (m), 5.30 and 5.26 (2m), 4.99 (m), 4.36-4.27 (m), 4.21 (m), 4.14-4.10 (m), 4.01-3.96 (m), 3.83-3.72 (2m), 3.57-3.53 (m); IR (KBr, cm⁻¹) 3426, 1752, 1706, 1624, 1579, 1452, 1397, 1328, 1113, 1035; MS (neg. ESI, M-H⁻) *m/z* 651.

### Example 17

### 13-[6-(Azido-6-deoxy-β-D-glucopyranosyl)-2,10-difluoro-12,13-dihydro-5H-indolo[2,3-a]pyrrolo[3,4-c]carbazole-5,7(6H)-dione (Ib: X₁=X_{1'}=F; X₂=X_{2'}=H=R₆; R₂-R₄=OH; R₅=N₃).

Sodium azide (0.29mg, 0.44 mmol) was added in one portion to a stirred solution of 2,10-difluoro-12,13-dihydro-13-[6-O-(methylsulfonyl)-β-D-glucopyranosyl]-5H-indolo[2,3-a]pyrrolo[3,4-c]carbazole-5,7(6H)-dione (132mg, 0.22 mmol) and powdered 4Å molecular sieves (50 mg) in anhydrous dimethylformamide (3 mL) before the mixture was heated to 60°C for 6h, cooled to ambient temperature, diluted wth ethyl acetate (some tetrahydrofuran was added) and washed with saturated sodium bicarbonate solution and brine. Following drying and solvent concentration, the residue was purified by flash chromatography on silica gel (elution with 10% methanol in chloroform) to yield the title compound (40 mg, 33%) as a yellow solid, decomp. pt. 265°C; ¹H NMR (500 MHz, DMSO-d₆) δ 11.89 (br s, 1H), 11.21 (br s, 1H), 11.14 (br s, 1H), 10.82 (s, 1H), 9.19 (dd, *J*=8.8 Hz, 1H), 9.13-9.06 (m, 3H), 7.90 (dd, *J*=10.8, 2.1 Hz, 1H), 7.71 (dd, *J*=10.5, 2.1 Hz, 1H), 7.49 (dd, *J*=9.6, 2.1 Hz, 1H), 7.35 (dd, *J*=9.4, 2.3 Hz, 1H), 7.30-7.22 (m, 4H), 6.33 (d, *J*=8.7 Hz, 1H), 6.27 (d, *J*=8.6 Hz, 1H), 5.72 (d, *J*=5.2 Hz, 1H), 5.61 (d, *J*=4.9 Hz, 1H), 5.33 (br s, 1H), 5.23-5.21 (m, 2H), 5.11 (d, *J*=5.0 Hz, 1H), 4.20-4.10 (m, 4H), 4.02-3.94 (m, 3H), 3.85-3.83 (m, 1H), 3.79-3.69 (m, 3H), 3.63-3.58 (m, 3H); IR (KBr, cm⁻¹) 3422, 2110, 1740, 1700, 1622, 1580, 1450, 1381, 1329, 1231, 1170, 1115, 1074, 829, 763; HRMS (neg. ESI, M-H-) calc'd for C₂₆H₁₇F₂N₆O₆ 547.1256, obsd 547.1199.

### Example 18

### 6-Amino-13-(6-amino-6-deoxy-β-D-glucopyranosyl)-2,10-difluoro-12,13-dihydro-5H-indolo[2,3-a]pyrrolo[3,4-c]carbazole-5,7(6H)-dione (Ic: X₁=X_{1'}=F; X₂=X_{2'}=H; R₂-R₄=OH; R₅=NH₂=R₆).

Hydrazine hydrate (2 mL) was added to 2,10-difluoro-12,13-dihydro-13-[6-deoxy-6-(1,3-dihydro-1,3-dioxo-2-isoquinolinyl)-β-D-glucopyranosyl]-5H-indolo[2,3-a] pyrrolo[3,4-c]carbazole-5,7(6H)-dione (19mg, 0.029 mmol) and the mixture was stirred at room temperature for 1h and at 50°C for 1h before it was concentrated down *in vacuo* for 24h. Purification of the residue by HPLC on a C₁₈ YMC Pack ODS column (30% isocratic flow using methanol/water/ 0.1% trifluoroacetic acid) furnished the title compound (10 mg, 27%) as an orange-yellow solid, m.p. >300°C; ¹H NMR (500 MHz, DMSO-d₆/D₂O) δ 8.94-8.89 (2m), 8.81 (m), 8.68 (m), 7.93 (m), 7.82 (d, *J*=10.0 Hz, 1H), 7.73 (d, *J*=10.2 Hz, 1H), 7.39 (d, *J*=9.5 Hz, 1H), 7.12-7.11 (m), 6.93 (m), 6.20 (d, *J*=8.9 Hz, 1H), 6.07 (d, *J*=7.7 Hz, 1H), 4.22 (m), 4.16-4.12 (m), 4.14 (t, *J*=8.9 Hz, 1H), 3.79 (t, *J*=8.9 Hz, 1H), 3.75-3.73 (m), 3.61-3.52 (m), 3.40-3.34 (m), 3.31-3.27 (m); IR (KBr, cm⁻¹) 3415, 1757, 1706, 1676, 1623, 1581, 1451, 1404, 1330, 1203, 1112, 839; HRMS (neg. ESI, M-H-) calc'd for C₂₆H₁₉F₂N₅O₆ 536.1460, obsd 536.1349.

### Example 19

### 13-[6-(Amino-6-deoxy-β-D-glucopyranosyl)-3,9-difluoro-12,13-dihydro-5H-indolo[2,3-a]pyrrolo[3,4-c]carbazole-5,7(6H)-dione (Ib: X₁=X₁'=F; X₂=X₂'=H=R₆; R₂-R₄=OH; R₅=NH₂).

To a solution of the compound, 13-[6-(Azido-6-deoxy-β-D-glucopyranosyl)-3,9-difluoro-12,13-dihydro-5H-indolo[2,3-a]pyrrolo[3,4-c]carbazole-5,7(6H)-dione (98.9 mg, 0.180 mmol) in absolute methanol (125 mL) under N₂ in a Parr was added palladium(II) chloride (90 mg, 0.51 mmol). The mixture was sonnicated for 10 min and then placed on a Parr shaker at 70 psi hydrogen pressure for 45 h. The mixture was filtered through a small pad of Celite, washed with methanol, and concentrated in vacuo. Purification on a Sephadex LH 20 column with methanol elution (0.4-0.5 mL/min flow rate) , gave 69.6 mg (70%) of the title compound as a yellow orange solid: 500 MHz COSY ¹H NMR (d6-DMSO) δ 11.20 (brs, 1H), 8.92 (dd, 1H, J = 9.7, 2.6 Hz), 8.81 (dd, 1H, J = 9.7, 2.6 Hz), 8.05 (dd, 1H, J = 9.0, 4.6 Hz), 7.95 (brs, 3H), 7.88 (dd, 1H, J = 8.9, 4.6 Hz), 7.48 (ddd, 1H, J = 2.5, 8.9, 9.1 Hz), 7.41 (ddd, 1H, J = 2.5, 8.9, 9.1 Hz), 6.41 (d, 1H, J = 8.8 Hz, 1'H), 5.70 (brs, 1H), 5.36 (brd, 2H, 2', 3' OH), 4.23-4.19 (m, 1H, 5'H), 4.06-4.02 (m, 1H, 2'H), 3.89-3.84 (m, 1H, 3'H), 3.56 (t, 1H, J = 8.9 Hz, 4'H), 3.32-3.25 (m, 1H, 6'H), 3.13-3.08 (m, 1H, 6"H); FAB mass spectrum, m/e 522 (M⁺).

### Example 20

### 4-[2-(Benzo[b]thien-2-yl)-5-fluoro-1H-indol-3-yl]-3-chloro-1-methyl-1H-pyrrole-2,5-dione (V: X₁=5F; X₂=H=R':R₆=Me; R"=benzo[b]thienyl; y=Cl).

To a solution of 2-(2-benzo[b]thienyl)-5-fluoroindole (10.0g, 0.37 mol) in anhydrous tetrahydrofuran (250 mL) under nitrogen was added via syringe ethylmagnesium bromide (3M, 13.1 mL, 0.39 mol) at a rate which kept the internal temperature between 45-50°C. The mixture was then heated to 50-55°C for 0.5h and cooled back to room temperature before a solution of 3,4-dichloro-1-methylmaleimide (7.41g, 0.41 mol) in anhydrous tetrahydrofuran was canulated into the reaction mixture. The mixture was refluxed for 2h, cooled, diluted with ethyl acetate, washed with saturated ammonium chloride solution and brine prior to drying and solvent concentration to 1/4 volume. Suction filtration of the suspension furnished the title compound (12.65g, 82%) as a red-violet solid, m.p. 250-251°C; ¹H NMR (300 MHz, DMSO-d₆) δ 12.50 (s, 1H), 8.03-7.99 (m, 1H), 7.93-7.89 (m, 1H), 7.83 (s, 1H), 7.52 (dd, *J*=8.9, 4.4 Hz, 1H), 7.46-7.36 (m, 2H), 7.29 (dd, *J*=9.9, 2.5 Hz, 1H), 7.16-7.09 (m, 1H), 3.37 (s, 3H); ¹³C NMR (75 MHz, DMSO-d₆) ppm 167.71, 165.34, 159.10, 156.00, 139.48, 134.18, 133.76, 133.19, 133.06, 132.66, 127.41, 127.27, 125.20, 125.02, 124.22, 123.64, 122.55, 113.23, 113.10, 111.89, 111.54, 105.40, 105.07, 100.56, 24.61; IR (KBr, cm⁻¹) 3336, 1778, 1706, 1636, 1490, 1441, 1383, 1297, 1170, 1010, 982, 881, 822, 800, 750, 736; MS (neg. ESI, M-H⁻) *m/z* 409. *Anal.* Calc'd for C₂₁H₁₂FN₂O₂S: C, 61.39; H, 2.94; N, 6.82. Found: C, 61.08; H, 2.88; N, 6.62.

### Example 21

### 3-Fluoro-6-methyl-5H,13H-benzo[b]thienyl[2,3-a]pyrrolo[3,4-c]carbazole-5,7-dione (VI: X₁=F; X₂=X_{1'}=X_{2'}=R'=H; Q=S; R₆=Me).

A suspension containing 4-[2-(benzo[b]thien-2-yl)-5-fluoro-1H-indol-3-yl]-3-chloro-1-methyl-1H-pyrrole-2,5-dione (12.6g, 30.7 mmol) in dry absolute ethanol/dioxane (60%, 1L) was placed in a 2L pyrex flask equipped with a reflux condenser . The mixture was stirred and irradiated (Hanovia medium-pressure Hg lamp, 450-W) for 14h before it was cooled to ambient temperature and refrigerated for an additional 24h at -5°C. Suction filtration of the suspension afforded the title compound (12.7g, 95%) as a fluorescent yellowish-green fiberglass like solid, m.p. >305°C ; ¹H NMR (500 MHz, DMSO-d₆) δ 9.50 (d, *J*=7.8 Hz, 1H), 8.39 (d, *J*=9.7 *Hz, 1H),* 8.05 (d, *J*=7.8 Hz, 1H), 7.55-7.47 (m, 3H), 7.33-7.29 (m, 1H), 3.55 (s, 0.3 dioxane, 3H), 2.96 (s, 3H); ¹³C NMR (125 MHz, DMSO-d₆ at 40°C) ppm 168.99, 168.73, 158.05, 138.98, 138.93, 137.97, 134.13, 129.91, 127.73, 127.07, 126.59, 125.40, 125.22, 122.99, 121.18, 118.50, 115.75, 115.55, 114.81, 112.85, 112.78, 109.87, 109.67, 66.45 (dioxane), 23.66; IR (KBr, cm⁻¹) 3263, 1756, 1702, 1694, 1629, 1494, 1455, 1374, 1291, 1256, 1222, 1168, 1156, 1110, 982, 869, 806, 755, 745, 704, 613; MS (neg. ESI, M-H-) *m/z* 373. *Anal.* Calc'd for C₂₁H₁₁FN₂O₂S •0.3Dioxane•1.5H₂O: C, 65.93; H, 3.44; N, 6.93; H₂O, 0.89. Found: C, 65.86; H, 3.43; N, 6.68; H₂O, 0.56.

### Example 22

### 3-Fluoro-6-methyl-13-[2,3,4,6-tetra-O-(phenylmethyl)-β-D-glucopyranosyl-5H,13H-benzo[b]thienyl[2,3-a]pyrrolo[3,4-c]carbazole-5,7(6H)-dione (VIII: X₁=F; X₂=X_{1'}=X_{2'}=H; Q=S; R₆=Me; PG=R"'=benzyl).

Diisopropyl azodicarboxylate (4.6 mL, 24.0 mmol, 4.5 eq.) was added dropwise to a cold (0°C) slight suspension of 3-fluoro-6-methyl-5H,13H-benzo[b]thienyl[2,3-a]pyrrolo[3,4-c]carbazole-5,7-dione (2.0g, 5.34 mmol), 2,3,4,6-tetra-O-benzyl-D-glucopyranose (8.67g, 16.03 mmol, 3.0 eq.) and triphenylphosphine (6.31g, 24.0 mmol, 4.5 eq.) in anhydrous tetrahydrofuran (200 mL). The mixture was stirred at 0°C for 1h before it was diluted with ethyl acetate and washed with 0.1N hydrochloric acid and brine. Following drying and solvent evaporation, the residue was purified by flash chromatography on silica gel (elution with 10% ethyl acetate in hexanes followed by 15% ethyl acetate in hexanes) to yield the title compound (4.60g, 96%) as a yellow foam, m.p. 64-70°C ; ¹H NMR (500 MHz, DMSO-d₆, 1:1 mixture of rotational isomers) δ 9.87 and 9.78 (2m, 1H), 9.00-8.99 and 8.85-8.83 (2m, 1H), 8.11-8.02 (2m, 1.5H), 7.73 (d, *J*=7.2 Hz, 0.5H), 7.58-7.54 (m, 2.5H), 7.38-7.23 (m, 15H), 7.18 (d, *J*=7.9 Hz, 0.5H), 6.84 (t, *J*=7.4 Hz, 0.5H), 6.78-6.75 (m, 0.5H), 6.67 (t, *J*=7.6 Hz, 1H), 6.55 (t, *J*=7.5 Hz, 1H), 6.51 (d, *J*=8.8 Hz, 0.5H), 6.43 (d, *J*=9.2 Hz, 0.5H), 6.12-6.10 (m, 2H), 4.95 (d, *J*=10.9 Hz, 0.5H), 4.88-4.79 (m, 3H), 4.74-4.65 (m, 1H), 4.58-4.48 (m, 2.5H), 4.36-4.29 (m, 1.5H), 4.24-4.20 (m, 0.5H), 4.14 (br s, 0.5H), 4.11-4.00 (m, 3H), 3.93 (d, J=10.6 Hz, 0.5H), 3.87 (br s, 1H), 3.18 (s, 3H); IR (KBr, cm⁻¹) 3423, 2920, 2866, 1759, 1699, 1622, 1481, 1453, 1380, 1283, 1255, 1213, 1180, 1160, 1089, 744, 697; MS (FAB, M⁺) *m/z* 896.

### Example 23

### 3-Fluoro-6-methyl-13-[β-D-glucopyranosyl]-5H,13H-benzo[b]thienyl[2,3-a] pyrrolo[3,4-c]carbazole-5,7(6H)-dione (Ia: X₁=F; X₂=X_{2'}=X_{1'}=H; Q=S; R₆=Me; R₂-R₅=OH).

To a cold (0°C), stirred solution of 3-fluoro-6-methyl-13-[2,3,4,6-tetra-O-(phenylmethyl)-β-D-glucopyranosyl]-5H,13H-benzo[b]thienyl[2,3-a]pyrrolo [3,4-c]carbazole-5,7(6H)-dione (4.5g, 5.02 mmol) in ethanethiol (30mL) was added cold (0°C) boron trifluoride etherate (6.36 mL, 50.2 mmol). The mixture was stirred at 0°C for 1h before it was allowed to stir at ambient temperature for a total of 48h. Additional boron trifluoride etherate (3.2 mL) was added after 12h and 24h. The solvent was removed *in vacuo* and the remaining residue was taken up in ethyl acetate/tetrahydrofuran and washed with IN hydrochloric acid and brine prior to drying and solvent concentration. Purification of the residue by flash chromatography on silica gel (gradient elution with 10% methanol in chloroform followed by 15% methanol in chloroform) yielded the title compound (2.53g, 94%) as a yellow solid, m.p. >305°C; ¹H NMR (500 MHz, DMSO-d₆) δ 9.85 and 9.71 (2d, *J*=7.4 and 7.5 Hz, 1H), 8.96 and 8.89 (2d, *J*=9.6 and 9.6 Hz, 1H), 8.13-8.05 (m, 1.25H), 7.99 (dd, *J*=9.0, 4.5 Hz, 0.75H), 7.61-7.41 (3m, 3H), 6.29 and 6.09 (2d, 8.8 and 9.3 Hz, 1H), 5.36-5.33 (3H), 4.84-4.72 (m, 1.5H), 4.04-3.98 (m, 1H), 3.92-3.86 (m, 1.75H), 3.76-3.68 (m, 2H), 3.63-3.54 (m, 1.75H), 3.16 (s, 3H); ¹³C NMR (125 MHz, DMSO-d₆) ppm 169.34, 169.19, 169.04, 158.81, 156.95, 140.06, 138.48, 137.05, 133.12, 131.70, 131.49, 130.85, 129.75, 128.36, 127.40, 127.20, 126.52, 125.78, 125.71, 125.39, 122.80, 122.66, 122.35, 119.81, 116.33, 116.08, 115.94, 115.74, 110.70, 110.50, 105.28, 87.77, 81.36, 77.72, 70.89, 69.86, 61.28, 24.33; IR (KBr, cm⁻¹) 3428, 1756, 1694, 1622, 1481, 1460, 1448, 1384, 1084, 745; MS (neg. ESI, M-H-) *m/z* 535.

### Example 24

### 3-Fluoro-13-(β-D-glucopyranosyl)-5H,13H-benzo[b]thienyl[2,3-a]pyrrolo[3,4-c] carbazole-5,7(6H)-dione (Ia: R₂-R₅=OH; R₆=X₂=X_{1'}=X_{2'}=H; Q=S).

3-Fluoro-6-methyl-13-(β-D-glucopyranosyl)-5H,13H-benzo[b]thienyl[2,3-a]pyrrolo[3,4-c]carbazole-5,7(6H)-dione (0.20g, 0.37 mmol) was suspended in a solution of 10% aqueous potassium hydroxide (30 mL) under nitrogen. The mixture was gently refluxed for 3h before it was titrated with concentrated HCl until a dense precipitate formed. The precipitate was taken up in ethyl acetate/tetrahydrofuran and washed with 1N hydrochloric acid and brine prior to drying and solvent concentration. The residue was dissolved in absolute ethanol (1 mL) and solid ammonium acetate (3.0g) was added. The mixture was fused at 150°C for 3h before it was cooled, diluted with ethyl acetate/tetrahydrofuran, washed with 1N sodium hydroxide, brine, dried and concentrated. Purification of this residue by flash chromatography on silica gel (gradient elution with 30% tetrahydrofuran in chloroform followed by 50% tetrahydrofuran in chloroform) furnished the title compound (69.5mg, 36%) as a yellow solid, m.p. >305°C; ¹H NMR (500 MHz, DMSO-d₆) δ 11.67 (s, 0.25H), 11.53 and 11.51 (2s, 1H), 9.95-9.93 and 9.91-9.89 (2m, 1H), 9.02 and 8.97 (2dd, *J*=9.8, 2.7 Hz and 9.7, 2.8 Hz, 1H), 8.24-8.23 and 8.18-8.16 (2m, 1H), 8.08 and 8.01 (2dd, *J*=9.4, 4.4 Hz and 9.1, 4.6 Hz, 1H), 7.69-7.63 (m, 2H), 7.54-7.48 (m, 1H), 6.34 and 6.09 (2d, *J*=8.9, 9.4 Hz, 1H), 5.37-5.35 (m, 1H), 5.28 and 5.22 (2br m, 2H), 4.75 (br m, 0.75H), 4.04 and 3.87-3.75 (2m, 3H), 3.68-3.58 (m, 2H), 3.55-3.52 (m, 2H); ¹³C NMR (125 MHz, DMSO-d₆) ppm 170.39, 170.16, 158.76, 156.89, 140.05, 138.34, 136.87, 133.09, 131.76, 128.32, 127.28, 126.62, 125.64, 122.64, 120.60, 116.16, 115.95, 115.84, 110.76, 110.56, 107.35, 87.71, 81.13, 77.45, 70.74, 69.79, 67.01, 61.23; IR (KBr, cm⁻¹) 3392, 1747, 1705, 1625, 1481, 1462, 1430, 1326, 1284, 1262, 1180, 1087, 804, 758, 747; HRMS (neg. ESI, M-H-) calc'd for C₂₆H₁₈FN₂O₇S 521.0897, obsd 521.0802.

### Example 25

### 3,9-Difluoro-12,13-dihydro-13-[3,6-O-(t-butyldimethylsilyl)-β-D-glucopyranosyl]-5H-indolo[2,3-a]pyrrolo [3,4-c] carbazole-5,7(6N-[4-(t-butyl)benzyl]-)-dione (Ib: X₁=X_{1'}= F; X₂=X_{2'}= H; R₆=4-(t-butyl)benzyl; Q=NH; R2 =R₄=OH; R₃=R₅=t-butyldimethylsilyloxy).

To a magnetically stirred solution of product from example 12 (750 mg, 1.12 mmol) and imidazole (1.9 g, 28 mmol) in anhydrous DMF (14 mL) under N₂ with stirring was added, neat via syringe, t-butyldimethylsilyl trifluoromethanesulfonate (2.96 g, 2.57 mL, 11.2 mmol). The reaction was stirred at room temperature for 1 h and quenched with absolute ethanol (5 mL). The mixture was diluted with ethyl acetate (150 mL) and washed with water (6 x 100 mL), and brine (100 mL), and dried (Na₂SO₄). This material was used directly in the next step without further purification. The analytical sample was purified by flash chromatography on silica gel with hexane/methylene chloride: 300 MHz ¹H NMR (CDCl₃) δ 10.06 (brs, 1H), 9.12 (dd, 1H, J = 9.2, 2.7 Hz), 7.88-7.81 (m, 1H), 7.72 (dd, 1H, J = 9.2, 3.9 Hz), 7.41 (ddd, 1H, J = 8.9, 8.9, 2.6 Hz), 7.25 (dd, 1H, J = 9.0, 4.1 Hz), 7.20-6.99 (m, 4H), 6.92 (ddd, 1H, J = 8.7, 8.7, 2.4 Hz), 5.97 (d, 1H, J = 8.9 Hz), 4.58-3.90 (m, 8H), 1.18 (s, 9H), 0.91 (s, 9H), 0.83 (s, 9H), 0.27 (s, 3H), 0.10 (s, 3H), 0.05 (s, 3H), -0.02 (s, 3H); FAB mass spectrum, m/e 897 (M⁺).

### Example 26

### 3,9-Difluoro-12,13-dihydro-13-[3 -O-(t-butyldimethylsilyl)-β-D-glucopyranosyl]-5H-indolo[2,3-a]pyrrolo [3,4-c] carbazole-5,7(6N-[4-(t-butyl)benzyl]-)-dione (Ib: X₁=X_{1'}= F; X₂=X_{2'} = H; R₆ =4-(t-butyl)benzyl; Q=NH; R₂ =R₄=OH; R₃ = t-butyldimethylsilyloxy)

To a magnetically stirred solution of the crude product from example 25 at -5° C was added a mixture of 9 :1 trifluoroacetic acid : water precooled to -5 °C. The reaction was stirred at -5° C for 45 min, diluted with ethyl acetate (200 mL), and washed with saturated aqueous sodium bicarbonate (6 x 150 mL), water (1 x 150 mL), and brine (1 x 150 mL), and dried (Na2SO4). Evaporation in vacuo, followed by flash chromatography on silica gel with 1-10% methanol in methylene chloride gave 59 mg (6%) of the starting material (i.e. compound of example 25), 78 mg (10%) of fully desilylated product (i.e. compound of example 12), and 585 mg (74%) of the pure title compound as a yellow solid: 500 MHz COSY ¹H NMR (d6-DMSO) δ 11.80 (brs, 1H), 8.82 (dd, 1H, J = 9.6, 2.7 Hz), 8.76 (dd, 1H, J = 9.7, 2.7 Hz), 8.03 (dd, 1H, J = 9.2, 4.4 Hz), 7.68 (dd, 1H, J = 8.9, 4.5 Hz), 7.51-7.45 (m, 2H), 7.38-7.31 (m, 4H), 6.28 (d, 1H, J = 9.1 Hz, 1'H), 6.08 (t, 1H, J = 3.9 Hz, 6'OH), 5.35 (d, 1H, J = 6.8 Hz, 4'OH), 4.88 (s, 2H), 4.81 (d, 1H, J = 6.8 Hz, 2'OH), 4.12-4.08 (m, 1H, 6'H), 3.98-3.93 (m, 1H, 5'H), 3.93-3.90 (m, 1H, 4'H), 3.86-3.82 (m, 1H, 6'H), 3.73 (t, 1H, J = 8.8 Hz, 3'H), 3.39 (ddd, 1H, J = 9.1, 8.8, 6.8 Hz, 2'H), 1.23 (s, 9H), 0.76 (s, 9H), 0.07 (s, 3H), -0.03 (s, 3H); FAB mass spectrum, m/e 783 (M⁺).

### Example 27

### 3,9-Difluoro-12,13-dihydro-13-[3-O-(t-butyldimethylsilyl)-6-O-(methanesulfonyl)-β-D-glucopyranosyl]-5H-indolo[2,3-a]pyrrolo [3,4-c] carbazole-5,7(6N-[4-(t-butyl)benzyl]-)-dione (Ib: X₁=X₁,= F; X₂=X_{2'} = H; R₆ =4-(t-butyl)benzyl; Q=NH; R₂ =R₄=OH; R₃=t-butyldimethylsilyloxy; R₅=methane sulfonyloxy)

To a magnetically stirred solution of the pure product from example 26 (403 mg, 0.514 mmol) in anhydrous pyridine (7 mL) at -10 °C under argon was added neat via syringe over 1 min, methanesulfonyl chloride (46 µL, 0.594 mmol). After 70 min at -10 °C, an aliquot was removed, added to a mixture of ethyl acetate and saturated aqueous copper(II) sulfate, and analyzed by TLC on silica gel using 1-2% methanol in methylene chloride. There was indicated mostly starting material with a small amount of the higher Rf product spot. Additional methanesulfonyl chloride (32 µL) was added, and the reaction maintained at -10 °C for 1 h. Over 30 min intervals, the reaction was treated successively with additional methanesulfonyl chloride (33 µL, 31 µL, 31 µL, and 24 µL). After a total of about 5 h at -10 °C, TLC showed the major product spot and a little of the lower Rf starting material. Over the last 1.5 h, the temperature of the reaction was allowed to rise to -1°C from -10°C. The reaction mixture was quenched by the addition of 200 µL of absolute ethanol, stirred for 5 min, and partitioned with ethyl acetate (600 mL) and saturated aqueous copper(II) sulfate (250 mL). The organic layer was washed with saturated aqueous copper(II) sulfate (200 mL), water (200 mL), and brine (200 mL), and dried (Na₂SO₄). Evaporation in vacuo, followed by flash chromatography on silica gel with 1% methanol in methylene chloride gave 404 mg (91%) of the pure title compound and 40 mg of recovered starting material: 300 MHz ¹H NMR (CDCl₃) δ 9.83 (brs, 1H), 9.08 (dd, 1H), 7.80-7.71 (m, 1H), 7.68 (dd, 1H), 7.52 (dd, 1H), 7.40 (ddd, 1H), 7.21-7.13 (m, 2H), 7.03-6.86 (m, 3H), 5.94 (d, 1H), 5.15-3.92 (m, 10H), 3.02 (s, 3H), 1.19 (s, 9H), 0.84 (s, 9H), 0.23 (s, 3H), -0.01 (s, 3H); FAB mass spectrum, m/e 861 (M⁺).

### Example 28

### 3,9-Difluoro-12,13-dihydro-13-[3-O-(t-butyldimethylsilyl)-6-(4N-morpholino)-β-D-glucopyranosyl]-5H-indolo[2,3-a]pyrrolo [3,4-c] carbazole-5,7(6N-[4-(t-butyl)benzyl]-)-dione (Ib: X₁=X_{1'}= F; X₂=X_{2'} = H; R₆ =4-(t-butyl)benzyl; Q=NH; R₂ =R₄=OH; R₃=t-butyldimethylsilyloxy; R₅=morpholino)

To a magnetically stirred solution of the pure product from example 27 (193 mg, 0.224 mmol) in anhydrous DMSO (5mL) under N₂ was added morpholine (210 µL, 2.41 mmol). The resulting red reaction mixture was stirred for 69 h at 47 °C and partitioned with ethyl acetate (350 mL) and saturated aqueous sodium bicarbonate (75 mL). The organic layer was washed with water (4 x 70 mL) and brine (75 mL), and dried (Na₂SO₄). Evaporation in vacuo, followed by flash chromatography on silica gel with 20-25% ethyl acetate in methylene chloride gave 9 mg (5%) of recovered starting material, and then102 mg (53%; 56% based on recovered starting material) of the pure title compound as a yellow solid: 300 MHz ¹H NMR (CDCl₃) δ 10.03 (brs, 1H), 9.09 (dd, 1H), 8.02-7.96 (m, 1H), 7.69 (dd, 1H), 7.39 (ddd, 1H), 7.30-7.23 (m, 1H), 7.21-7.17 (m, 2H), 7.07-6.98 (m, 3H), 5.96 (d, 1H), 4.53-3.93 (m, 6H), 3.72-3.57 (m, 4H), 3.19-2.98 (m, 2H), 2.82-2.71 (m, 2H), 2.68-2.58 (m, 2H), 1.19 (s, 9H), 0.88 (s, 9H), 0.27 (s, 3H), 0.08 (s, 3H); ESI(NEG) mass spectrum, m/e 851 (M-H)⁻.

### Example 29

### 3,9-Difluoro-12,13-dihydro-13-[6-(4N-morpholino)-β-D-glucopyranosyl]-5H-indolo[2,3-a]pyrrolo [3,4-c] carbazole-5,7(6H)-dione (Ia: X₁=X_{1'}= F; X₂=X_{2'}= R₆ = H; R₅= 4N-morpholino; Q=NH; R₂-R₄=OH).

To a magnetically stirred solution of the pure product from example 28 (205 mg, 0..240 mmol) in absolute ethanol (160 mL) was added aqueous potassium hydroxide (4.45 M, 42 ml, 187 mmol). The resulting deep red solution was heated in an open flask until all the ethanol was evaporated and a solid gums out (about 1.5 h). The mixture was cooled under a stream of nitrogen and concentrated HCl (12 N; 17 mL, 204 mmol) was added. The mixture was stirred 5 min and absolute ethanol (90 mL) was added, followed by solid ammonium acetate (85 g). The mixture was heated with stirring in an open flask just below boiling for 2.5-3 h, then to 180 °C (internal temperature of the melt) over about 2 h. The mixture was further heated to 200 °C over 10-15 min, rapidly cooled under a stream of nitrogen to about 50-60 °C, and digested with water (200 mL), saturated aqueous sodium bicarbonate (200 mL), and ethyl acetate (800 ml). The organic layer was washed with brine (200 mL), the combined aqueous layers extracted with fresh ethyl acetate (300 mL), and the latter extract was washed with brine (100 mL). The combined organic extracts were dried (Na₂SO₄) and evaporated in vacuo. The crude material was dissolved in methanol (400 mL) and treated with anhydrous potassium carbonate (2.0 g) and potassium fluoride dihydrate (1.03 g), and stirred at room temperature for 15 h. The solvent was evaporated in vacuo, the residue redissolved in methanol (250 mL), treated with 1 N HCl (15 mL), and evaporated in vacuo. The residue was dissolved in absolute ethanol (500 mL), evaporated in vacuo, and this process was again repeated. Recrystallization from 95% ethanol gave 79.6 mg (53%, 2 crops) of the pure title compound as its HCl salt: 500 MHz COSY ¹H NMR (d6-DMSO) δ 12.67 (brs, 1H), 11.19 (s,1H), 8.92 (dd, 1H, J = 9.7, 2.6 Hz), 8.79 (dd, 1H, J = 9.8, 2.3 Hz), 8.05 (dd, 1H, J = 9.0, 4.5 Hz), 7.94 (dd, 1H, J = 8.7,4.5 Hz), 7.46 (ddd, 1H, J = 2.5, 8.9, 9.0 Hz), 7.41 (ddd, 1H, J = 2.6, 9.0, 9.1 Hz), 6.61 (d, 1H, J = 8.6 Hz, 1'H), 3.97 (t, 1H, J = 8.8 Hz, 2'H), 3.91-3.16 (m, 13 H).

### Example 30

### 3,9-Difluoro-12,13-dihydro-13-[6-azido-β-D-glucopyranosyl]-5H-indolo[2,3-a]pyrrolo [3,4-c] carbazole-5,7(6-hydroxy)dione (Ic)

To a magnetically stirred solution of the pure 3,9-Difluoro-12,13-dihydro-13-[6-azido-β-D-glucopyranosyl]-5H-indolo[2,3-a]pyrrolo [3,4-c] carbazole-5,7[6(4-t-butylbenzyl)) -dione (20 mg, 0.029 mmol) in absolute ethanol (5 mL) was added aqueous potassium hydroxide (4.45 M, 1.1 ml, 4.9 mmol). The resulting deep red solution was heated in an open flask until all the ethanol was evaporated and a solid gums out (about 1.5 h). The mixture was cooled under a stream of nitrogen and absolute ethanol (5 mL) was added, followed by solid hydroxylamine hydrochloride(685 mg). The mixture was refluxed with stirring for 4 h, then heated to 110 °C (internal temperature of the melt after solvent was boiled off) for about 3 h. The mixture was cooled under a stream of nitrogen to room temperature, and digested with 1 N HCl (80 mL) and ethyl acetate (400 ml). The organic layer was washed with 1 N HCl (3 x 50 mL), water (2 x 50 mL), and brine (100 mL), and dried (Na₂SO₄). Evaporation in vacuo, followed by purification on a Sephadex LH 20 column with methanol elution (0.4-0.5 mL/min flow rate) gave 4.3 mg (27%) of the title compound as a yellow red solid: IR (KBr) 2114 cm⁻¹; 500 MHz COSY ¹H NMR (CD₃OD) δ 8.53 (dd, 1H, J = 9.6, 2.1 Hz), 8.36 (dd, 1H, J = 9.5, 1.9 Hz), 7.68 (dd, 1H, J = 8.8, 3.7 Hz), 7.36 (dd, 1H, J = 8.8, 4.2 Hz), 7.24 (ddd,, 1H, J = 8.7, 8.7, 2.0 Hz), 7.14 (ddd,, 1H, J = 8.9, 8.8, 2.3 Hz), 5.98 (d, 1H, J = 9.0 Hz, 1'H), 4.47 (d, 1H, J = 12.1 Hz, 6'H), 4.28 (d, 1H, J = 12.1 Hz, 6"H), 4.11 (d, 1H, J = 9.4 Hz, 5'H), 3.91 (t, 1H, J = 9.4 Hz, 4'H), 3.61 (t, 1H, J = 9.0 Hz, 2'H), 3.46 (dd, 1H, J = 9.4, 9.0 Hz, 3'H); FAB mass spectrum, m/e 564 (M⁺).

### Example 31

### 3,9-Difluoro-12,13-dihydro-13-[6-amino-β-D-glucopyranosyl]-5H-indolo[2,3-a]pyrrolo [3,4-c] carbazole-5,7(6-hydroxy)dione (Ic)

To a solution of product from example 30 (3.0 mg, 0.0053 mmol) in absolute methanol (2 mL) under N₂ in a Parr was added palladium(II) chloride (15 mg, 0.09 mmol). The mixture was sonnicated for 10 min and then placed on a Parr shaker at 65 psi hydrogen pressure for 24 h. The mixture was filtered through a small pad of Celite, washed with methanol, and concentrated in vacuo to give 2.0 mg (66%) of the title compound as a yellow orange solid: FAB mass spectrum, m/e 538 (M⁺).

Additional Examples of formula I compounds, which may be synthesized by modifications of the foregoing synthetic procedures, are set forth in Table IV wherin the substituents are the same as in Example 29 unless otherwise mentioned.

**Table IV**

| Example | R₂ | R₅ | R₆ | Q |
|---|---|---|---|---|
| 32 | OH | N3 | 4-t-butyl-benzyl | NH |
| 33 | OH | N3 | H | NH |
| 34 | OH | imidazole | H | NH |
| 35 | OH | 4-Mepiperazine | H | NH |
| 36 | OH | 4N-morpholino | H | S |
| 37 | OH | NH2 | H | S |
| 38 | OH | 4N-morpholino | H | O |
| 39 | OH | NH2 | H | O |
| 40 | OH | N3 | H | O |
| 41 | OH | N(CH2CH2OH)2 | H | S |
| 42 | OH | CN | H | S |
| 43 | OH | COOH | H | NH |
| 44 | OH | Nω Ornithine | H | NH |
| 45 | O | OH | H | NH |
| 46 | N-OH | OH | H | NH |
| 47 | OH | F | H | NH |
| 48 | OH | F | OH | NH |

The analytical data for some of the Examples shown in Table IV is given below:

### Example 32:

IR (KBr) 2110 cm⁻¹; 500 MHz COSY ¹H NMR (CD₃OD) δ 8.81-8.8.79 (m, 2H), 7.68 (dd, 1H, J = 9.1, 4.0 Hz), 7.56 (dd, 1H, J = 8.8, 4.2 Hz), 7.38-7.25 (m, 6H), 6.05 (d, 1H, J = 8.5 Hz, 1'H), 4.75 (s, 2H), 4.29 (d, 1H, J = 12.8 Hz, 6'H), 4.17-4.06 (m, 2H, 5',6"H), 4.00 (t, 1H, J = 8.9 Hz, 4'H), 3.70-3.65 (m, 2H, 2',3'H), 1.27 (s, 9H); FAB mass spectrum, m/e 694 (M⁺).

### Example 33:

Yellow solid: IR (KBr) 2112, 1750, 1700 cm⁻¹; 500 MHz COSY ¹H NMR (CD₃OD) δ 8.78-8.72 (m, 2H), 7.77 (dd, 1H, J = 9.1, 4.1 Hz), 7.55 (dd, 1H, J = 8.8, 4.2 Hz), 7.33-7.26 (m, 2H), 6.12 (d, 1H, J = 8.5 Hz, 1'H), 4.27 (d, 1H, J = 11.5 Hz, 6'H), 4.15-4.07 (m, 2H, 5', 6"H), 4.01 (t, 1H, J = 8.7 Hz, 4'H), 3.74-3.67 (m, 2H, 2', 3'H); FAB mass spectrum, m/e 548 (M⁺).

### Example 34:

Yellow-orange solid: 500 MHz ¹H NMR (CD₃OD) δ 8.81-8.61 (m, 2H), 7.88-7.04 (m, 7H), 6.32 (d, 1H, J = 8.9 Hz, 1'H), 4.20-3.30 (m, 6H); FAB mass spectrum, m/e 574 (MH⁺).

### Example 35:

Yellow solid: FAB mass spectrum, m/e 605 (M⁺).

### Example 36:

Yellow solid, m.p. 257-273°C; ¹H NMR (500 MHz, DMSO-d₆) d 11.73 (br s, 1H), 9.53-9.40 (2m, 1H), 8.65-8.63 (m, 1H), 8.25-7.91 (4m, 2H), 7.52-7.25 (3m, 2H), 6.21 and 6.10 (2d, *J*=8.8, 9.3 Hz, 1H), 5.47-4.99 (series of m, 3H), 4.10-3.86 (3m, 3H), 3.56-3.53 (m, 7H), 2.99-2.57 (series of m, 4H); IR (KBr, cm⁻¹) 3412, 2924, 2800, 1706, 1653, 1602, 1567, 1481, 1463, 1425, 1321, 1301, 1198, 1110, 1067, 916, 804, 764, 742; MS (+ESI, M+H⁺) *m*/*z* 610.

### Example 37

### 12-[6-(Amino-6-deoxy-β-D-glucopyranosyl)-3-fluoro-5H,13H-benzo[b]thienyl[2,3-a]pyrrolo[3,4-c]carbazole-5,7(6H)-dione

Triphenylphosphine (107 mg, 0.41 mmol) was added in one portion to a stirred solution of 12-[6-(azido-6-deoxy-β-D-glucopyranosyl)-3-fluoro-5H,13H-benzo[b]thienyl [2,3-a]pyrrolo[3,4-c]carbazole-5,7(6H)-dione (75mg, 0.14 mmol) in wet tetrahydrofuran (3 mL) under nitrogen before the mixture was heated to 50°C for 28h, cooled to ambient temperature and treated with aqueous ammonium hydroxide solution for 1h at ambient temperature and for 1h at 50°. Upon cooling to room temperature, the mixture was concentrated down *in vacuo* and the residue was taken up in methanol, acidified with IN HCl/Et₂O and evaporated to dryness. Purification of this residue by HPLC on a YMC Pack ODS (20x100) column operating at 87%B yielded the title compound (54 mg, 71%) as a yellow solid, decomp. pt. 290°C (sealed tube); ¹H NMR (500 MHz, DMSO-d₆) δ 11.55 (br s, 1H), 9.94-9.90 (m, 1H), 9.03-8.96 (2m, 1H), 8.30-8.26 (m, 4H), 8.12-8.08 (m, 1H), 7.71-7.29 (series of m, 6H), 6.36 and 6.15 (2d, *J*=8.9, 9.3 Hz, 1H), 5.81 and 5.55 (2m, 2H), 4.23-3.70 (5m, 2H), 3.60-3.57 and 2.94 (2m, 2H), 1.68-1.41 (3m, 2H); IR (KBr, cm⁻¹) 3401, 1702, 1624, 1482, 1460, 1328, 1284, 1209, 1182, 1087, 757, 746; MS (FAB, MH+) *m*/z 522.

### Example 39

### 3,9-Difluoro-12-(6-amino-6-deoxy-β-D-glucopyranosyl)benzofurano[2,3-a]-pyrrolo[3,4-c] carbazole-5,7-dione

To a magnetically stirred solution of 3,9-Difluoro-12-(β-D glucopyranosyl)benzofurano[2,3-a]pyrrolo[3,4-c]-carbazole-5,7-dione (50 mg, 0.09 mmol) and powdered molecular sieves 4 angstrom (100 mg) in anhydrous pyridine (3 mL) at -30 °C under Ar was added neat via syringe, methanesulfonyl chloride (10µl, 0.12 mmol). The reaction mixture was stirred at -30 °C for 1h, then at -10 °C for 0.5h followed by an additional portion of methanesulfonyl chloride (10µl, 0.12 mmol). This resulting mixture was then stirred at 0 °C for 1h, suction-filtered and concentrated. The residue was taken up in EtOAc/THF and washed with water, dried (Na₂SO₄) and evaporated. The resulting residue was washed with toluene to give a crude yellow product which was directly used for the following reaction without further purification. Sodium azide (62 mg, 0.95 mmol) was added to a stirred solution of 3,9-Difluoro-12-(6-O-(methylsulfonyl)-β-D-glucopyranosyl)benzofurano[2,3-a]pyrrolo[3,4-c]-carbazole-5,7-dione (54 mg, 0.09 mmol) in anhydrous DMF (3 mL) before the mixture was heated at 70 °C for 1h. The reaction mixture was cooled to ambient temperature, diluted with water, dried (Na₂SO₄) and concentrated in vacuo. The crude yellow product was directly used for the following reaction without further purification. A mixture of 3,9-Difluoro-12-(6-azido-6-deoxy- β-D-glucopyranosyl) benzofurano [2,3-a]pyrrolo[3,4-c]-carbazole-5,7-dione (Example 40) (49 mg, 0.09 mmol) and 10% palladium on charcoal (50 mg) in a mixture of ethanol (3 mL) and THF (1 mL) was hydrogenated at 1 atm for 21h. The resulting mixture was filtered, washed with methanol and THF, and concentrated in vacuo. The crude product was chromatographed (60% CH₂Cl₂: 20% THF and 20% of a solution of 90% MeOH and 10% NH₄OH) to afford the title compound (20 mg, 42%) as a yellow solid:
IR (KBr) 1753, 1701, 1479 cm⁻¹: ¹H NMR (DMSO-d₆, 400 MHz) δ 8.87-8.82 (m, 1H), 8.56-8.49 (m, 1H), 8.10-7.99 (m, 2H), 7.67-7.50 (m, 2H), 6.53 (d, 0.7 H, J=9.0 Hz), 6.14 (d, 0.3H, J=9.0 Hz), 5.44-5.30 (m, 2H), 4.28-2.86 (m, 7H): HPLC: 90.4% (247 nm).

### Example 45:

300 MHz ¹H NMR (d6-acetone) δ 11.97 (brs, 1H), 9.91 (s, 1H), 9.12 (dd, 1H), 8.88 (dd, 1H), 7.92 (dd, 1H), 7.69 (dd, 1H), 7.47-7.23 (m, 2H), 7.48 (ddd, 1H, J = 2.5, 8.9, 9.1 Hz), 7.41 (ddd, 1H, 1=2.5,8.9, 9.1 Hz), 6.73 (s, 1H, 1'H), 4.60-3.95 (m, 6H).

### Example 47:

yellow-orange solid: 500 MHz 1H NMR (CDCl3 with drop d6-DMSO) d 10.36 (d, 1H, JH-F = 4.2 Hz), 9.98 (brs, 1H), 8.81 (dd, 1H, J= 9.5, 2.6 Hz), 8.68
(dd, 1H, J= 9.5, 2.5 Hz), 7.47 (dd, 1H), 7.30 - 7.24 (m, 1H), 7.15-7.04 (m, 2H), 5.84 (d,1H, J = 8.5 Hz), 5.02 (dd, 1H, JH-F = 45.2 Hz, JH-H = 10.1 Hz), 4.72 (dd, 1H, JH-F = 49.6 Hz, JH-H = 10.1 Hz), 4.03-3.82 (m, 2H), 3.71 - 3.60 (m,2H); Neg ESI mass spectrum, m/e 524 (M-H)-.

### Example 48:

500 MHz ¹H NMR (d6-DMSO) δ 8.80-7.20 (m, 6H), 6.38 (d, 1H), 5.15-3.45 (m, 6H). ESI(NEG) mass spectrum, m/e 540 (M-H)-.

Additional Examples of formula I compounds, which may be synthesized by modifications of the foregoing synthetic procedures, are set forth in Table V wherein the substituents are the same as in Example 29 unless otherwise mentioned.

**Table V**

| Example No | R₄ | R₅ | R₆ | Q |
|---|---|---|---|---|
| 49 | OH | Cl | H | NH |
| 50 | OH | Cl | H | S |
| 51 | OH | Cl | H | O |
| 52 | OH | F | H | S |
| 53 | OH | F | H | O |
| 54 | OH | SCH3 | H | NH |
| 55 | OH | SCH3 | H | S |
| 56^{Δ} | OH | N3 | N | S |
| 57 | OH | S(O)CH3 | H | S |
| 58 | OH | S(O)CH3 | H | NH |
| 59 | OH | SCH2COOH | H | NH |
| 60^{Δ} | OH | 1-piperidine | H | S |
| 61^{Δ} | OH | thiomorpholine -S-Oxide | H | S |
| 62 | OH | S-(2-pyridine) | H | S |
| 63 | OH | S-(2-pyridine -N-Oxide) | H | S |
| 64 | OH | S-(2-(4-OH) pyrimidinyl) | H | S |
| 65 | OH | SCH2CF3 | H | S |
| 66 | OH | S(O)CH2CF3 | H | S |
| 67 | OH | S-(2-imidazole) | H | S |
| 68 | OH | NEt2 | OH | NH |
| 69 | OH | F | OH | S |
| 70 | N3 | F | H | NH |
| 71⁺ | R4=R4'=F | OH | H | NH |
| 72 | F | F | H | NH |
| 73 | NH2 | F | H | NH |
| 74 | CH3 | F | H | NH |
| 75 | N3 | H | H | NH |
| 76⁺ | R4-R4'=O | OH | H | NH |
| 77 | R4=R4'=H | F | H | NH |
| 78 | OH | F | -CH2CH2NH-(-C(NH)NH2) | NH |
| 79 | NH2 | H | H | NH |
| 80 | F | H | H | NH |
| 81 | R4=R4'=F | H | H | O |
| 82 | R4=R4'=F | H | H | S |
| 83 | F | H | H | S |
| 84 | CH3 | F | H | S |
| 85 | OCH3 | F | H | S |
| 86 | OCH3 | F | H | NH |
| 87 | N3 | OH | H | NH |
| 88 | NH2 | OH | H | NH |
| 89^{*} | OH | OCH3 | H | NH |

| | | | | |
|---|---|---|---|---|
| ^{Δ}Note that in examples 56, 60 and 61, X'₁ is hydrogen. *Note that in example 89, X₂ and X'₂ are, respectively 2- and 10- fluoro, also R_{5'} and R_{5"} taken together is = 0. | | | | |
| ⁺ Note that that in examples 71 and 76, X₂ and X'₂ are respectively 2- and 10- fluoro. | | | | |

Analytical data for some of the examples shown in Table V are given below.

### Example 50:

yellow solid, m.p. 242-248°C dec.; ¹H NMR (500 MHz, DMSO-d₆) δ 11.55 (br m, 1H), 9.75-9.57 (2m, 1H), 9.01-8.87 (2m, 1H), 8.23-7.98 (3m, 2H), 7.57-7.34 (2m, 2H), 6.33 and 6.21 (2d, *J*=8.8, 9.4 Hz, 1H), 5.69-5.61 (2m, 1H), 5.44-5.17 (2m, 2H), 4.12-3.96 (m, 4H), 3.66-3.55 (2m, 2H); IR (KBr, cm⁻¹) 3392, 2926, 1703, 1622, 1602, 1567, 1481, 1463, 1426, 1324, 1198, 1085, 915, 806, 763, 742; MS (-ESI, M-H-) *m*/*z* 557.

### Example 52:

yellow solid, m.p. 248-250°C; ¹H NMR (500 MHz, DMSO-d₆) δ 11.73, 11.65 and 11.62 (3s, 1H), 9.81-9.68 (2m, 1H), 9.07-8.99 (2m, 1H), 8.27-8.00 (m, 2H), 7.61-7.50 (m, 1H), 6.38 and 6.26 (2d, *J*=8.8, 9.3 Hz, 1H), 5.69-5.21 (series of m, 3H), 5.05-4.82 (m, 2H), 4.25-3.82 (m, 2H), 3.72-3.61 (m, 1H); IR (KBr, cm⁻¹) 3384, 1706, 1622, 1602, 1568, 1481, 1463, 1325, 1198, 1086, 916, 806, 763, 742; MS (-ESI, M-H-) *m/z* 541.

### Example 54:

300 MHz ¹H NMR (CD₃OD) d 8.80 (dd,1 H, J = 9.6, 2.3 Hz), 8.72 (dd, 1H, J = 9.9, 2.8 Hz), 7.84-7.73 (m, 2H), 7.37-7.26 (m, 2 H), 6.15 (d, 1 H, J = 9.5 Hz), 4.34-4.27 (m, 1 H), 4.14 (t, 1 H, J = 9.5 Hz), 3.97 (t, 1 H, J = 9.0 Hz), 3.74 (t, 1 H, J = 9.0 Hz), 3.45 (dd, 1 H, J = 14.8, 1.8 Hz), 3.15 (dd, 1H, J = 14.8, 3.9 Hz), 2.19 (s, 3 H); Neg ESI mass spectrum, m/e 552 (M-H)

### Example 56

### 12-[6-(Azido-6-deoxy-β-D-glucopyranosyl)-3-fluoro-5H,13H-benzo[b]thienyl[2,3-a]pyrrolo[3,4-c]carbazole-5,7(6H)-dione.

Sodium azide (216 mg, 3.30 mmol) was added in one portion to a stirred solution of 3-fluoro-13-[6-O-(methylsulfonyl)-β-D-glucopyranosyl]-5H,13H-benzo[b]thienyl[2,3-a]pyrrolo[3,4-c]carbazole-5,7(6H)-dione (0.20 g, 0.33 mmol) in anhydrous dimethylformamide (3 mL) before the mixture was heated to 120°C for 3h, cooled to ambient temperature and concentrated down *in vacuo.* The residue was purified by flash chromatography on silica gel (elution with 40% tetrahydrofuran in hexanes) to yield the title compound (141 mg, 78%) as a yellow solid, decomp. pt. 265°C; ¹H NMR (500 MHz, DMSO-d₆) δ 11.51 (br s, 1H), 9.94-9.87 (2m, 1H), 9.03-8.95 (2m, 1H), 8.20-7.99 (3m, 2H), 7.67-7.63 (m, 2H), 7.53-7.49 (m, 1H), 6.37 and 6.20 (2d, *J*=8.9 and 9.4 Hz, 1H), 5.54-5.14 (series of m, 3H), 4.10-3.76 (4m, 3H), 3.67-3.26 (m, 3H); IR (KBr, cm⁻¹) 3332, 2103, 1702, 1481, 1461, 1431, 1372, 1324, 1283, 1230, 1211, 1178, 1079, 746; MS (-ESI, M-H⁻) *m/z* 546.

### Example 57

Yellow solid, m.p. 268-277°C (dec.); ¹H NMR (500 MHz, DMSO-d₆) δ 11.52 (br s, 1H), 9.75-9.51 (3m, 1H), 9.00-8.88 (2m, 1H), 8.30-7.97 (3m, 2H), 7.66-7.22 (3m, 2H), 6.35-6.20 (2m, 1H), 5.86-5.56 (m, 1H), 5.42-5.35 (m, 2H), 4.28-3.89 (m, 2H), 3.72-3.56 (m, 2H), 3.50-3.32 (m, 1H), 3.27-3.14 (m, 1H), 2.61-2.53 (4s, 3H); IR (KBr, cm⁻¹) 3412, 2950, 1707, 1625, 1605, 1481, 1464, 1385, 1324, 1198, 1086, 916, 764, 742; LCMS (-ESI, M-H⁻) *m*/*z* 685.

### Example 58:

yellow-orange solid (mixture of diasteriomers at sulfur): 500 MHz ¹H NMR (d6-DMSO; mixture of 2 diasteriomers and rotomers) d 8.85-8.74 (m, 2 H), 8.15-7.75 (m, 2 H), 7.55-7.35 (m, 2 H), 6.45-6.10 (m, 1H), 5.80-5.25 (m, 3 H), 4.60-3.10 (m, 6 H), 2.76, 2.74, 2.58, and 2.56 (all s, 3 H); Neg ESI mass spectrum, m/e 568 (M-H)⁻.

### Example 59

300 MHz ¹H NMR (CD₃OD) d 8.87 (dd, 1H, J=9.6, 2.6 Hz), 8.78 (dd, 1H, J=9.8, 2.6 Hz), 7.94 (dd, 1H, J=8.9, 4.4 Hz), 7.83 (dd, 1H, J=9.0, 4.1 Hz), 7.38-7.27 (m, 2H), 6.14 (d, 1H, J=9.3 Hz), 4.34-4.18 (m, 2H), 3.94 (t, 1H, J=9.2 Hz), 3.73 (t, 1H, J=8.9 Hz), 3.51-3.22 (m, 4H). Neg ESI mass spectrum, m/e 596 (M-H)⁻.

### Example 60:

yellow solid, mp 228-232°C; ¹H NMR (500 MHz, DMSO-d₆) δ 11.88 and 11.76 (2br m, 1H), 9.75-9.69 (2m, 1H), 8.73-8.62 (2m, 1H), 8.18 and 8.09 (2d, *J*=4.8, 4.7 Hz, 1H), 7.99-7.92 (2m, 1H), 7.65-7.35 (m, 3H), 6.28 and 6.07 (2d, *J*=8.9, 9.2 Hz, 1H), 5.29-4.97 (3m, 3H), 4.10-3.85 (3m, 2H), 3.60-3.48 (m, 2H), 2.97-2.70 (3m, 2H), 2.58 (m, 3H), 1.56-1.44 (m, 5H), 1.34 (m, 2H); IR (KBr, cm⁻¹) 3406, 2934, 1705, 1622, 1481, 1461, 1430, 1374, 1321, 1284, 1179, 1081, 1038, 758, 747; MS (+ESI, M+H⁺) *m/z* 590.

### Example 61:

yellow solid, mp 228-232°C; ¹H NMR (500 MHz, DMSO-d₆) δ 11.88 and 11.76 (2br m, 1H), 9.75-9.69 (2m, 1H), 8.73-8.62 (2m, 1H), 8.18 and 8.09 (2d, *J*=4.8, 4.7 Hz, 1H), 7.99-7.92 (2m, 1H), 7.65-7.35 (m, 3H), 6.28 and 6.07 (2d, *J*=8.9, 9.2 Hz, 1H), 5.29-4.97 (3m, 3H), 4.10-3.85 (3m, 2H), 3.60-3.48 (m, 2H), 2.97-2.70 (3m, 2H), 2.58 (m, 3H), 1.56-1.44 (m, 5H), 1.34 (m, 2H); IR (KBr, cm⁻¹) 3406, 2934, 1705, 1622, 1481, 1461, 1430, 1374, 1321, 1284, 1179, 1081, 1038, 758, 747; MS (+ESI, M+H⁺) *m/z* 590.

### Example 62:

yellow solid, m.p. 280-282°C; ¹H NMR (500 MHz, DMSO-d₆) δ 11.64 (br s, 1H), 9.83-9.68 (2m, 1H), 9.08-8.97 (2m, 1H), 8.45-7.10 (series of m, 8H), 6.37 and 6.25 (2d, *J*=8.8, 9.1 Hz, 1H), 5.74-5.68 (2m, 1H), 5.50-5.21 (2m, 2H), 4.20-4.04 (2m, 4H), 3.71-3.60 (m, 2H); IR (KBr, cm⁻¹) 3369, 2918, 1752, 1712, 1601, 1580, 1558, 1482, 1456, 1413, 1319, 1256, 1197, 1094, 1020, 914, 805, 762; MS (-ESI, M-H⁻) *m/z* 632.

### Example 63:

yellow solid, decomp. pt 248°C; ¹H NMR (500 MHz, DMSO-d₆) δ 11.65 (br s, 1H), 9.78-9.73 (2m, 1H), 9.07-8.97 (2m, 1H), 8.30-7.95 (series of m, 3H), 7.61-7.42 (2m, 3H), 7.15-7.06 (m, 2H), 6.37 and 6.25 (2d, *J*=8.8, 9.3 Hz, 1H), 5.81-5.22 (series of m, 3H), 4.32-4.12 (3m, 3H), 3.77-3.63 (m, 3H); IR (KBr, cm⁻¹) 3401,2963,1752,1707,1475,1465,1424,1261,1198,1092,1021, 801; LCMS (+ESI, M+H⁺) *m*/*z* 650.

### Example 64

Obtd 15.1mg (49%), yellow solid, m.p. 254-265°C (dec.); ¹H NMR (500 MHz, DMSO-d₆) δ 11.61 (br s, 1H), 9.76-9.66 (2m, 1H), 9.01-8.92 (2m, 1H), 8.31-7.96 (3m, 2H), 7.78 (br s, 1H), 7.58-7.45 (m, 2H), 6.30 and 6.19 (2d, *J*=8.9, 9.4 Hz, 1H), 5.99 (br s, 1H), 5.50-5.16 (series of m, 3H), 4.12-3.92 (3m, 3H), 3.61-3.39 (m, 3H); IR (KBr, cm⁻¹) 3392, 2927, 1706, 1661, 1604, 1567, 1532, 1480, 1463, 1384, 1324, 1085, 916, 826, 807, 764, 742; MS (-ESI, M-H⁻) *m/z* 649.

### Example 65:

yellow solid, m.p. 244-246°C; ¹H NMR (500 MHz, DMSO-d₆) δ 11.63 (br s, 1H), 9.75-9.67 (2m, 1H), 9.00-8.94 (2m, 1H), 8.28-8.20 (2m, 1H), 8.11-7.97 (2m, 1H), 7.57-7.50 (m, 2H), 6.30 and 6.16 (2d, *J*=8.8, 9.3 Hz, 1H), 5.63-5.19 (series of m, 3H), 4.05-4.02 (m, 1H), 3.61-3.07 (m, 6H); IR (KBr, cm⁻¹) 3412, 1762, 1703, 1602, 1482, 1464, 1425, 1315, 1259, 1199, 1082, 916, 807, 763, 742; MS (-ESI, M-H⁻) *m*/*z* 637.

### Example 66:

Obtd 4.7 mg (55%), yellow solid, m.p. 252-254°C (dec.); ¹H NMR (500 MHz, DMSO-d₆) δ 11.60 (br s, 1H), 9.83-9.71 (2m, 1H), 9.07-9.01 (2m, 1H), 8.30-8.07 (3m, 2H), 7.65-7.56 (2m, 2H), 6.43-6.30 (2m, 1H), 5.73-5.71 (m, 1H), 5.55-5.20 (2m, 2H), 4.21-4.07 (m, 4H), 3.81-3.52 (2m, 4H); IR (KBr, cm⁻¹) 3384, 2926, 1706, 1622, 1602, 1568, 1482, 1464, 1426, 1384, 1309, 1259, 1230, 1081, 916, 805, 763, 742; LCMS (-ESI, M-H⁻) *m*/*z* 653.

### Example 67

Obtd 10.5 mg (26%), yellow solid, decomp. pt 290°C; ¹H NMR (500 MHz, DMSO-d₆) δ 12.34 and 12.23 (2s, 1H), 11.58 (s, 1H), 9.75-9.63 (2m, 1H), 9.01-8.92 (2m, 1H), 8.23-8.15 (m, 1H), 8.17-7.96 (m, 1H), 7.53-7.46 (m, 2H), 7.11-6.85 (series of m, 2H), 6.32 and 6.15 (2d, *J*=8.9, 9.3 Hz, 1H), 5.85-5.14 (series of m, 3H), 4.11-3.98 (m, 2H), 3.71-3.49 (m, 4H); IR (KBr, cm⁻¹) 3242, 2926, 1751, 1705, 1602, 1481, 1463, 1426, 1325, 1198, 1086, 915, 763, 742; MS (-ESI, M-H⁻) *m*/*z* 621.

### Example 71

### 2,3,9,10-Tetrafluoro-12-(4-deoxy-4,4-difluoro-b-D-glucopyranosyl)indolo[2,3-a]pyrrolo-[3,4-c]carbazole-5,7-dione

To a solution of Dess-Martin periodinane (0.470 g, 1.11 mmol) in 30 mL of dichloromethane was added dropwise a solution of 6-(4-*tert*-butylbenzyl)-2,3,9,10-tetrafluoro-12-(2,3,6-tri-O-benzyl-b-D-glucopyranosyl)indolo[2,3-a][pyrrolo [3,4-c]-carbazole-5,7-dione (0.540 g, 0.55 mmol) in 20 mL of dichloromethane and the mixture was stirred at room temperature under Ar for 1 h. Another 0.470g (1.11 mmol) of Dess-Martin reagent was added and stirring was continued for 2 h. The resulting mixture was diluted with dichloromethane, washed (sat. NaHCO₃-Na₂S₂O₃, sat. NaHCO₃, brine), dried (Na₂SO₄) and evaporated. The residue was chromatographed (SiO₂/hexane-ethyl acetate, 2:1) to give the ketone (0.360 g, 67%) as a dark yellow solid.

To a portion of the ketone (0.067 g, 0.07 mmol) in 2 mL of dichloromethane was added DAST (0.036 mL, 0.28 mmol) and the mixture was stirred at room temperature under Ar for 18 h. The mixture was then partitioned with dichloromethane-sat. NaHCO₃ and the organic phase was separated, washed (brine), dried (Na₂SO₄) and evaporated to give a gum. Flash chromatography (SiO₂/hexane-ethyl acetate, 3:1) gave 6-(4-*tert*-butylbenzyl)-2,3,9,10-tetrafluoro-12-(4-deoxy-4,4-difluoro-2,3,6-tri-O-benzyl-b-D-glucopyranosyl)-indolo[2,3-a]pyrrolo[3,4-c]carbazole-5,7-dione (0.054 g, 54%) as a yellow solid:
¹H NMR (CDCl₃, 400 MHz) d 10.21 (s, 1H), 9.14 (dd, J=10.6, 8.4 Hz, 1H), 9.02 (dd, J=10.7, 8.2 Hz, 1H), 7.53 (d, J=8.7 Hz, 2H), 7.40 (d, J=8.7 Hz, 2H), 7.34 (m, 5H), 7.20-7.12 (m, 6H), 6.97 (dd, J=10.0, 6.6 Hz, 1H), 6.80 (t, J=7.4 Hz, 1H), 6.72 (t, J=7.6 Hz, 2H), 6.13 (d, J=7.2 Hz, 2H), 5.82 (d, J=8.6 Hz, 1H), 4.97 (m, 3H), 4.80 (d, J=11.1 Hz, 1H), 4.65 and 4.61 (ab q, J=12.0 Hz, 2H), 4.24-4.01 (m, 6H), 3.37 (d, J=10.6 Hz, 1H), 1.28 (s, 9H).

This material was deprotected in the usual manner (i. aq. NaOH, THF-EtOH; conc. HCl; ii. NH₄OAc, D; iii. H₂, Pd(OH)₂-C, CHCl₃-MeOH) to give the title compound (24 % overall yield) as a yellow solid:
IR (KBr) 3410, 1747, 1704, 1596, 1478, 1323 cm⁻¹;
¹H NMR (DMSO-d₆, 400 MHz ) d 11.39 (s, 1H), 11.34 (s, 1H), 8.93 (m, 2H), 8.03 (dd, J=11.8, 6.8 Hz, 1H), 7.62 (dd, J=10.7, 6.9 Hz, 1H), 6.54 (d, J=9.2 Hz, 1H), 6.08 (m, 2H), 5.48 (d, J=5.9 Hz, 1H), 4.43 (m, 1H), 4.22 (m, 1H), 4.06 (m, 2H), 3.66 (m, 1H).
MS (ESI) m/e 578 (M-H).
HPLC: 91.1% (320 mn).

### Example 75:

yellow solid: 500 MHz ¹H NMR (d6-DMSO) δ 11.85 (s, 1H), 11.18 (s, 1H), 8.92 (dd, 1H), 8.81 (dd, 1H), 8.06 (dd, 1H), 7.78 (dd, 1H), 7.49-7.39 (m, 2H), 6.28 (d, 1H, 1'H, J=8.8 Hz), 5.85 (d, 1H, 3'OH), 5.45 (d, 1H, 2'OH), 4.25 - 3.89 (m, 3H, 2'H, 3'H, 5'H), 3.68 (t, 1H, 4'H), 1.42 (d, 3H, 6'H); Negative ESI mass spectrum, m/e 531 (M-H)⁻. IR (KBr) 2112 cm⁻¹.

### Example 79

yellow solid: 500 MHz ¹H NMR (d6-DMSO) δ 12.72 (s, 1H), 11.18 (brs, 1H), 8.92 (dd, 1H), 8.80 (dd, 1H), 8.21 (brs, 2H), 8.07 (dd, 1H), 7.94 (dd, 1H), 7.48-7.39 (m, 2H), 6.60 (d, 1H J = 7.8 Hz, 1'H), 5.98 (brs, 1H, 3'OH), 5.48 (brs, 1H, 2'OH), 4.55 - 4.50 (m, 1H, 5'H), 4.07 - 4.02 (m, 2H, 2'H, 3'H), 3.19 (t, 1H, 4'H), 1.43 (d, 3H, 6'CH₃); Negative ESI mass spectrum 505 (M-H).

### Example 87:

yellow solid: 300 MHz ¹H NMR (CD₃OD) δ 8.84 (dd, 1 H), 8.74 (dd, 1H), 7.77 (dd, 1H), 7.35-7.24 (m, 2 H), 6.14 (d, 1H, J = 9.2 Hz), 4.23-3.95 (m, 4 H), 3.90 (t, 1H, J = 9.2 Hz), 3.74 (t, 1H, J = 9.0 Hz); IR (KBr) 2114 cm⁻¹ ; Neg ESI mass spectrum, m/e 547 (M-H)⁻.

### Example 88:

yellow solid 500 MHz ¹H NMR (CD₃OD) δ 8.89 (dd, 1H), 8.77 (dd, 1H), 7.81 (dd, 1H), 7.63 (dd, 1H), 7.33-7.26 (m, 2 H), 6.15 (d, 1H, J = 8.9 Hz), 4.24-3.88 (m, 3 H), 3.73 (t, 1H; J = 8.9 Hz), 3.59 (dd, 1 H, J = 9.6, 8.9 Hz), 3.54 (t, 1 H, J = 9.6 Hz); Neg ESI mass spectrum, m/e 521 (M-H)⁻.

### Example 89:

yellow solid: IR (KBr) 3435, 3345, 1740, 1713, 1477, 1320 cm⁻¹; ¹H NMR (THF-d₈, 400 MHz) δ 11.50 (s, 1H), 10.12 (s, 1H), 9.08 (dd, J=11.0, 8.6 Hz, 1H), 8.99 (dd, J=11.0, 8.4 Hz, 1H), 7.71 (dd, J=11.5, 6.6 Hz, 1H), 7.63 (dd, J=10.3, 6.7 Hz, 1H), 6.11 (d, J=8.9 Hz, 1H), 5.30 (d, J=4.5 Hz, 1H), 5.01 (d, J=4.1 Hz, 1H), 4.62 (d, J=5.1 Hz, 1H), 4.56 (d, J=9.7 Hz, 1H), 4.00 (s, 3H), 3.97-3.91 (m, 1H), 3.78-3.54 (m, 2H).

### Example 90

### 3,9-Difluoro-12,13-dihydro-13-( 2-fluoro-b-D-glucopyranosyl)-5(H)indolo[2,3-a] pyrrolo[3,4-c] carbazole-5,7-dione.

Employing the Mitsunobu procedure described earlier in Example 22, 250 mg (0.465 mmol) of the product from example 6 and 210 mg (0.464 mmol) of 2-Fluoro-3,4,6-Tri-O-benzyl-D-glucopyranose (prepared by treating commercially available Tri-O-benzyl-D-glucal (1.5 g, 3.6 mmol) with xenon difluoride (1.0 g, 5.91 mmol) in acetonitrile (50 mL) and water (5 mL) for 3 h at room temperature. Typical workup followed by flash chromatography on silica gel with 5% ethyl acetate in methylene chloride gave 380 mg (23.4%) of the pure 2-Fluoro-3,4,6-Tri-O-benzyl-D-glucopyranose. This procedure was a modification of a published method: See T. Hayashi, B. W. Murray, R. Wang, and C.-H. Wong Bioorganic and Medicinal Chemistry, **1997**, 5, 497-500.). Following purification by flash chromatography using 20-60 % methylene chloride in hexane, 120 mg (28%) of reasonably pure glcosylated product was obtained: Removal of the benzyl protecting groups by using transfer hydogenation conditions (95%EtOH/Cyclohexene/20%Pd(OH)2/C, reflux for 6-48hrs) followed by removal of the 4-t-bytylbenzyl protecting group under base hydrolusis conditions (4.45M KOH, EtOH/reflux; conc. Hcl; NH4OAc/EtOH/heat for 8-48hrs) provided after purification by flash chromatography on silica gel with acetone : methylene chloride : ethyl acetate, followed by further purification on Sephadex LH-20 in methanol gave 15 mg (39% for the 2 deblocking steps) of the pure title compound as a yellow-orange solid: 300 MHz ¹H NMR (d6-DMSO) d 11.68 (brs, 1H), 11.28, (brs, 1 H), 8.88 (dd, 1H), 8.78 (dd, 1H), 8.10 (dd, 1H), 7.68 (dd, 1 H), 7.60-7.43 (m, 2H), 6.77 (dd,1H, J = 8.8, 2.5 Hz), 6.23 (brs, 1 H), 5.77 (brs, 1 H), 5.62 (brs, 1 H), 4.32 (dt, 1 H, J = 50.7, 9.0 Hz), 4.15-3.80 (m, 5 H); Neg ESI mass spectrum, m/e 524 (M-H)⁻.

### Example 91

### 3,9-Difluoro-12-(β-D-glucopyranosyl)benzofurano[2,3-a]pyrrolo[3,4-c]-carbazole-5,7-dione

Yellow solid
IR (KBr) 1757, 1706, 1483 cm⁻¹.
¹H NMR (DMSO-d₆, 400 MHz) δ 11.45 (brs, 1H), 8.84-8.79 (m, 1H), 8.53-8.46 (m, 1H), 8.08-7.81 (m, 1H), 7.64-7.50 (m, 2H), 6.49 (d, 0.7 H, J=8.9 Hz), 6.07 (d, 0.3 H, J=8.9 Hz), 5.38-5.16 (m, 3H), 4.75-4.21 (m, 1H), 3.93-3.49 (m, 6H). HPLC: 97.5% (305 nm).

### Example 92

### 3-Bromo-9-fluoro-12-(β-D-glucopyranosyl)benzofurano[2,3-a]pyrrolo[3,4-c]-carbazole-5,7-dione

Yellow solid
IR (KBr) 1775, 1708 cm⁻¹.
¹H NMR (DMSO-d₆, 400 MHz) δ 11.69 and 11.49 (2 brs, 1H), 8.97 (d, 0.3H, J=2.0 Hz), 8.91 (d, 0.7H, J=2.0 Hz), 8.85-8.79 (m, 1H), 8.08-7.77 (m, 3H), 7.59-7.49 (m, 1H), 6.47 (d, 0.7H, J=9.0 Hz), 6.07 (d, 0.3H, J=9.0 Hz), 5.37-5.21 (m, 3H), 4.79-4.20 (m, 1H), 3.92-3.54 (m, 6H).
HPLC: 92.5% (260 nm).

### Example 93

### 3-Cyano-9-fluoro-12-(β-D-glucopyranosyl)benzofurano[2,3-a]pyrrolo[3,4-c]-carbazole-5,7-dione

3-Bromo-9-fluoro-12-(2,3,4,6-tetra-O-benzyl-β-D-glucopyranosyl)benzofurano [2,3-a]pyrrolo[3,4-c]-carbazole-5,7-dione (189 mg, 0.2 mmol) was combined with zinc cyanide (14 mg, 0.6 mmol) and tetrakis-(triphenylphosphine) palladium(0) (12 mg, 0.01 mmol) in deoxygenated DMF (2mL) and the yellow slurry was heated to 80 °C under nitrogen for 16h. The mixture was cooled to room temperature, diluted with EtOAc, washed with water and brine, dried (Na₂SO₄) and evaporated. The resulting residue was chromatographed (Hex:EtOAc, 7:3) to give 3-Cyano-9-fluoro-12-(2,3,4,6-tetra-O-benzyl-β-D-glucopyranosyl)benzofurano[2,3-a]pyrrolo[3,4-c]-carbazole-5,7-dione (160 mg, 90%) as a yellow solid: IR (KBr) 2235 cm⁻¹. To a solution of 3-Cyano-9-fluoro-12-(2,3,4,6-tetra-O-benzyl-β-D-glucopyranosyl)benzofurano[2,3-a]pyrrolo[3,4-c] -carbazole-5,7-dione (58 mg, 0.06 mmol)in 5 mL of dry CH₂Cl₂ was added dropwise boron trichloride (0.39 mL, 1.0 M sol. in CH₂Cl₂) at -78 °C. The resulting solution was warmed up to 0 °C and stirred for 2h and cooled back to -78 °C before the addition of methanol (5 mL). This resulting mixture was warmed up to ambient temperature. The solvent was removed in vacuo and the remaining residue was taken up in EtOAc/THF and washed with 10% aq. HCl and brine prior to drying (Na₂SO₄) and solvent concentration. Purification of the residue by preparative TLC (THF:Hex, 9:1) yielded the title compound (18 mg, 53%) as a yellow solid: IR (KBr) 3417, 2220, 1757, 1708, 1635, 1478 cm⁻¹: ¹H NMR (DMSO-d₆, 400 MHz) δ 9.05 (s, 0.3H), 8.90 (s, 0.7H), 8.70-8.67 (m, 1H), 8.16-7.95 (m, 3H), 7.48-7.45 (m, 1H), 6.42 (d, 0.7H, J=8.3 Hz), 6.11 (d, 0.3H, J=8.3 Hz), 5.45-5.37 (m, 3H), 4.17-3.50 (7H): HPLC: 91.4% (260 nm).

### Example 94

### 3-Iodo-9-fluoro-12-(β-D-glucopyranosyl)benzofurano[2,3-a]pyrrolo[3,4-c]-carbazole-5,7-dione

3-Bromo-9-fluoro-12-(2,3,4,6-tetra-O-benzyl-β-D-glucopyranosyl)benzofurano [2,3-a]pyrrolo[3,4-c]-carbazole-5,7-dione (189 mg, 0.2 mmol) was combined with bis(tributyltin) (0.2 mL, 0.4 mmol) and tetrakis-(triphenylphosphine) palladium(0) (23 mg, 0.02 mmol) in deoxygenated NMP (2mL) and the yellow slurry was heated to 90 °C under nitrogen for 18h. The mixture was cooled to room temperature, diluted with EtOAc, washed with water and brine, dried (Na₂SO₄) and evaporated. The resulting residue was chromatographed (Hex:EtOAc, 4:1) to give 3-Tributylstannyl-9-fluoro-12-(2,3,4,6-tetra-O-benzyl-β-D-glucopyranosyl)benzofurano[2,3-a]pyrrolo[3,4-c]-carbazole-5,7-dione (150 mg, 90%) as a yellow oil. To a solution of this yellow oil in 5 ml of CHCl₃ was added I₂ (33mg, 0.13 mmol) and the mixture was stirred at room temperature for 0.5h. The resulting mixture was treated with saturated NaHSO₃, washed with water, brine, dried and evaporated. This crude residue (118 mg, 91%) was treated with boron trichloride (0.71 mL, 1.0 M sol. in CH₂Cl₂) as previously described to give the title compound as a yellow solid: IR (KBr) 3140, 3040, 1753, 1703, 1405 cm⁻¹: ¹H NMR (DMSO-d₆ and D₂O exchange, 400 MHz) δ 9.05 (d, 0.3H, J=1.7 Hz), 8.89 (d, 0.7H, J=1.7 Hz), 8.67 (dd, 0.3H, J=9.5, 2.7 Hz), 8.62 (dd, 0.7H, J=9.5, 2.7 Hz), 7.98-7.64 (m, 3H), 7.47-7.41 (m, 1H), 6.42 (d, 0.7H, J=9.0 Hz), 6.00 (d, 0.3H, J=9.0 Hz), 4.26-3.50 (m, 6H): HPLC: 94.9% (320 nm).

### Example 95

### 6-(4-tert-Butylbenzyl)-2,3,9,10-tetrafluoro-12-(2,3,4,6-tetra-O-benzyl-β-D-gluco-pyranosyl)indolo[2,3-a]pyrrolo[3,4-c]-carbazole-5,7-dione

To a suspension of 6-(4-*tert*-butylbenzyl)-2,3,9,10-tetrafluoroindolo[2,3-a]-pyrrolo[3,4-c]-carbazole-5,7-dione (1.131 g, 2.08 mmol) and anhydrous Na₂SO₄ (5.0 g) in 25 mL of dry THF was added finely pulverized KOH (0.932 g, 16.6 mmol). The resulting mixture was vigorously stirred at room temperature under Ar for 1.5 h. To the resulting dark purple mixture was added a solution of 2,3,4,6-tetra-O-benzyl-α-D-glucopyranosyl chloride (1.450 g, 2.60 mmol) in 10 mL of dry THF and stirring was continued for 24 h. An additional 0.200 g (0.36 mmol) of the chlorosugar was added and stirring was continued for another 24 h. The mixture was then diluted with ethyl acetate and quenched with 1 N HCl. The organic phase was separated, washed (brine), dried (Na₂SO₄) and evaporated to give a yellow foam. Flash chromatography (pre-adsorbed on SiO₂; eluted with hexane-ethyl acetate, 5:1) gave the product as a bright yellow glass. This glass was taken up in dichloromethane and the solution was diluted with methanol. Concentration of this solution on the rotovap, followed by drying *in vacuo*, gave the title compound (1.240 g, 54%) as a bright yellow solid:
IR (KBr) 3307, 1748, 1694, 1593, 1473, 1072 cm⁻¹;
¹H NMR (CDCl₃, 400 MHz) δ 10.58 (s, 1H), 9.18 (dd, J=10.7, 8.2 Hz, 1H), 9.07 (dd, J=10.8, 8.2 Hz, 1H), 7.53 (d, J=8.3 Hz, 2H), 7.41 (m, 6H), 7.28 (m, 8H), 7.22 (m, 4H), 6.94 (dd, J=10.1, 6.5 Hz, 1H), 6.88 (t, J=7.3 Hz, 1H), 6.81 (t, J=7.3 Hz, 2H), 6.16 (d, J=7.2 Hz, 2H), 5.75 (d, J=8.4.Hz, 1H), 5.03 (d, J=10.8 Hz, 1H), 4.96 (m, 2H), 4.90 (m, 2H), 4.78 (d, J=10.8 Hz, 1H), 4.64 (d, J=12.2 Hz, 1H), 4.58 (d, J=12.2 Hz, 1H), 4.29 (t, J=10.4 Hz, 1H), 4.07 (d, J=10.1 Hz, 1H), 4.00-3.85 (m, 5H), 3.15 (d, J=9.2 Hz, 1H), 1.28 (s, 9H). Anal. Calcd for C₆₅H₅₅F₄N₃O₇: C, 73.22; H, 5.20; N, 3:94. Found: C, 72.92;
H, 5.58; N, 4.02.

### Example 96

### 2,3,9,10-Tetrafluoro-12-(β-D-glucopyranosyl)indolo[2,3-a]pyrrolo[3,4-c]-carbazole-5,7-dione

IR (KBr) 3432, 3310, 1743, 1702, 1475, 1331 cm⁻¹;
¹H NMR (DMSO-d₆, 400 MHz) δ 11.86 (s, 1H), 11.31 (s, 1H), 8.99 (dd, J=11.1, 8.5 Hz, 1H), 8.92 (dd, J=11.2, 8.3 Hz, 1H), 8.18 (dd, J=11.7, 6.9 Hz, 1H), 7.62 (dd, J=11.0, 7.0 Hz, 1H), 6.26 (d, J=9.0 Hz, 1H), 6.19 (t, J=4.2 Hz, 1H), 5.43 (d, J=4.8 Hz, 1H), 5.17 (d, J=5.7 Hz, 1H), 4.99 (d, J=5.6 Hz, 1H), 4.10 (dd, J=10.7, 4.2 Hz, 1H), 3.92 (m, 2H), 3.82 (m, 1H), 3.57 (m, 1H), 3.41 (m, 1H).
HPLC: 97.1% (320 nm).

### Example 97

### 2,3,9,10-Tetrafluoro-12-(6-fluoro-6-deoxy-β-D-glucopyranosyl)indolo[2,3-a]pyrrolo[3,4-c]carbazole-5,7-dione

IR (KBr) 3440, 3365, 1750, 1705, 1478 cm⁻¹.
¹H NMR (DMSO-d₆, 400 MHz) δ 12.04 (s, 0.5 H), 11.31 (s, 0.5 H), 11.27 (s, 0.5 H), 10.68 (s, 0.5 H), 9.07 (dd, J=11.3, 8.8 Hz, 0.5 H), 8.95 (dd, J=19.4, 9.2 Hz, 1H), 8.87 (dd, J=10.9, 8.9 Hz, 0.5 H), 8.17 (dd, J=11.9, 6.9 Hz, 0.5 H), 7.91 (dd, J=11.5, 7.1 Hz, 0.5 H), 7.71 (dd, J=10.7, 6.9 Hz, 0.5 H), 7.50 (dd, J=9.6, 6.9 Hz, 0.5 H), 6.34 (d, J=8.8 Hz, 0.5 H), 6.32 (d, J=7.9 Hz, 0.5 H), 5.98-4.73 (m, 5H), 4.22-3.46 (m, 4H).
HPLC: 98.2% (320 nm).

### Example 98

### 6-(4-tert-Butylbenzyl)-2,3,9,10-tetrafluoro-12-(2,3,6-tri-O-benzyl-β-D-galactopyranosyl)indolo[2,3-a]-pyrrolo[3,4-c]carbazole-5,7-dione

A solution of 6-(4-*tert*-butylbenzyl)-2,3,9,10-tetrafluoro-12-(2,3,6-tri-O-benzyl-4-O-(4-methoxybenzyl)-β-D-galactopyranosyl)indolo[2,3-a]pyrrolo[3,4-c]carb-azole-5,7-dione (0.135 g, 0.12 mmol) in 10 mL of 10% TFA-CH₂Cl₂ was stirred at room temperature under Ar for 20 min. The resulting mixture was diluted with dichloromethaneand then it was washed (sat. NaHCO₃), dried (MgSO₄) and evaporated. The resulting residue was chromatographed (SiO₂/ethyl acetate-hexane, 1:2) to afford the title compound (0.107 g, 90%) as a yellow solid:
¹H NMR (DMSO-d₆, 400 MHz) δ 12.21 (s, 1H), 9.00 (dd, J=11.1, 8.4 Hz, 1H), 8.90 (dd, J=11.2, 8.4 Hz, 1H), 8.11 (dd, J=12.0, 6.9 Hz, 1H), 7.61-7.18 (m, 15H), 6.71 (t, J=7.4 Hz, 1H), 6.61 (t, J=7.7 Hz, 2H), 6.39 (d, J=9.2 Hz, 1H), 6.18 (d, J=7.2 Hz, 2H), 4.89 (s, 2H), 4.87 (d, J=13.9 Hz, 1H), 4.71 (d, J=12.1 Hz, 1H), 4.58 (s, 1H), 4.49 (s, 2H), 4.33 (m, 1H), 4.20 (m, 2H), 4.00-3.66 (m, 4H), 3.58 (d, J=11.2 Hz, 1H), 1.26 (s, 9H).

### Example 99

### 6-(4-tert-Butylbenzyl)-2,3,9,10-tetrafluoro-12-(2,3,6-tri-O-benzyl-4-deoxy-β-D-galactopyranosyl)indolo[2,3-a]-pyrrolo[3,4-c]carbazole-5,7-dione

To a solution of 6-(4-*tert*-butylbenzyl)-2,3,9,10-tetrafluoro-12-(2,3,6-tri-O-benzyl-β-D-galactopyranosyl)indolo[2,3-a]-pyrrolo[3,4-c]carbazole-5,7-dione (0.402 g, 0.41 mmol) in 10 mL of acetonitrile was added DMAP (0.100 g, 0.82 mmol), followed by phenyl chlorothionoformate (0.085 g, 0.49 mmol) and the mixture was heated to reflux under Ar for 19 h. An additional 0.043 g (0.25 mmol) of phenyl chlorothionoformate and 0.030 g (0.25 mmol) of DMAP were then added and the mixture was heated to reflux for 19 h. The cooled mixture was partitioned with ethyl acetate and saturated NaHCO₃ and the organic phase was washed, dried and evaporated. The residue was flash chromatographed (SiO₂/ethyl acetate-hexane, 1:3) to give the thionocarbonate (0.320 g, 0.29 mmol, 70%) as a solid. This material was dissolved in 10 mL of toluene, the solution was purged with a stream of Ar bubbles for 15 min and then AIBN (0.010 g, 0.06 mmol) and tributlytin hydride (0.126 g, 0.43 mmol) were added. The resulting solution was heated to reflux under Ar for 18 h. Another 0.126 g of tributyltin hydride and 0.010 g of AIBN were added and refluxing was continued for 4 h. The cooled mixture was evaporated and the residue was chromatographed (SiO₂/ethyl acetate-hexane, 1:2) to give the title compound (0.215 g, 78%) as a yellow solid:
IR cm⁻¹;
¹H NMR (CDCl₃, 400 MHz) δ 10.69 (s, 1H), 9.15 (dd, J=10.7, 8.4 Hz, 1H), 9.06 (dd, J=10.7, 8.3 Hz, 1H), 7.53-7.24 (m, 15H), 6.98 (dd, J=10.1, 6.6 Hz, 1H), 6.83 (m, 1H), 6.78 (m, 2H), 6.18 (d, J=7.0 Hz, 2H), 5.68 (d, J=8.9 Hz, 1H), 4.95-4.68 (m, 5H), 4.19-3.75 (m, 7H), 3.30 (d, J=10.5 Hz, 1H), 2.58 (m, 1H), 2.35 (m, 1H), 1.26 (s, 9H).

### Example 100

### 2,3,9,10-Tetrafluoro-12-(4-deoxy-β-D-glucopyranosyl)indolo[2,3-a]pyrrolo[3,4-c]carbazole-5,7-dione

IR (KBr) 3440, 1745, 1710, 1474 cm⁻¹.
¹H NMR (DMSO-d₆, 400 MHz) δ 11.89 (s, 1H), 11.31 (s, 1H), 8.99 (dd, J=9.8, 8.9 Hz, 1H), 8.92 (dd, J=10.3, 9.0 Hz, 1H), 8.14 (dd, J=11.7, 6.5 Hz, 1H), 7.64 (dd, J=10.5, 6.9 Hz, 1H), 6.21 (t, J=4.5 Hz, 1H), 6.18 (d, J=9.2 Hz, 1H), 5.07 (d, J=5.6 Hz, 1H), 4.96 (d, J=5.4 Hz, 1H), 4.28 (d, J=12.1 Hz, 1H), 3.90-3.75 (m, 3H), 2.27 (m, 1H), 2.01 (m, 1H).
HPLC: 96.7% (320 nm).

### Example 101

### 2,3,9,10-Tetrafluoro-12-(2,3,4-tri-O-benzyl-β-D-glucopyranosyl)indolo[2,3-a]-pyrrolo[3,4-c]carbazole-5,7-dione

A solution of 2,3,9,10-tetrafluoro-12-(2,3,4-tri-O-benzyl-6-O-(4-methoxybenzyl)-β-D-glucopyranosyl)indolo[2,3-a]pyrrolo[3,4-clcarbazole-5,7-dione (0.410 g, 0.43 mmol) in 10 mL of 10% TFA-CH₂Cl₂ was stirred at room temperature under Ar for 15 min. The resulting mixture was diluted with ethyl acetate (50 mL) and then it was washed (1M, NaHCO₃, 2 x 50 mL; H₂O, 2 x 50 mL; brine, 50 mL) dried (MgSO₄) and evaporated. The resulting residue was chromatographed (SiO₂/2-26% ethyl acetate-hexane) to afford the title compound (0.346 g, 97%) as a yellow solid:
IR (CH₂Cl₂) 3333, 1753, 1700, 1478, 1093 cm⁻¹;
¹H NMR (DMSO-d₆, 400 MHz) δ 11.76 (s, 1H), 11.33 (s, 1H), 8.99 (dd, J=10.8, 8.5 Hz, 1H), 8.93 (dd, J=10.9, 8.5 Hz, 1H), 8.14 (dd, J=11.8, 6.8 Hz, 1H), 7.66 (dd, J=10.7, 6.9 Hz, 1H), 7.40 (m, 5H), 7.26 (m, 5H), 6.98 (t, J=7.4 Hz, 1H), 6.83 (t, J=7.6 Hz, 2H), 6.55 (d, J=8.9 Hz, 1H), 6.44 (m, 1H), 6.10 (d, J=7.6 Hz, 2H), 4.96 (d, J=11.1 Hz, 1H), 4.92 (d, J=11.0 Hz, 1H), 4.82 (m, 2H), 4.23 (t, J=9.4 Hz, 1H), 4.20-4.06 (m, 3H), 3.98-3.95 (m, 2H), 3.66 (t, J=9.0 Hz, 1H), 2.94 (d, J=10.6 Hz, 1H);
HPLC: 98.9% (320 nm).
Anal. Calcd for C₄₇H₃₅F₄N₃O₇: C, 68.03; H, 4.25; N, 5.07. Found: C, 68.00; H, 4.72; N, 4.79.

### Example 102

### 2,3,9,10-Tetrafluoro-12-((2,3,4-tri-O-benzyl-β-D-glucopyranosid)uronate)indolo-[2,3-a]pyrrolo[3,4-c]carbazole-5,7-dione

To a solution of 2,3,9,10-tetrafluoro-12-(2,3,4-tri-O-benzyl-β-D-glucopyranosyl)-indolo[2,3-a]pyrrolo[3,4-c]carbazole-5,7-dione (0.125 g, 0.15 mmol) in 12 mL of dry DMF was added pyridinium dichromate (PDC) (0.282 g, 0.75 mmol) and the mixture was stirred at room temperature for 4 h. Another 0.282 g (0.75 mmol) of PDC was then added and stirring was continued for 16 h. An additional portion of PDC (0.282 g, 0.75 mmol) was added and this was repeated after 24 h. A total of 1.128 g (3.0 mmol) of PDC was added and the reaction was run for 4 d. The resulting mixture was cooled at 5°C, treated with 10 mL of saturated NaHSO₃ and then diluted with water (25 mL). This mixture was extracted with ethyl acetate-THF (1:1,4 x 25 mL) and the combined extract was washed (2 x 25 mL sat. NaHSO₃, 25 mL brine), dried (MgSO₄) and evaporated. The resulting residue was chromatographed (SiO₂/ 0-20% MeOH-CH₂Cl₂) to give the title compound (0.069 g, 55%) as a yellow solid:
IR (KBr) 3412, 3260, 1746, 1710, 1597, 1477, 1320 cm⁻¹;
¹H NMR (DMSO-d₆, 400 MHz) δ 11.21(s, 1H), 9.02 (dd, J=10.8, 8.8 Hz, 1H), 8.57 (brs, 1H), 8.00 (m, 1H), 7.85 (m, 1H), 7.45 (d, J=7.0 Hz, 2H), 7.33 (m, 3H), 7.19 (s, 5H), 7.00 (t, J=7.4 Hz, 1H), 6.86 (t, J=7.5 Hz, 2H), 6.38 (d, J=7.2 Hz, 1H), 6.07 (d, J=7.3 Hz, 2H), 4.93 (d, J=10.8 Hz, 1H), 4.77 (d, J=11.2 Hz, 1H), 4.73 (d, J=10.8 Hz, 1H), 4.64 (d, J=11.2 Hz, 1H), 4.29 (d, J=8.8 Hz, 1H), 4.04 (m, 1H), 3.89 (m, 2H), 3.35 (m, 1H), 2.82 (m, 1H).

### Example 103

### 2,3,9,10-Tetrafluoro-12-[(β-D-glucopyranosid)uronic acid]indolo[2,3-a]pyrrolo-[3,4-c]carbazole-5,7-dione

A mixture of 2,3,9,10-tetrafluoro-12-[(2,3,4-tri-O-benzyl-β-D-glucopyranosid)-uronate]indolo[2,3-a]pyrrolo[3,4-c]carbazole-5,7-dione (0.030 g, 0.035 mmol) and 20% palladium hydroxide on charcoal (0.030 g) in a mixture of methanol (5 mL) and tetrahydrofuran (5 mL) was hydrogenated at 1 atm for 20 h. Another 30 mg of 20% palladium hydroxide on charcoal was added and the hydrogenation was continued for another 24 h. The resulting mixture was filtered, the filtercake washed with THE-MeOH-H₂O (10:10:1, 4x5 mL) and the filtrate evaporated to give a solid residue. Flash chromatography (SiO₂/2-20% MeOH-THF and then 20% MeOH-THF containing 1-4%H₂O) afforded the title compound (0.006 g, 30%) as a yellow solid:
IR (KBr) 3425, 3260, 1740, 1707, 1600, 1475, 1322 cm⁻¹;
¹H NMR (DMSO-d₆, 400 MHz) δ 13.11 (br s, 1H), 10.96 (br s, 1H), 9.02-7.51 (m, 5H), 6.21 (d, J=8.9 Hz, 1H), 5.4-4.0 (m, 8H).

### Example 104

### 3-Carboxy-9-fluoro-12-(β-D-glucopyranosyl)benzofurano[2,3-a]pyrrolo[3,4-c]-carbazole-5,7-dione

3-Cyano-9-fluoro-12-(2,3,4,6-tetra-O-benzyl-β-D-glucopyranosyl)benzofurano [2,3-a]pyrrolo[3,4-c]-carbazole-5,7-dione (380 mg, 0.43 mmol) in EtOH:THF (10 mL:5 mL) was added a 4.0 M solution of NaOH (10 mL). The mixture was gently refluxed for 24h. This resulting mixture was cooled to 0 °C and treated with concentrated HCl (15 mL). This solution was stirred at ambient temperature for 24h before it was taken up in EtOAc:THF and washed with water and brine prior to drying and solvent concentration. To this residue was added solid ammonium acetate (5.0g) and the mixture was fused at 150 °C for 1h before it was cooled, diluted EtOAc:THF, washed with water, brine, dried (Na₂SO₄) and concentrated. A portion of this crude product (60 mg) was hydrogenated (Pd/C) as previously described to give the title compound (18 mg, 50%) as a yellow solid: IR (KBr) 3420, 1756, 1710, 1561, 1395 cm⁻¹: ¹H NMR (DMSO-d₆ and D₂O exchange, 400 MHz) δ 9.26 (brs, 1H), 8.83-8.76 (m, 1H), 8.22 (brs, 1H), 7.99-7.96 (m, 1H), 7.83-7.68 (m, 1H), 7.55-7.45 (m, 1H), 6.51 (d, 0.6H, J=8.9 Hz), 5.99 (d, 0.4H, J=8.9 Hz), 4.31-3.43 (m, 6H): HPLC: 94.0% (320 nm).

### Example 105

### 3,9-Difluoro-6-[(2-guanidino)-ethyl]-12,13-dihydro-13-(b-D-glucopyranosyl)-5(H)indolo[2,3-a] pyrrolo[3,4-c] carbazole-5,7-dione.

To a magnetically stirred solution of the pure product from Example 10 (3.35 g, 3.25 mmol) in absolute ethanol (600 mL) was added aqueous potassium hydroxide (4.45 M, 60.0 ml, 267 mmol). The resulting deep red solution was heated in an open flask until about one-half of the ethanol was evaporated (about 3 h). The mixture was cooled under a stream of nitrogen and concentrated HCl (12 N; 175 mL) was added. The mixture was stirred 15 min and then partitioned with water (300 mL) and ethyl acetate (800 ml). The organic layer was washed with water (300 mL), saturated aqueous sodium bicarbonate (300 mL), and brine (300 mL), dried (Na₂SO₄), and evaporated in vacuo. The resulting solid anhydride was treated with ethylene diamine (50 mL) and heated to boiling for about 4-5 h h, dissolved in absolute ethanol (175 mL), refluxed for about 6 h, and then concentrated in vacuo. Purification by flash chromatography on silica gel with 2-10% methanol in methylene chloride gave 1.72 g (55%) of the pure 6N-(2-amino)-ethyl) derivative of the starting compound as a yellow-orange solid: 300 MHz ¹H NMR (CDCl₃) d 10.65 (brs, 1 H), 9.05 (dd, 1H), 8.94 (dd, 1 H), 7.55 (dd, 1 H), 7.45-7.14 (m, 18 H), 7.00 (t, 1 H), 6,87 (t, 2 H), 6.14 (d, 2 H), 5.95 (d, 1 H), 5.05-4.58 (m, 6 H), 4.40-4.34 (m, 1 H), 4.12-3.85 (m, 8 H), 3.22-3.15 (m, 2 H), 2.96 (d, 1 H): Pos ESI mass spectrum, m/e 927 (M+H)⁺.

To a magnetically stirred solution of the pure product from above (1.72g, 1.86 mmol.) in anhydrous THF (20 mL) was added N,N-diisopropylethylamine (314 mL), and N,N'-bis(benzyloxycarbonyl)-s-methylisothiourea (800 mg, 2.23 mmol: K. Nowak, L. Kania Rocz. Chem. 1969, 43, 1953) and the reaction was heated in a 70 °C oil bath for 24 h. The solution is cooled to room temperature, evaporated in vacuo, and purified by flash chromatography on silica gel using 5% ethyl acetate in methylene chloride to give 1.57 g (68%) of the pure di-CBz protected guanidine derivative of the starting amine as a yellow solid: 300 MHZ ¹H NMR (CDCl3) d 11.80 (brs, 1 H), 10.70 (brs, 1 H), 9.18 (brs, 1 H), 9.08 (dd, 1H), 8.92 (dd, 1 H), 7.62 -7.10 (m, 24 H), 6.96 (t, 1 H), 6.85 (t, 2 H), 6.13 (d, 2 H), 5.95 (d, 1H), 5.20-4.60 (m, 10H), 4.36 (t, 1H), 4.20-3.85 (m, 10H), 3.02 (d, 1H).

To a solution of the product from above (1.58 g, 1.38 mmol.) in 3:1 methanol/ethyl acetate (120 mL) and 1N HCL (18 mL) under N₂ was added 20% palladium(II) hydroxide on carbon (722 mg). The mixture was placed on a parr shaker under 65 psi hydrogen pressure for 3 days and then filtered through a small pad of celite. The celite was washed with methanol (3 x 50 mL) and concentrated in vacuo to give a orange solid. Purification on Sephadex LH-20 with methanol elution gave 754 mg (91%) of the pure title compound as a red-orange solid: 500 MHZ ¹H NMR (CD₃OD) d 8.83 (dd, 1H, J=9.7, 2.8 Hz), 8.73 (dd, 1H, J=8.9, 2.5 Hz), 7.77 (dd, 1H, J=9.2, 4.1 Hz), 7.63 (dd, 1H, J=8.9, 4.4 Hz), 7.34-7.25 (m, 2H), 6.15 (d, 1H, J=8.5 Hz), 4.32-4.17 (m, 2H), 4.11-3.97 (m, 2H), 3.91-3.84 (m, 2H), 3.78-3.66 (m, 2H), 3.60-3.53 (m, 2H). FAB mass spectrum, m/e 609 (M+H)⁺.

### Example 106

### 2,3,9,10-Tetrafluoro-12-((6-deoxy-6,6-difluoro-b-D-glucopyranosyl)indolo[2,3-a]-pyrrolo[3,4-c]carbazole-5,7-dione (R₅, R_{5'}= F, R₂-R₄= OH, X₁, X_{1'}, X₂, X_{2'}= F, Q= NH)

A solution of 2,3,9,10-tetrafluoro-12-(2,3,4-tri-O-benzyl-b-D-glucopyranosyl)indolo [2,3,-a]pyrrolo[3,4-c]carbazole-5,7-dione (0.166 g, 0.20 mmol) in 5 mL of dichloromethane was added to a cold (5°C) solution of Dess-Martin periodinane (0.106 g, 0.25 mmol) in 5 mL of dichloromethane. The resulting mixture was stirred at room temperature under Ar for 2 h. The reaction mixture was subsequently diluted with ethyl acetate (25 mL), washed (1M NaHCO₃-30% Na₂S₂O₃, 2x10 mL; 1M NaHCO₃, 2x10 mL; H₂O, 10 mL; brine, 10 mL), dried (MgSO₄) and evaporated. The resulting residue was chromatographed (SiO₂/2-30% ethyl acetate-hexane) to give 2,3,9,10-tetrafluoro-12-((2,3,4-tri-O-benzyl-b-D-glucopyranosid)uronaldehyde)indolo[2,3-a]pyrrolo[3,4-c]-carbazole-5,7-dione (0.118 g, 71%) as a pale yellow solid:
IR (CH₂Cl₂), 3344, 2930, 1753, 1724, 1597, 1479,1322 cm⁻¹;
¹H NMR (CDCl₃, 400 MHz) d 11.08 (s, 0.3H), 10.68 (s, 1H), 3.85 (s, 0.3H), 8.97 (m, 0.3H), 8.79 (m, 0.3H), 8.43 (m, 1H), 7.99 (br s, 0.7H), 7.83 (m, 1H), 7.68 (m, 0.3H), 7.53-6.73 (m, 14H), 6.27 (m, 1H), 6.05 (d, J=7.2 Hz, 2H), 5.80 (d, J=9.0 Hz, 1H), 5.61 (s, 2H), 5.09 (d, J=11.1 Hz, 1H), 4.91 (m, 3H), 4.29-3.85 (m, 4H), 3.74 (t, J=8.8 Hz, 1H), 2.92 (d, J=10.3 Hz, 1H).
To a cold (5°C) solution of the aldehyde (0.053 g, 0.085 mmol) in 5 mL of dichloromethane under Ar was added DAST (0.022 mL, 0.17 mmol) dropwise. The resulting mixture was stirred at room temperature for 18 h. The reaction mixture was then diluted with ethyl acetate (20 mL), washed (1M, NaHCO₃, 2 x 10 mL; H₂O, 2 x 10 mL; brine, 10 mL), dried (MgSO₄) and evaporated. The residue was chromatographed (SiO₂/2-24% ethyl acetate-hexane) to give 2,3,9,10-tetrafluoro-12-(2,3,4-tri-O-benzyl-6-deoxy-6,6-difluoro-b-D-glucopyranosyl)indolo[2,3-a]pyrrolo[3,4-c]carbazole-5,7-dione (0.046 g, 85%) as a pale yellow solid:
IR (CH₂Cl₂), 3400, 1757, 1728, 1596, 1478, 1320 cm⁻¹;
¹H NMR (CDCl₃, 400 MHz ) d 9.98 (s, 1H), 8.89 (m, 2H), 7.71 (m, 1H), 7.51-7.26 (m, 11H), 7.09 (m, 1H), 7.00 (t, J=7.4 Hz, 1H), 6.85 (t, J=7.5 Hz, 2H), 6.24 (t, J=53.7 Hz, 1H), 6.13 (d, J=7.2 Hz, 2H), 5.80 (d, J=9.0 Hz, 1H), 5.04 (d, J=11.2 Hz, 1H), 4.89 (s, 2H), 4.84 (d, J=11.2 Hz, 1H), 4.23 (m, 1H), 4.3 (m, 2H), 3.95 (d, J=10.3 Hz, 1H), 3-81 (m, 1H), 3.04 (m, 1H);
MS (ESI) m/e 848 (M-H).

A mixture of the tri-O-benzylglucopyranoside (0.035 g, 0.041 mmol) and 20% Pd(OH)₂-C in a mixutre of methanol (5 mL) and chloroform (2 mL) was hydrogenated at 1 atm pressure for 16 h. The resulting mixture was filtered and the filtercake was washed with tetrahydrofuran-methanol (1:1). The filtrate was evaporated and the residue chromatographed (SiO₂/2-12% MeOH-CH₂Cl₂) to give the title compound (0.019 g, 80%) as a yellow solid:
IR (KBr), 3385, 1748, 1713, 1595, 1476, 1326 cm⁻¹;
¹H NMR (THF-d₈, 400 MHz) d 10.55 (s, 1H), 10-18 (s-1H), 9.14 (dd, J=10.6, 8.8 Hz, 1H), 9.04 (dd, J=10.6, 8.6 Hz, 1H), 7.79 (dd, J=11.5, 6.6 Hz, 1H), 7.44 (dd, J=9.8, 7.1 Hz, 1H), 6.56 (t, J=53.7 Hz, 1H), 6.18 (d, J=7.9 Hz, 1H), 5.52 (brs, 1H), 5-10 (brs, 1H), 4.71 (br s*,* 1H), 4.27 (m, 1H), 3.95 (m, 1H), 3.69 (m, 2H). HPLC: 95.2% (230 nm).

## Claims

1. A compound of formula I, or a pharmaceutically acceptable salt and/or solvate thereof 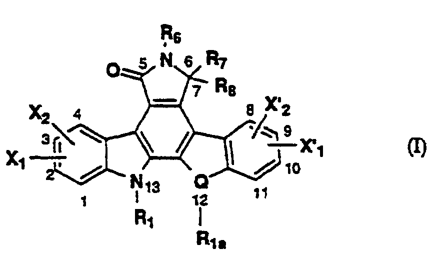 wherein:
R₁ and R₁ₐ are independently hydrogen, a pentose group (A) or a hexose group (B) of the formulas 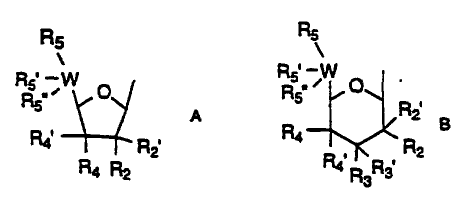 provided that one of R₁ and R₁ₐ is hydrogen and the other is not hydrogen;
R₂, R₃, R₄, R₅ and R_{2'}, P_{3'}, R_{4'}, R_{5"} and R_{5'} are independently hydrogen, C₁₋₇ alkyl, C₁₋₇ cydoalkyl, O, azido, halogen, NR₉R₁₀, NHC(O)NR₉R₁₀, NHC(O)OR, OR, -C(O)Rₐ, SR, -OSO₂R_{c}, or together form =N-OH,, =O, =NR, provided R₂, R₃, R₄, R₅ and R_{2'}, R_{3'}, R_{4'}, R_{5"}_and R_{5'} are not simultaneously all hydrogen, OH, alkoxy or alkyl, and further provided that R₃ or R3' is not -NH₂, except when R₆ is -(CH₂)ₙNHC(=NH)NH₂, said C₁₋₇ alkyl optionally substituted with one to six of the same or different halogen, CN, NO₂, aryl or heteroaryl, said aryl or heteroaryl substituted with one or two groups independently selected from NR₉R₁₀, OH, COOR₉, SO₃R₉ or OCOR₉;
Rₐ is H, OH, C₁₋₇ alkoxy or NR₉R₁₀;
R_{c} is C₁₋₇ alkyl or aryl;
R and R₁₁ are independently hydrogen, C₁₋₇ alkyl, C₁₋₇ cycloalkyl, heteroaryl, non-aromatic cyclic 5-8 membered ring containing either one or two heteroatoms selected from O or N, (CH2)n NR₉R₁₀, (CH₂)ₙOR₉ or (CH₂)ₙCOOR₉, said C₁₋₇ alkyl optionally substituted with one to six of the same or different halogen, OH, CN, NO₂, aryl or heteroaryl, said aryl or heteroaryl substituted with one or two groups independently selected from NR₉R₁₀, OH, COOR₉, SO₃R₉ or OCOR₉;
R₉ and R₁₀ are independently hydrogen, C₁₋₇ alkyl, C₁₋₇ cycloalkyl, benzyl, aryl, heteroaryl, any of which groups except hydrogen can be substituted with one to six of the same or different halogen, OH, NH₂, CN, NO₂, -C(=NH)NH2, -CH(=NH), CH(R_{b}) (CH₂)ₙ COOH or CH(R_{b}) (CH₂)ₙ NH₂ or COOR₁₁, or R₉ and R₁₀ together with the nitrogen atom to which they are attached form a cyclic 5-8 membered non-aromatic ring containing either one or two heteroatoms selected from O, N, or S or R₉ and R₁₀ together form =CHRR₁₁.
R_{b} is H or COOH;
R₆ is hydrogen, C₁₋₇ alkyl, aryl, arylalkyl, OR₁₀, NR₉R₁₀, or OCO(CH₂)ₙNR₉R₁₀, said C₁₋₇ alkyl optionally substituted with one to six of the same or different halogen, NR₉R₁₀, CN, NO₂, aryl, said aryl substituted with one or two groups independently selected from NR₉R₁₀, OH, COOR₉, SO₃R₉ or OCOR₉;
R₇ and R₈ are independently OH or H, or taken together is O;
X₁, X'₁, X₂ and X'₂ are independently H, halogen, OH, -CN, -NC, CF₃, -CORₐ, NO₂, OR, O(CH₂)ₙNR₉R₁₀, O(CH₂)ₙOR₉ or O(CH₂)ₙCOOR₉; provided X₂, X'₂, X*₁ and X', are not 1, 11-dichloro; provided further when X₂ and X'₂ are each H, X, and X'₁ are each independently H or halogen, R, is hexose, R₇ and R₈ together is O, and each of R₂, R₅, and R₄ are OH, R_{2'}, R_{3'}, R_{4'}, and R_{5'} and R_{5"} are each H, Q is NH, and then each of R₃ and R₆ are not NH₂ and R₃ is not methoxy when R₆ is H;
W is C or N;
Q is O, NR₉, S or CH₂; and
n is an integer from 0-4.

2. A compound of claim 1, wherein in R₁ or R₁ₐ all substituents are H except R₂, R₃, and R₄ are each OH; and R₅ is NR₉R₁₀.

3. A compound of claim 1, wherein in R₁ or R₁ₐ all substituents are H except R₂, R₃ and R₅ are each OH; and R₄ is NR₉R₁₀.

4. A compound of claim 1, wherein in R₁ or R₁ₐ all substituents are H except R₂, R₃ and R₄ are each OH; and R₅ is halogen.

5. A compound of claim 1, wherein R₇ and R₈ taken together is O.

6. A compound of claim 1, wherein:
X₁, X'₁, X₂ and X'₂ are independently halogen.

7. A compound of claim 1, wherein said halogen group is fluoro.

8. A compound of claim 1, wherein Q is O, S or NH.

9. A compound of claim 1 wherein R₂, R₃, and R₅ are each OH; and R₄ is NR₉R₁₀, halogen or N₃.

10. A compound of claim 1 wherein in R₁ or R₁ₐ all substituents are H except R₅, R₃ and R₄ are each OH; and R₂ is halogen.

11. A compound of claim 1 wherein in R₁ or R₁ₐ all substituents are H except R₂, R₃ are each OH; R₅ is halogen; and R₄ is azido, NR₉R₁₀ or OR.

12. A compound of claim 1 wherein wherein in R₁ or R₁ₐ all substituents are H except R₂, R₃ are each OH; R₅ is halogen; and R₄ is halogen, H or alkyl.

13. A compound of claim 1 wherein in R₁ or R₁ₐ all substituents are H except R₂ and R₃ are each hydrogen or hydroxy; R₄ is hydrogen, halogen, C₁₋₇ alkyl or azido; and R₅ is hydroxy, azido, C₁₋₇ alkyl, halogen or NR₉R₁₀.

14. A compound of claim 1 wherein in R₁ or R₁ₐ all substituents are H except R₃ and R₄ are each hydrogen or hydroxy; R₂ is hydrogen, halogen, C₁₋₇ alkyl or azido; and R₅ is hydroxy, azido, C₁₋₇ alkyl, halogen or NR₉R₁₀.

15. A compound of claim 1 wherein in R₁ or R₁ₐ all substituents are H except R₃ and R₅ are each hydrogen or hydroxy; R₂ is hydrogen, halogen, C₁₋₇ alkyl or azido; and R₄ is hydroxy, azido, C₁₋₇ alkyl, halogen or NR₉R₁₀.

16. A compound of claim 1 wherein in R₁ or R₁ₐ all substituents are H except R₂ and R₄ are each hydrogen or hydroxy; R₃ is hydrogen, halogen, C₁₋₇ alkyl or azido; and R₅ is hydroxy, azido, C₁₋₇ alkyl, halogen or NR₉R₁₀.

17. A pharmaceutical formulation which comprises an antitumor effective amount of a compound of formula I or a pharmaceutically acceptable salt and/or solvate thereof, as claimed in any one of claims 1-16.

18. A tumor-growth inhibiting amount of a compound of formula I as claimed in any one of claims 1-16, or a pharmaceutically acceptable salt and/or solvate thereof, for inhibiting tumor growth in a mammalian host.

## Patentansprüche

1. Verbindung der Formel I oder ein pharmazeutisch verträgliches Salz und/oder Solvat davon 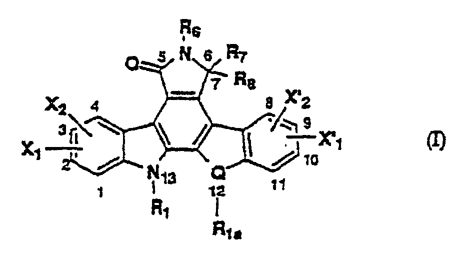 in der:
R₁ und R₁ₐ unabhängig voneinander ein Wasserstoffatom, einen Pentoserest (A) oder einen Hexoserest (B) der Formeln 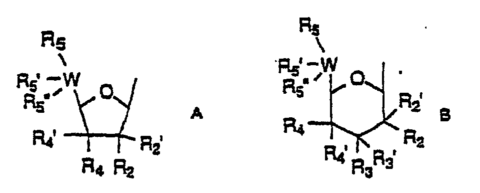 bedeuten, mit der Maßgabe, dass einer der Reste R₁ und R₁ₐ ein Wasserstoffatom darstellt und der andere kein Wasserstoffatom bedeutet;
R₂, R₃, R₄, R₅ und R_{2'}, R_{3'}, R_{4'}, R_{5"} und R_{5'} unabhängig voneinander ein Wasserstoffatom, einen C₁₋₇-Alkylrest, einen C₁₋₇-Cycloalkylrest, ein O-Atom, eine Azidogruppe, ein Halogenatom, einen Rest NR₉R₁₀, NHC(O)NR₉R₁₀, NHC(O)OR, OR, -C(O)Rₐ, SR oder -OSO₂R_{c} darstellen oder zusammen eine Gruppe =N-OH oder =O oder einen Rest =NR bilden, mit der Maßgabe, dass R₂, R₃, R₄, R₅ und R_{2'}, R_{3'}, R_{4'}, R_{5"} und R_{5'} nicht alle gleichzeitig ein Wasserstoffatom, eine OH-Gruppe, einen Alkoxy- oder Alkylrest bedeuten und mit der weiteren Maßgabe, dass R₃ oder R_{3'} keine NH₂-Gruppe darstellt, außer wenn R₆ einen Rest -(CH₂)ₙNHC(=NH)NH₂ bedeutet, wobei der C₁₋₇-Alkylrest gegebenenfalls mit ein bis sechs gleichen oder verschiedenen Resten aus einem Halogenatom, CN, NO₂, einem Aryl-oder Heteroarylrest substituiert ist, wobei der Aryl- oder Heteroarylrest mit einer oder zwei Gruppen, die unabhängig voneinander aus den Resten NR₉R₁₀, OH, COOR₉, SO₃R₉ oder OCOR₉ ausgewählt sind, substituiert ist;
Rₐ ein H-Atom, eine OH-Gruppe, einen C₁₋₇-Alkoxyrest oder einen Rest NR₉R₁₀ darstellt;
R_{c} einen C₁₋₇-Alkyl- oder Arylrest bedeutet;
R und R₁₁ unabhängig voneinander ein Wasserstoffatom, einen C₁₋₇-Alkylrest, einen C₁₋₇-Cycloalkylrest, einen Heteroarylrest, einen nicht-aromatischen cyclischen 5 - 8-gliedrigen Ring, der entweder ein oder zwei aus einem O- oder N-Atom ausgewählte Heteroatome enthält, einen Rest (CH₂)ₙNR₉R₁₀, (CH₂)ₙOR₉ oder (CH₂)ₙCOOR₉ darstellen, wobei der C₁₋₇-Alkylrest gegebenenfalls mit ein bis sechs gleichen oder verschiedenen Resten aus einem Halogenatom, OH, CN, NO₂, einem Aryl- oder Heteroarylrest substituiert ist, wobei der Aryl-oder Heteroarylrest mit einer oder zwei Gruppen, die unabhängig voneinander aus einem Rest NR₉R₁₀, OH, COOR₉, SO₃R₉ oder OCOR₉ ausgewählt sind, substituiert ist;
R₉ und R₁₀ unabhängig voneinander ein Wasserstoffatom, einen C₁₋₇-Alkylrest, einen C₁₋₇-Cycloalkylrest, eine Benzylgruppe, einen Aryl- oder Heteroarylrest bedeuten, wobei beliebige dieser Reste mit Ausnahme des Wasserstoffatoms mit ein bis sechs gleichen oder verschiedenen Resten aus einem Halogenatom, OH, NH₂, CN, NO₂, -C(=NH)NH₂, -CH(=NH), CH(R_{b})(CH₂)ₙCOOH oder CH(R_{b})(CH₂)ₙNH₂ oder COOR₁₁ substituiert sein können, oder R₉ und R₁₀ zusammen mit dem Stickstoffatom, an das sie gebunden sind, einen cyclischen 5 - 8-gliedrigen nicht-aromatischen Ring, der entweder ein oder zwei aus einem O-, N-oder S-Atom ausgewählte Heteroatome enthält, bilden oder R₉ und R₁₀ zusammen =CHRR₁₁ bilden;
R_{b} ein H-Atom oder eine COOH-Gruppe darstellt;
R₆ ein Wasserstoffatom, einen C₁₋₇-Alkylrest, einen Arylrest, einen Arylalkylrest, einen Rest OR₁₀, NR₉R₁₀ oder OCO(CH₂)ₙNR₉R₁₀, wobei der C₁₋₇-Alkylrest gegebenenfalls mit ein bis sechs gleichen oder verschiedenen Resten aus einem Halogenatom, NR₉R₁₀, CN, NO₂ und einem Arylrest substituiert ist, wobei der Arylrest mit einer oder zwei Gruppen substituiert ist, die unabhängig voneinander aus einem Rest NR₉R₁₀, OH, COOR₉, SO₃R₉ oder OCOR₉ ausgewählt sind;
R₇ und R₈ unabhängig voneinander eine OH-Gruppe oder ein H-Atom bedeuten oder zusammengenommen ein O-Atom darstellen;
X₁, X'₁, X₂ und X'₂ unabhängig voneinander ein H-Atom, ein Halogenatom, einen Rest OH, -CN, -NC, CF₃, -CORₐ, NO₂, OR, O(CH₂)ₙNR₉R₁₀, O(CH₂)ₙOR₉ oder O(CH₂)ₙCOOR₉ bedeuten; mit der Maßgabe, dass X₂, X'₂, X₁ und X'₁ keine 1,11-Dichlorgruppe darstellen; mit der weiteren Maßgabe, dass, wenn X₂ und X'₂ jeweils ein H-Atom bedeuten, X₁ und X'₁ jeweils unabhängig voneinander ein H- oder Halogenatom darstellen, R₁ eine Hexose bedeutet, R₇ und R₈ zusammen ein O-Atom darstellen und jeder der Reste R₂, R₅ und R₄ eine OH-Gruppe bedeutet, R_{2'}, R_{3'}, R_{4'} und R_{5'} und R_{5"} jeweils ein H-Atom darstellen, Q eine NH-Gruppe bedeutet und dann jeder der Reste R₃ und R₆ keine NH₂-Gruppe darstellt und R₃ keine Methoxygruppe bedeutet, wenn R₆ ein H-Atom darstellt;
W ein C- oder N-Atom bedeutet;
Q ein O-Atom, einen Rest NR₉, ein S-Atom oder eine CH₂-Gruppe darstellt und
n eine ganze Zahl von 0 - 4 ist.

2. Verbindung nach Anspruch 1, wobei in R₁ oder R₁ₐ alle Substituenten H-Atome bedeuten, jedoch R₂, R₃ und R₄ jeweils eine OH-Gruppe darstellen und R₅ den Rest NR₉R₁₀ bedeutet.

3. Verbindung nach Anspruch 1, wobei in R₁ oder R₁ₐ alle Substituenten H-Atome bedeuten, jedoch R₂, R₃ und R₅ jeweils eine OH-Gruppe darstellen und R₄ den Rest NR₉R₁₀ bedeutet.

4. Verbindung nach Anspruch 1, wobei in R₁ oder R₁ₐ alle Substituenten H-Atome bedeuten, jedoch R₂, R₃ und R₄ jeweils eine OH-Gruppe darstellen und R₅ ein Halogenatom bedeutet.

5. Verbindung nach Anspruch 1, in der R₇ und R₈ zusammengenommen ein O-Atom darstellen.

6. Verbindung nach Anspruch 1, in der X₁, X'₁, X₂ und X'₂ unabhängig voneinander ein Halogenatom bedeuten.

7. Verbindung nach Anspruch 1, wobei der Halogenrest ein Fluoratom ist.

8. Verbindung nach Anspruch 1, wobei Q ein O- oder S-Atom oder eine NH-Gruppe darstellt.

9. Verbindung nach Anspruch 1, wobei R₂, R₃ und R₅ jeweils eine OH-Gruppe bedeuten und R₄ einen Rest NR₉R₁₀, ein Halogenatom oder eine N₃-Gruppe darstellt.

10. Verbindung nach Anspruch 1, wobei in R₁ oder R₁ₐ alle Substituenten H-Atome bedeuten, jedoch R₅, R₃ und R₄ jeweils eine OH-Gruppe darstellen und R₂ ein Halogenatom bedeutet.

11. Verbindung nach Anspruch 1, wobei in R₁ oder R₁ₐ alle Substituenten H-Atome bedeuten, jedoch R₂ und R₃ jeweils eine OH-Gruppe darstellen, R₅ ein Halogenatom bedeutet und R₄ eine Azidogruppe, einen Rest NR₉R₁₀ oder OR darstellt.

12. Verbindung nach Anspruch 1, wobei in R₁ oder R₁ₐ alle Substituenten H-Atome bedeuten, jedoch R₂ und R₃ jeweils eine OH-Gruppe darstellen, R₅ ein Halogenatom bedeutet und R₄ ein Halogenatom, ein H-Atom oder einen Alkylrest darstellt.

13. Verbindung nach Anspruch 1, wobei in R₁ oder R₁ₐ alle Substituenten H-Atome bedeuten, jedoch R₂ und R₃ jeweils ein Wasserstoffatom oder eine Hydroxygruppe darstellen, R₄ ein Wasserstoffatom, ein Halogenatom, einen C₁₋₇-Alkykest oder eine Azidogruppe bedeutet und R₅ eine Hydroxygruppe, eine Azidogruppe, einen C₁₋₇-Alkylrest, ein Halogenatom oder einen Rest NR₉R₁₀ darstellt.

14. Verbindung nach Anspruch 1, wobei in R₁ oder R₁ₐ alle Substituenten H-Atome bedeuten, jedoch R₃ und R₄ jeweils ein Wasserstoffatom oder eine Hydroxygruppe darstellen, R₂ ein Wasserstoffatom, ein Halogenatom, einen C₁₋₇-Alkylrest oder eine Azidogruppe bedeutet und R₅ eine Hydroxygruppe, eine Azidogruppe, einen C₁₋₇-Alkylrest, ein Halogenatom oder einen Rest NR₉R₁₀ darstellt.

15. Verbindung nach Anspruch 1, wobei in R₁ oder R₁ₐ alle Substituenten H-Atome bedeuten, jedoch R₃ und R₅ jeweils ein Wasserstoffatom oder eine Hydroxygruppe darstellen, R₂ ein Wasserstoffatom, ein Halogenatom, einen C₁₋₇-Alkylrest oder eine Azidogruppe bedeutet und R₄ eine Hydroxygruppe, eine Azidogruppe, einen C₁₋₇-Alkylrest, ein Halogenatom oder einen Rest NR₉R₁₀ darstellt.

16. Verbindung nach Anspruch 1, wobei in R₁ oder R₁ₐ alle Substituenten H-Atome bedeuten, jedoch R₂ und R₄ jeweils ein Wasserstoffatom oder eine Hydroxygruppe darstellen, R₃ ein Wasserstoffatom, ein Halogenatom, einen C₁₋₇-Alkylrest oder eine Azidogruppe bedeutet und R₅ eine Hydroxygruppe, eine Azidogruppe, einen C₁₋₇-Alkylrest, ein Halogenatom oder einen Rest NR₉R₁₀ darstellt.

17. Pharmazeutische Zubereitung, die eine antitumorwirksame Menge einer Verbindung der Formel I oder eines pharmazeutisch verträglichen Salzes und/oder Solvates davon, wie in einem der Ansprüche 1 - 16 beansprucht, umfasst.

18. Tumorwachstumshemmende Menge einer Verbindung der Formel I, wie in einem der Ansprüche 1-16 beansprucht, oder eines pharmazeutisch verträglichen Salzes und/oder Solvates davon zum Hemmen von Tumorwachstum bei einem Säuger.

## Revendications

1. Composés de la formule I, ou son sel et/ou solvate pharmaceutiquement acceptable 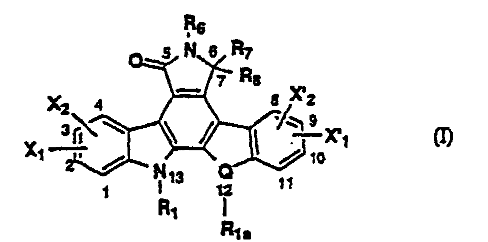 où:
R₁ et R₁ₐ sont indépendamment hydrogène, un groupe pentose (A) ou un groupe hexose (B) des formules 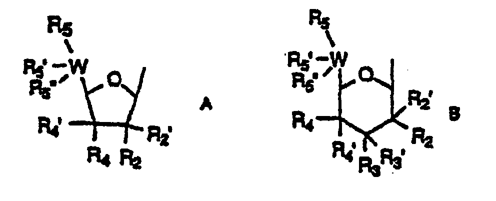 à condition que l'un de R₁ et R₁ₐ soit hydrogène et que l'autre ne soit pas hydrogène;
R₂, R₃, R₄, R₅ et R_{2'}, R_{3'}, R_{4'}, R_{5"} et R₅, sont indépendamment hydrogène, alkyle C₁₋₇, cycloalkyle C₁₋₇, O, azido, halogène, NR₉R₁₀, NHC(O)NR₉R₁₀, NHC(O)OR, OR, -C(O)Ra, SR, -OSO₂R_{c}, ou forment ensemble =N-OH, =O, =NR, à condition que R₂, R₃, R₄, R₅ et R_{2'}, R_{3'}, R_{4'}, R_{5"}, et R_{5'} ne soient pas simultanément tous de l'hydrogène, OH, alcoxy ou alkyle, et à condition de plus que R₃ ou R_{3'} ne soit pas -NH₂, sauf quand R₆ est -(CH₂)ₙNHC(=NH)NH₂, ledit alkyle C₁₋₇ facultativement substitué par un à six du même ou différent halogène, CN, NO₂, aryle ou hétéroaryle, ledit aryle ou hétéroaryle substitué par un deux groupes indépendamment sélectionnés parmi NR₉R₁₀, OH, COOR₉, SO₃R₉ ou OCOR₉;
Rₐ est H, OH, alcoxy C₁₋₇ ou NR₉R₁₀;
R_{c} est alkyle C₁₋₇ ou aryle;
R et R₁₁ sont indépendamment hydrogène, alkyle C₁₋₇, cycloalkyle C₁₋₇, hétéroaryle, cycle de 5-8 membres cyclique non aromatique contenant soit un ou deux hétéroatomes sélectionnés parmi O ou N, (CH₂)ₙ NR₉R₁₀, (CH₂)ₙOR₉ ou (CH₂)ₙCOOR₉, ledit alkyle C₁₋₇ facultativement substitué par un à six du même ou différent halogène, OH, CN, NO₂, aryle ou hétéroaryle, ledit aryle ou hétéroaryle substitué par un ou deux groupes indépendamment sélectionnés parmi NR₉R₁₀, OH, COOR₉, SO₃R₉ ou OCOR₉;
R₉ et R₁₀ sont indépendamment hydrogène, alkyle C₁₋₇, cycloalkyle C₁₋₇, benzyle, aryle, hétéroaryle, chacun de ces groupes à l'exception de l'hydrogène peut être substitué par un à six du même ou différent halogène, OH, NH₂, CN, NO₂, -C(=NH)NH₂, -CH(=NH), CH(R_{b})(CH₂)ₙ COOH ou CH(R_{b}) (CH₂) NH₂ ou COOR₁₁, ou bien R₉ et R₁₀ avec l'atome d'azote auquel ils sont attachés forment un cycle non aromatique de 5-8 membres cyclique contenant soit un ou deux hétéroatomes sélectionnés parmi O, N, ou S ou bien R₉ et R₁₀ forment ensemble =CHRR₁₁.
R_{b} est H ou COOH;
R₆ est hydrogène, alkyle C₁₋₇, aryle, arylalkyle, OR₁₀, NR₉R₁₀, ou OCO(CH₂)ₙNR₉R₁₀, ledit alkyle C₁₋₇ facultativement substitué par un à six même ou différent halogène, NR₉R₁₀, CN, NO₂, aryle, ledit aryle substitué par un ou deux groupes indépendamment sélectionnés parmi NR₉R₁₀, OH, COOR₉, SO₃R₉ ou OCOR₉;
R₇ et R₈ sont indépendamment OH ou H, ou bien pris ensemble est O;
X₁, X'₁, X₂ et X'₂ sont indépendamment H, halogène, OH, -CN, -NC, CF₃, -CORₐ, NO₂, OR, O(CH₂)ₙNR₉R₁₀, O(CH₂)ₙOR₉ ou O(CH₂)ₙCOOR₉ ; à condition que X₂, X'₂, X₁ et X'₁, ne soient pas 1, 11-dichloro; à condition de plus que quand X₂ et X'₂ sont chacun H, X₁ et X'₁ soient chacun indépendamment H ou halogène, R₁ est hexose, R₇ et R₈ ensemble est O, et chacun de R₂, R₅, et R₄ sont OH, R₂', R₃', R₄', et R₅' et R₅" sont chacun H, Q est NH, et chacun de R₃ et R₆ ne sont pas NH₂ et R₃ n'est pas méthoxy quand R₆ est H;
W est C ou N;
Q est O, NR₉, *S* ou CH₂; et
n est un entier de 0-4.

2. Composé de la revendication 1, où dans R₁ ou R₁ₐ, tous les substituants sont H à l'exception de R₂, R₃ et R₄ sont chacun OH; et R₅ est NR₉R₁₀.

3. Composé de la revendication 1, où dans R₁ ou R₁ₐ, tous les substituants sont H à l'exception de R₂, R₃ et R₅ sont chacun OH; et R₄ est NR₉R₁₀.

4. Composé de la revendication 1, où dans R₁ ou R₁ₐ, tous les substituants sont H à l'exception de R₂, R₃ et R₄ sont chacun OH; et R₅ est halogène.

5. Composé de la revendication 1, où R₇ et R₈ pris ensemble est O.

6. Composé de la revendication 1, où:
X₁, X'₁, X₂ et X'₂ sont indépendamment halogène.

7. Composé de la revendication 1, où ledit groupe halogène est fluoro.

8. Composé de la revendication 1, où Q est O, S ou NH.

9. Composé de la revendication 1 où R₂, R₃, et R₅ sont chacun OH; et R₄ est NR₉R₁₀, halogène ou N₃.

10. Composé de la revendication 1 où dans R₁ ou R₁ₐ, tous les substituants sont H à l'exception de R₅, R₃ et R₄ sont chacun OH; et R₂ est halogène.

11. Composé de la revendication 1 où dans R₁ ou R₁ₐ tous les substituants sont H à l'exception de R₂, R₃ sont chacun OH; R₅ est halogène et R₄ est azido, NR₉R₁₀ ou OR.

12. Composé de la revendication 1 où où dans R₁ ou R₁ₐ, tous les substituants sont H, à l'exception de R₂, R₃ sont chacun OH; R₅ est halogène; et R₄ est halogène, H ou alkyle.

13. Composé de la revendication 1 où dans R₁ ou R₁ₐ, tous les substituants sont H à l'exception de R₂ et R₃ sont chacun hydrogène ou hydroxy; R₄ est hydrogène, halogène, alkyle C₁₋₇ ou azido; et R₅ est hydroxy, azido, alkyle C₁₋₇, halogène ou NR₉R₁₀.

14. Composé de la revendication 1 où dans R₁ ou R₁ₐ, tous les substituants sont H à l'exception de R₃ et R₄ sont chacun hydrogène ou hydroxy; R₂ est hydrogène, halogène, alkyle C₁₋₇ ou azido; et R₅ est hydroxy, azido, alkyle C₁₋₇, halogène ou NR₉R₁₀.

15. Composé de la revendication 1 où dans R₁ ou R₁ₐ, tous les substituants sont H à l'exception de R₃ et R₅ sont chacun hydrogène ou hydroxy; R₂ est hydrogène, halogène, alkyle C₁₋₇ ou azido; et R₄ est hydroxy, azido, alkyle C₁₋₇, halogène ou NR₉R₁₀.

16. Composé de la revendication 1 où dans R₁ ou R₁ₐ, tous les substituants sont H à l'exception de R₂ et R₄ sont chacun hydrogène ou hydroxy; R₃ est hydrogène, halogène, alkyle C₁₋₇, ou azido; et R₅ est hydroxy, azido, alkyle C₁₋₇, halogène ou NR₉R₁₀.

17. Formulation pharmaceutique qui comprend une quantité efficace antitumeur d'un composé de formule I ou son sel et/ou solvate pharmaceutiquement acceptable selon l'une quelconque des revendications 1-16.

18. Quantité d'inhibition de la croissance d'une tumeur d'un composé de la formule I selon l'une quelconque des revendications 1-16 ou son sel et/ou solvate pharmaceutiquement acceptable pour l'inhibition de la croissance d'une tumeur chez un hôte mammalien.
